# EUROPÄISCHE PATENTSCHRIFT

(11) **EP 1 562 939 B1**
(45) Veröffentlichungstag und Bekanntmachung des Hinweises auf die Patenterteilung: **21.01.2009**
(21) Anmeldenummer: 03776875.1
(22) Anmeldetag: 30.10.2003
(51) Int. Cl.: C07D 413/12, C07D 265/32, C07D 409/12, A61K 31/5377, A61P 7/02, A61P 9/00, A61P 35/00

(54) **CARBONSÄUREAMIDE**
CARBOXAMIDES
CARBOXAMIDES

(30) Priorität: 21.11.2002 DE 10254336
(43) Veröffentlichungstag der Anmeldung: 17.08.2005
(73) Patentinhaber: Merck Patent GmbH, 64293 Darmstadt (DE)
(72) Erfinder: DORSCH, Dieter, 64372 Ober-Ramstadt (DE); CEZANNE, Bertram, 64546 Mörfelden-Walldorf (DE); MEDERSKI, Werner, 64673 Zwingenberg (DE); TSAKLAKIDIS, Christos, 69469 Weinheim (DE); WURZIGER, Hanns, 64291 Darmstadts (DE); GLEITZ, Johannes, 64289 Darmstadt (DE); VAN AMSTERDAM, Christoph, 64295 Darmstadt (DE)
(86) Internationale Anmeldenummer: PCT/EP2003/012080
(87) Internationale Veröffentlichungsnummer: WO 2004/046138

(56) Entgegenhaltungen:
- WO-A-00/71510
- WO-A-02/06269
- WO-A-02/48099
- WO-A-02/057236

## Beschreibung

Die Erfindung betrifft Verbindungen der Formel I 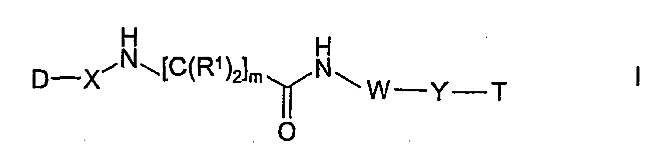 worin
- D: unsubstituierter oder ein- oder mehrfach durch Hal, A, OR² , N(R²)₂, NO₂, CN, COOR² oder CON(R²)₂ substituierter aromatischer Carbo- oder Heterocyclus mit 0 bis 4 N-, O- und/oder S-Atomen,
- X: -C=O oder C(R³)₂,
- W: -[C(R³)₂]ₙ-,
- R¹: H oder A, das durch OR³, S(O)ₙR³ , N(R³)₂, CN, COOR³, CON(R³)₂, OCON(R³)₂, N(R³)COOR³, N(R³)CON(R³)_{2,} N(R³)SO₂R³, SO₂N(R³)₂ oder -C≡C- substituiert sein kann,
- R²: H, A, -[C(R³)₂]ₙ-Ar', -[C(R³)₂]ₙ-Het', -[C(R³)₂]ₙ-Cycloalkyl, -[C(R³)₂]ₙ-N(R₃)₂ oder -[C(R³)₂]ₙ-OR³,
- R³: H oder A,
- Y: Alkylen, Cycloalkylen, Het-diyl oder Ar-diyl,
- T: einen ein- oder zweikernigen gesättigten, ungesättigten oder aromatischen Carbo- oder Heterocyclus mit 0 bis 4 N-, O- und/oder S-Atomen, der ein- oder zweifach durch =O, =S, =NR², =N-CN, =N-NO₂, =NOR², =NCOR², =NCOOR², =NOCOR² substituiert ist und ferner ein-, zwei- oder dreifach durch R², Hal, A, -[C(R³)₂]ₙ-Ar, -[C(R³)₂]ₙ-Het, -[C(R³)₂]ₙ-Cycloalkyl, OR², N(R²)₂, NO₂, CN, COOR², CON(R²)₂, NR²COA, NR²CON(R²)₂, NR²SO₂A, COR², SO₂NR² und/oder S(O)ₙA substituiert sein kann,
- A: unverzweigtes oder verzweigtes Alkyl mit 1-10 C-Atomen, worin eine oder zwei CH₂-Gruppen durch O- oder S-Atome und/oder durch -CH=CH-Gruppen und/oder auch 1-7 H-Atome durch F ersetzt sein können,
- Ar: unsubstituiertes oder ein-, zwei- oder dreifach durch Hal, A, OR², N(R²)₂, NO₂, CN, COOR², CON(R²)₂, NR²COA, NR²CON(R²)₂, NR²SO₂A, COR², SO₂N(R²)₂, S(O)ₙA, -[C(R³)₂]ₙ-COOR² oder -O-[C(R³)₂]ₒ-COOR² substituiertes Phenyl, Naphthyl oder Biphenyl,
- Ar': unsubstituiertes oder ein-, zwei- oder dreifach durch Hal, A, OR³, N(R³)₂, NO₂, CN, COOR³, CON(R³)₂, NR³COA, NR³CON(R³)₂, NR³SO₂A, COR³, SO₂N(R³)₂, S(O)ₙA, -[C(R³)₂]ₙ-COOR³ oder -O-[C(R³)₂]ₒ-COOR³ substituiertes Phenyl,
- Het: einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Carbonylsauerstoff, =S, =N(R²)₂, Hal, A, -[C(R³)₂]ₙ-Ar, -[C(R³)₂]ₙ-Het', -[C(R³)₂]ₙ-Cycloalkyl, -[C(R³)₂]ₙ-OR², -[C(R³)₂]ₙ-N(R³)₂, NO₂, CN, -[C(R³)₂]ₙ-COOR^{2'} -[C(R³)₂]ₙ₋CON(R²)₂, -[C(R³)₂]ₙ-NR²COA, NR₂CON(R²)₂, -[C(R³)₂]ₙ-NR²SO₂A, COR², SO₂NR² und/oder S(O)ₙA substituiert sein kann,
- Het': einen ein- oder zweikernigen gesättigten, ungesättigten oder aromatischen Heterocyclus mit 1 bis 4 N-, O- und/oder S-Atomen, der unsubstituiert oder ein- oder zweifach durch Carbonylsauerstoff, =S, =N(R³)₂, Hal, A, OR³, N(R³)₂, NO₂, CN, COOR³, CON(R³)_{2,} NR³COA, NR³CON(R³)₂, NR³SO₂A, COR³, SO₂NR³ und/oder S(O)ₙA substituiert sein kann,
- Hal: F, Cl, Br oder I,
- m: 1 oder 2,
- n: 0, 1 oder 2,
- o: 1, 2 oder 3
bedeuten,
sowie ihre pharmazeutisch verwendbaren Salze Solvate und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie Faktor Xa inhibierende Eigenschaften und können daher zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens eingesetzt werden.

Die erfindungsgemäßen Verbindungen der Formel I können weiterhin Inhibitoren der Gerinnungsfaktoren Faktor VIIa, Faktor IXa und Thrombin der Blutgerinnungskaskade sein.

Aromatische Amidinderivate mit antithrombotischer Wirkung sind z.B. aus der EP 0 540 051 B1, WO 98128269, WO 00/71508, WO 00/71511. WO 00/71493, WO 00/71507, WO 00/71509, WO 00/71512, WO 00/71515 oder WO 00/71516 bekannt. Cyclische Guanidine zur Behandlung thromboembolischer Erkrankungen sind z.B. in der WO 97/08165 beschrieben. Aromatische Heterocyclen mit Faktor Xa inhibitorischer Aktivität sind z.B. aus der WO 96/10022 bekannt. Substituierte N-[(Aminoiminomethyl)-phenylalkyl]-azaheterocyclylamide als Faktor Xa Inhibitoren sind in WO 96/40679 beschrieben.
Andere Carbonsäureamidderivate sind aus WO 02/48099 und WO 02/57236 bekannt.
Weitere Faktor Xa Inhibitoren sind in WO 00/76970, WO 00/76971 und WO 01/96303 beschrieben.

Der antithrombotische und antikoagulierende Effekt der erfindungsgemäßen Verbindungen wird auf die inhibierende Wirkung gegenüber der aktivierten Gerinnungsprotease, bekannt unter dem Namen Faktor Xa, oder auf die Hemmung anderer aktivierter Serinproteasen wie Faktor VIIa, Faktor IXa oder Thrombin zurückgeführt.

Faktor Xa ist eine der Proteasen, die in den komplexen Vorgang der Blutgerinnung involviert ist. Faktor Xa katalysiert die Umwandlung von Prothrombin in Thrombin. Thrombin spaltet Fibrinogen in Fibrinmonomere, die nach Quervernetzung elementar zur Thrombusbildung beitragen. Eine Aktivierung von Thrombin kann zum Auftreten von thromboembolischen Erkrankungen führen. Eine Hemmung von Thrombin kann jedoch die in die Thrombusbildung involvierte Fibrinbildung inhibieren.
Die Messung der Inhibierung von Thrombin kann z.B. nach der Methode von G. F. Cousins et al. in Circulation 1996, 94, 1705-1712 erfolgen.

Eine Inhibierung des Faktors Xa kann somit verhindern, daß Thrombin gebildet wird.
Die erfindungsgemäßen Verbindungen der Formel I sowie ihre Salze greifen durch Inhibierung des Faktors Xa in den Blutgerinnungsprozeß ein und hemmen so die Entstehung von Thromben.

Die Inhibierung des Faktors Xa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Hauptmann et al. in Thrombosis and Haemostasis 1990, 63, 220-223 beschrieben.

Die Messung der Inhibierung von Faktor Xa kann z.B. nach der Methode von T. Hara et al. in Thromb. Haemostas. 1994, 71, 314-319 erfolgen.

Der Gerinnungsfaktor Vlla initiiert nach Bindung an Tissue Faktor den extrinsischen Teil der Gerinnungskaskade und trägt zur Aktivierung des Faktors X zu Faktor Xa bei. Eine Inhibierung von Faktor Vlla verhindert somit die Entstehung des Faktors Xa und damit eine nachfolgende Thrombinbildung.
Die Inhibierung des Faktors Vlla durch die erfindungsgemäßen Verbindungen und die Messung der antikoagulierenden und antithrombotischen Aktivität kann nach üblichen in vitro- oder in vivo-Methoden ermittelt werden. Ein übliches Verfahren zur Messung der Inhibierung von Faktor Vlla wird z.B. von H. F. Ronning et al. in Thrombosis Research 1996, 84, 73-81 beschrieben.

Der Gerinnungsfaktor IXa wird in der intrinsischen Gerinnungskaskade generiert und ist ebenfalls an der Aktivierung von Faktor X zu Faktor Xa beteiligt. Eine Inhibierung von Faktor lXa kann daher auf andere Weise verhindern, daß Faktor Xa gebildet wird.
Die Inhibierung von Faktor IXa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Chang et al. in Journal of Biological Chemistry 1998, 273, 12089-12094 beschrieben.

Die erfindungsgemäßen Verbindungen können weiterhin zur Behandlung von Tumoren, Tumorerkrankungen und/oder Tumormetastasen verwendet werden.
Ein Zusammenhang zwischen dem Tissuefaktor TF / Faktor Vlla und der Entwicklung verschiedener Krebsarten wurde von T.Taniguchi und N.R.Lemoine in Biomed. Health Res. (2000), 41 (Molecular Pathogenesis of Pancreatic Cancer), 57-59, aufgezeigt.
Die im nachfolgenden aufgeführten Publikationen beschreiben eine antitumorale Wirkung von TF-Vll und Faktor Xa Inhibitoren bei verschiedenen Tumorarten:
K.M. Donnelly et al. in Thromb. Haemost. 1998; 79: 1041-1047;
E.G. Fischer et al. in J. Clin. Invest. 104: 1213-1221 (1999);
B.M. Mueller et al. in J. Clin. Invest. 101: 1372-1378 (1998);
M.E. Bromberg et al. in Thromb. Haemost. 1999; 82: 88-92

Die Verbindungen der Formel I können als Arzneimitteiwirkstoffe in der Human- und Veterinärmedizin eingesetzt werden, insbesondere zur Behandlung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, venöse Thrombose, pulmonale Embolie, arterielle Thrombose, myocardiale lschämie, instabile Angina und auf Thrombose basierender Schlaganfall.
Die erfindungsgemäßen Verbindungen werden auch zur Behandlung oder Prophylaxe von atherosklerotischen Erkrankungen wie koronarer arterieller Erkrankung, cerebraler arterieller Erkrankung oder peripherer arterieller Erkrankung eingesetzt.
Die Verbindungen werden auch in Kombination mit anderen Thrombolytika bei myocardialem Infarkt eingesetzt, ferner zur Prophylaxe zur Reocclusion nach Thrombolyse, percutaner transluminaler Angioplastie (PTCA) und koronaren Bypass-Operationen.
Die erfindungsgemäßen Verbindungen werden ferner verwendet zur Prävention von Rethrombose in der Mikrochirurgie, ferner als Antikoagulantien im Zusammenhang mit künstlichen Organen oder in der Hämodialyse.
Die Verbindungen finden ferner Verwendung bei der Reinigung von Kathetern und medizinischen Hilfsmitteln bei Patienten *in vivo*, oder als Antikoagulantien zur Konservierung von Blut, Plasma und anderen Blutprodukten *in vitro.* Die erfindungsgemäßen Verbindungen finden weiterhin Verwendung bei solchen Erkrankungen, bei denen die Blutkoagulation entscheidend zum Erkrankungsverlauf beiträgt oder eine Quelle der sekundären Pathologie darstellt, wie z.B. bei Krebs einschließlich Metastasis, entzündlichen Erkrankungen einschließlich Arthritis, sowie Diabetes.

Die erfindungsgemäßen Verbindungen finden weiterhin Verwendung zur Behandlung von Migräne (F.Morales-Asin et al., Headache, 40, 2000, 45-47).

Bei der Behandlung der beschriebenen Erkrankungen werden die erfindungsgemäßen Verbindungen auch in Kombination mit anderen thrombolytisch wirksamen Verbindungen eingesetzt, wie z.B. mit dem "tissue plasminogen activator" t-PA, modifiziertem t-PA, Streptokinase oder Urokinase. Die erfindungsgemäßen Verbindungen werden mit den anderen genannten Substanzen entweder gleichzeitig oder vorher oder nachher gegeben.
Besonders bevorzugt ist die gleichzeitige Gabe mit Aspirin, um ein Neuauftreten der Thrombenbildung zu verhindern.
Die erfindungsgemäßen Verbindungen werden auch verwendet in Kombination mit Blutplättchen-Glycoprotein-Rezeptor (IIb/IIIa)-Antagonisten, die die Blutplättchenaggregation inhibieren.

Gegenstand der Erfindung sind die Verbindungen der Formel I und ihre Salze sowie ein Verfahren zur Herstellung von Verbindungen der Formel I nach den Ansprüchen 1-16 sowie ihrer pharmazeutisch verwendbaren Salze Solvate und Stereoisomere, dadurch gekennzeichnet, daß man
a) eine Verbindung der Formel II

   H₂N-W-Y-T II

   worin
   W, Y und T die in Anspruch 1 angegebenen Bedeutungen haben,
   mit einer Verbindung der Formel III 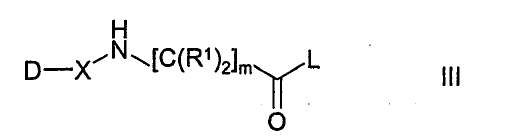 worin
   - L: Cl, Br, I oder eine freie oder reaktionsfähig funktionell abgewandelte OH-Gruppe bedeutet und
   - R¹, m,: X und D die in Anspruch 1 angegebenen Bedeutungen haben,
   umsetzt,
   oder
b) zur Herstellung von Verbindungen der Formel I,
   in denen X -C=O bedeutet,
   eine Verbindung der Formel IV 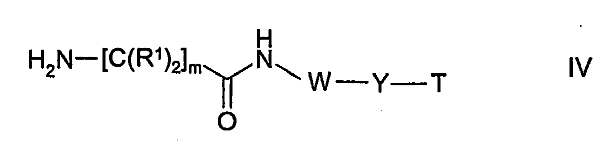 worin R¹, m, W, Y und T die in Anspruch 1 angegebenen Bedeutungen
   haben,
   mit einer Verbindung der Formel V

   D-CO-L V

   worin
   - L: Cl, Br, I oder eine freie oder reaktionsfähig funktionell abgewandelte OH-Gruppe bedeutet und
   - D: die in Anspruch 1 angegebene Bedeutung hat,
   umsetzt,
   oder
c) zur Herstellung von Verbindungen der Formel I,
   in denen X CH₂ bedeutet,
   eine Verbindung der Formel IV 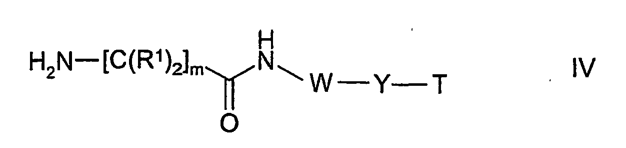 worin R¹, m, W, Y und T die in Anspruch 1 angegebenen Bedeutungen
   haben,
   mit einer Verbindung der Formel VI

   D-CHO VI

   worin
   D die in Anspruch 1 angegebene Bedeutung hat,
   in einer reduktiven Aminierung
   umsetzt,
   und/oder
   eine Base oder Säure der Formel I in eines ihrer Salze umwandelt.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in lnt. J. Pharm. 115, 61-67 (1995) beschrieben ist.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Für alle Reste, die mehrfach auftreten, wie z.B. R¹ in (R¹)₂, gilt, daß deren Bedeutungen unabhängig voneinander sind.

Vor- und nachstehend haben die Reste bzw. Parameter D, W, X, Y, T, R¹ die bei der Formel I angegebenen Bedeutungen, falls nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1-, 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, worin eine oder zwei CH₂-Gruppen durch O- oder S-Atome und/oder durch -CH=CH-Gruppen und/oder auch 1-7 H-Atome durch F ersetzt sein können.

Cycloalkyl bedeutet vorzugsweise Cyclopropyl, Cyclobutyl, Cylopentyl, Cyclohexyl oder Cycloheptyl.
Alkylen bedeutet vorzugsweise Methylen, Ethylen, Propylen, Butylen, Pentylen oder Hexylen, ferner verzweigtes Alkylen.

COR² bedeutet z.B. CHO oder -COA.
-COA (Acyl) bedeutet vorzugsweise Acetyl, Propionyl, ferner auch Butyryl, Pentanoyl, Hexanoyl oder z.B. Benzoyl.
Hal bedeutet vorzugsweise F, Cl oder Br, aber auch I.

Aromatischer Carbocyclus bedeutet z.B. Phenyl, Biphenyl oder Naphthyl. Gesättigter Carbocyclus bedeutet vorzugsweise Cycloalkyl, wie z.B. Cyclohexan oder Cyclopentan.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m-oder p-Aminophenyl, o-, m- oder p-(N-Methylamino)-phenyl, o-, m- oder p-(N-Methylaminocarbonyl)-phenyl, o-, m- oder p-Acetamidophenyl, o-, m-oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m-oder p-Bromphenyl, o-, m- oder p- Chlorphenyl, o-, m- oder p-(Methyl-sulfonamido)-phenyl, o-, m- oder p-(Methylsulfonyl)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-di-methylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet vorzugsweise z.B. unsubstituiertes oder ein- oder zweifach durch Hal, A, OA, SO₂A, COOR², SO₂NH₂ oder CN substituiertes Phenyl. Ar bedeutet insbesondere bevorzugt z.B. unsubstituiertes oder ein- oder zweifach durch Hal, A, OA, SO₂A, SO₂NH₂, COOR² oder CN substituiertes Phenyl, wie z.B. Phenyl, 2-Methylsulfonylphenyl, 2-Aminosulfonylphenyl, 2-, 3- oder 4-Chlorphenyl, 4-Methylphenyl, 4-Bromphenyl, 3-Fluor-4-methoxyphenyl, 4-Trifluormethoxyphenyl, 4-Ethoxyphenyl, 2-Methoxyphenyl, 3-Cyanphenyl oder 4-Ethoxycarbonylphenyl.

Ganz besonders bevorzugt bedeutet Ar unsubstituiertes oder ein- oder zweifach durch A und/oder Hal substituiertes Phenyl.

Y bedeutet vorzugsweise Het-diyl oder Ar-diyl, besonders bevorzugt unsubstituiertes oder einfach durch A, OA, Cl oder F substituiertes 1,4-Phenylen, ferner auch Pyridin-diyl, vorzugsweise Pyridin-2,5-diyl oder Piperidin-diyl.
Y bedeutet insbesondere unsubstituiertes oder einfach durch Methyl, Ethyl,-Propyl, Cl oder F substituiertes 1,3- oder 1,4-Phenylen.
Y bedeutet ganz besonders bevorzugt unsubstituiertes oder ein- oder zweifach durch A und/oder Hal substituiertes Phenylen, z.B. unsubstituiertes oder einfach durch Methyl, Ethyl, Propyl, Cl oder F substituiertes 1,4-Phenylen.

Unsubstituiertes Het bedeutet z.B. 2- oder 3-Furyl, 2- oder 3-Thienyl, 1-, 2-oder 3-Pyrrolyl, 1-, 2, 4- oder 5-Imidazolyl, 1-, 3-, 4- oder 5-Pyrazolyl, 2-, 4-oder 5-Oxazolyl, 3-, 4- oder 5-lsoxazolyl, 2-, 4- oder 5-Thiazolyl, 3-, 4- oder 5-lsothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1-oder 5-Tetrazolyl, 1,2,3-Oxadiazol-4- oder -5-yl, 1,2,4-Oxadiazol-3- oder - 5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6-oder 7-Indolyl, 4- oder 5-lsoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.

Die heterocyclischen Reste können auch teilweise oder vollständig hydriert sein.
Het kann also z. B. auch bedeuten 2,3-Dihydro-2-, -3-, -4- oder -5-furyl, 2,5-Dihydro-2-, -3-, -4- oder 5-furyl, Tetrahydro-2- oder-3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder - 4-pyranyl, 1,4-Dioxanyl, 1,3-Dioxan-2-, -4- oder -5-yl, Hexahydro-1-, -3-oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Unsubstituiertes Het' hat die für Het oben angegebenen bevorzugten Bedeutungen.

T bedeutet vorzugsweise einen ein- oder zweikernigen gesättigten, ungesättigten oder aromatischen Heterocyclus mit 1 bis 2 N- und/oder O-Atomen, der ein- oder zweifach durch =O, =S, =NR², =N-CN, =N-NO₂, =NOR², =NCOR², =NCOOR² oder =NOCOR² substituiert ist und ferner ein-oder zweifach durch Hal oder A substituiert sein kann.

T bedeutet in einer weiteren Ausführungsform vorzugsweise z.B. 2-lmino-piperidin-1-yl, 2-Imino-pyrrolidin-1-yl, 2-Imino-1*H*-pyridin-1-yl, 3-Imino-morpholin-4-yl, 4-Imino-1*H*-pyridin-1-yl, 2,6-Diimino-piperidinl-yl, 2-Imino-piperazin-1-yl, 2,6-Diimino-piperazin-1-yl, 2,5-Diimino-pyrrolidin-1-yl, 2-Imino-1,3-oxazolidin-3-yl, 3-Imino-2*H*-pyridazin-2-yl, 2-Imino-azepan-1-yl, 2-Hydroxy-6-imino-piperazin-1-yl oder 2-Methoxy-6-imino-piperazin-1-yl.

T bedeutet insbesondere einen ein- oder zweikernigen gesättigten oder ungesättigten Heterocyclus mit 1 bis 2 N- und/oder O-Atomen, der ein-oder zweifach durch =O, =S oder =NH substituiert ist.

T bedeutet besonders bevorzugt ein- oder zweifach durch =O oder =NH substituiertes Piperidin-1-yl, Pyrrolidin-1-yl, Pyridin-1-yl, Morpholin-4-yl, Piperazin-1-yl, 1,3-Oxazolidin-3-yl, Pyridazin-2-yl, Pyrazin-1-yl, Azepan-1-yl oder 2-Aza-bicyclo[2.2.2]-octan-2-yl,

D bedeutet vorzugsweise unsubstituierter oder ein- oder zweifach durch Hal substituierter aromatischer Fünfringheterocyclus mit 1 bis 2 N-, O- und/oder S-Atomen.
In einer weiteren Ausführungsform bedeutet D vorzugsweise ein- oder zweifach durch Hal substituiertes Thienyl, Thiazolyl oder Furyl.
D bedeutet weiterhin bevorzugt ein- oder zweifach durch Hal substituiertes Thienyl oder Phenyl.
Insbesondere bedeutet D einen ein- oder zweifach durch Hal substituierten Thienylring.

R¹ bedeutet bevorzugt H oder A, das durch OR³, CON(R³)₂, N(R³)₂, S(O)ₙR³, COOR³, OCON(R³)₂, N(R³)COOR³ oder -C≡C- substituiert sein kann.

R¹ bedeutet insbesondere H oder A, das durch OH, OA', CONH₂, NH₂, N(A')₂, SO₂A', SA', COOA', COOH, OCONH₂, -C≡C- oder NHCOOA' substituiert sein kann, wobei A' Alkyl mit 1, 2, 3, 4, 5 oder 6 C-Atomen bedeutet.

R² bedeutet vorzugsweise z.B. H oder Alkyl mit 1, 2, 3, 4, 5 oder 6 C-Atomen.
R³ bedeutet vorzugsweise H oder Alkyl mit 1, 2, 3, 4, 5 oder 6 C-Atomen. X bedeutet vorzugsweise -C=O oder CH₂, ganz besonders bevorzugt -C=O.
W ist vorzugsweise eine Bindung, d.h. es fehlt oder W ist CH₂. Ganz besonders bevorzugt fehlt W.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis lo ausgedrückt werden, die der Formel I entsprechen und worin die nicht näher bezeichneten Reste die bei der Formel I angegebene Bedeutung haben, worin jedoch
- in Ia: D unsubstituierter oder ein- oder zweifach durch Hal substituierter aromatischer Fünfringheterocyclus mit 1 bis 2 N-, O- und/oder S-Atomen, bedeutet;
- IN iB: d ein- oder zweifach durch Hal substituierter Thienylring bedeutet;
- in Ic: R² H oder Alkyl mit 1, 2, 3, 4, 5 oder 6 C-Atomen bedeutet;
- in Id: R¹ H oder A, das durch OR³, CON(R³)₂, N(R³)_{2,} S(O)ₙR³, COOR³, OCON(R³)₂, N(R³)COOR³ oder -C≡C- substituiert sein kann, bedeutet;
- in Ie: X -C=O bedeutet;
- in If: W fehlt bedeutet;
- in Ig: Y Ar-diyl bedeutet;
- in Ih: T einen ein- oder zweikenigen gesättigten, ungesättigten oder aromatischen Heterocyclus mit 1 bis 2 N- und/oder O-Atomen, der ein- oder zweifach durch =O, =S, =NR², =N-CN, =N-NO₂, =NOR², =NCOR², =NCOOR² oder =NOCOR² substituiert ist und ferner ein- oder zweifach durch Hal oder A substituiert sein kann, bedeutet;
- in Ii: T einen ein- oder zweikernigen gesättigten oder ungesättigten Heterocyclus mit 1 bis 2 N- und/oder O-Atomen, der ein- oder zweifach durch =O, =S oder =NH substituiert ist, bedeutet;
- in Ij: T ein- oder zweifach durch =O oder =NH substituiertes Piperidin-1-yl, Pyrrolidin-1-yl, Pyridin-1-yl, Morpholin-4-yl, Piperazin-1-yl, 1,3-Oxazolidin-3-yl, Pyridazin-2-yl, Pyrazin-1-yl, Azepan-1-yl oder 2-Aza-bicyclo[2.2.2]-octan-2-yl, bedeutet;
- in Ik: Ar unsubstituiertes oder ein- oder zweifach durch Hal, A, OA, SO₂A, COOR², SO₂NH₂ oder CN substituiertes Phenyl, bedeutet;
- in Il: Ar unsubstituiertes oder ein- oder zweifach durch A und/oder Hal substituiertes Phenyl, bedeutet;
- in Im: D unsubstituierter oder ein- oder zweifach durch Hal substituierter aromatischer Fünfringheterocyclus mit 1 bis 2 N-, O- und/oder S-Atomen,
R¹ H oder A, das durch OR³, CON(R³)₂, N(R³)₂, S(O)ₙR³, COOR³, OCON(R³)₂, N(R³)COOR³ oder -C≡C- substituiert sein kann,
R² H oder Alkyl mit 1, 2, 3, 4, 5 oder 6 C-Atomen,
X -C=O oder CH₂,
W fehlt,
Y Ar-diyl,
Ar unsubstituiertes oder ein- oder zweifach durch A und/oder Hal substituiertes Phenyl,
T einen ein- oder zweikernigen gesättigten oder ungesättigten Heterocyclus mit 1 bis 2 N- und/oder O-Atomen, der ein-oder zweifach durch =O, =S oder =NH substituiert ist, bedeutet;
- in In: D ein- oder zweifach durch Hal substituiertes Thienyl, Thiazolyl oder Furyl,
R¹ H oder A, das durch OR³, CON(R³)₂, N(R³)₂, S(O)ₙR³, COOR³, OCON(R³)₂, N(R³)COOR³ oder -C ≡C- substituiert sein kann,
R² H oder Alkyl mit 1, 2, 3, 4, 5 oder 6 C-Atomen,
X -C=O oder CH₂,
W fehlt,
Y Ar-diyl,
Ar unsubstituiertes oder ein- oder zweifach durch A und/oder Hal substituiertes Phenyl,
T ein- oder zweifach durch =O oder =NH substituiertes Piperidin-1-yl, Pyrrolidin-1-yl, Pyridin-1-yl, Morpholin-4-yl, Piperazin-1-yl, 1,3-Oxazolidin-3-yl, Pyridazin-2-yl, Pyrazin-1-yl, Azepan-1-yl oder 2-Aza-bicyclo[2.2.2]-octan-2-yl, bedeutet;
- In Io: D ein- oder zweifach durch Hal substituiertes Thienyl oder Phenyl,
R¹ H oder Alkyl mit 1-6 C-Atomen, das durch OR³, CON(R³)₂, N(R³)₂, S(O)ₙR³, COOR³, OCON(R³)₂, N(R³)COOR³ oder -C≡C- substituiert sein kann,
R² H oder Alkyl mit 1, 2, 3, 4, 5 oder 6 C-Atomen,
R³ H oder Alkyl mit 1, 2, 3, 4, 5 oder 6 C-Atomen,
X -C=O oder CH₂,
W fehlt oder CH₂,
Y Ar-diyl,
A Alkyl mit 1, 2, 3, 4, 5 oder 6 C-Atomen, worin eine oder zwei CH₂-Gruppen durch O- oder S-Atome und/oder durch -CH=CH-Gruppen und/oder auch 1-7 H-Atome durch F ersetzt sein können,
Ar unsubstituiertes oder ein- oder zweifach durch A und/oder Hal substituiertes Phenyl,
T ein- oder zweifach durch =O oder =NH substituiertes Piperidin-1-yl, Pyrrolidin-1-yl, Pyridin-1-yl, Morpholin-4-yl, Piperazin-1-yl, 1,3-Oxazolidin-3-yl, Pyridazin-2-yl, Pyrazin-1-yl, Azepan-1-yl oder 2-Aza-bicyclo[2.2.2]-octan-2-yl, bedeutet;
sowie ihre pharmazeutisch verwendbaren Salze, Solvate und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel umsetzt.

Die Ausgangsverbindungen der Formeln II, III, IV und V sind in der Regel bekannt. Sind sie neu, so können sie aber nach an sich bekannten Methoden hergestellt werden.

Verbindungen der Formel I können vorzugsweise erhalten werden, indem man Verbindungen der Formel II mit Verbindungen der Formel III umsetzt.

In den Verbindungen der Formel III bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).
Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.
Aktivierte Ester werden zweckmäßig in situ gebildet, z. B. durch Zusatz von HOBt oder N-Hydroxysuccinimid.

Die Umsetzung erfolgt in der Regel in einem inerten Lösungsmittel, in Gegenwart eines säurebindenden Mittels vorzugsweise einer organischen Base wie DIPEA, Triethylamin, Dimethylanilin, Pyridin oder Chinolin oder eines Überschusses der Carboxykomponente der Formel III.
Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.
Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure, Essigsäure oder Trifluoressigsäure (TFA); Nitroverbindungen wie Nitromethan oder Nitrobenzol; Ester wie Ethylacetat oder Gemische der genannten Lösungsmittel.

Verbindungen der Formel I, worin X-C=O bedeutet, können weiter vorzugsweise erhalten werden, indem man Verbindungen der Formel IV mit Verbindungen der Formel V umsetzt.
Die Umsetzung erfolgt in der Regel in einem inerten Lösungsmittel und unter Bedingungen wie oben angegeben.
In den Verbindungen der Formel V bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulforiyloxy) oder Arylsulfonyloxy mit 6-10 C-Atomen (bevorzugt Phenyl- oder p-Tolylsulfonyloxy).
Aktivierte Ester werden zweckmäßig in situ gebildet, z. B. durch Zusatz von HOBt oder N-Hydroxysuccinimid.

Die Umsetzung erfolgt in der Regel in einem inerten Lösungsmittel, in Gegenwart eines säurebindenden Mittels vorzugsweise einer organischen Base wie DIPEA, Triethylamin, Dimethylanilin, Pyridin oder Chinolin oder eines Überschusses der Carboxykomponente der Formel V.
Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.
Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.
Als inerte Lösungsmittel eignen sich die oben genannten.

Verbindungen der Formel I, worin X CH₂ bedeutet, können weiter vorzugsweise erhalten werden, indem man Verbindungen der Formel IV mit Verbindungen der Formel VI umsetzt.
Die Umsetzung erfolgt in der Regel unter Bedingungen einer reduktiven Aminierung wie sie jedem Fachmann bekannt sind.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate können zur Isolierung und /oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.
Auch physiologisch unbedenkliche organische Basen, wie z.B. Ethanolamin können verwendet werden.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder oder auch als Nasenspray. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen verwendet werden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Gegenstand der Erfindung sind ferner Arzneimittel enthaltend mindestens eine Verbindung der Formel I und/oder ihre pharmazeutisch verwendbaren Salze, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Salze, Solvate und Stereo-isomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Salze Solvate und Stereoisomere, einschließlich deren Mischungen in allen Verhältnissen,
und einer wirksamen Menge eines weiteren Arzneimittelwirkstoffs gelöst oder in lyophilisierter Form vorliegt.

Gegenstand der Erfindung ist ferner die Verwendung von Verbindungen der Formel I und/oder ihrer pharmazeutisch verwendbaren Salze, Solvate und Stereoisomere, einschließlich deren Mischungen in allen Verhältnissen,
zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen,
in Kombination mit mindestens einem weiteren Arzneimittelwirkstoff.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
- Massenspektrometrie (MS):: EI (Elektronenstoß-Ionisation) M⁺
FAB (Fast Atom Bombardment) (M+H)⁺
ESI (Electrospray lonization) (M+H)⁺ (wenn nichts anderes angegeben)

### Beispiel 1

Die Herstellung von (R)-2-[(5-Chlor-thiophen-2-carbonyl)-amino]-N-[3-methyl-4-(3-oxo-morpholin-4-yl)-phenyl]-4-methyl-valeriansäureamid, erfolgt analog nachstehendem Schema:

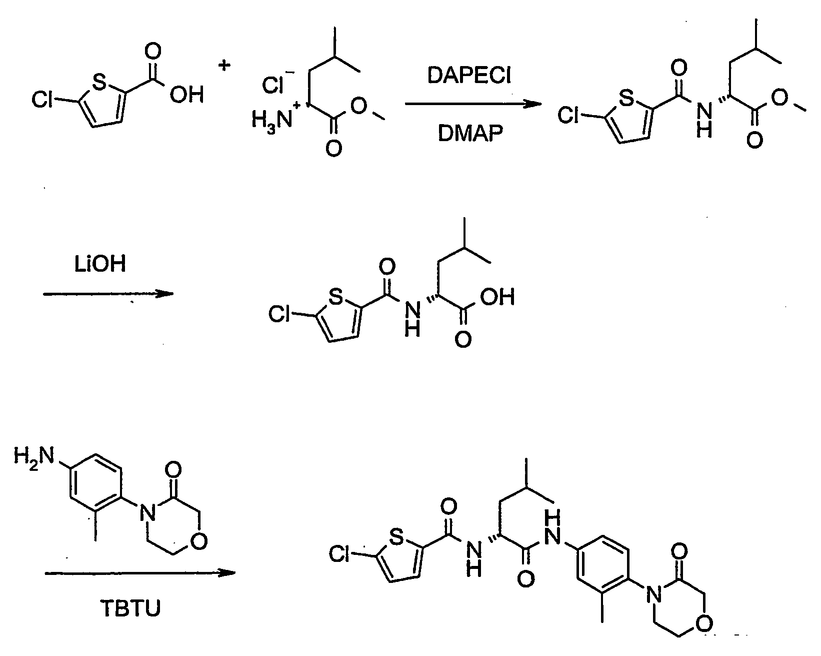

Eine Lösung von 4.48 g (72.5 mmol) 2-Chlorthiophen-5-carbonsäure und 5.00 g (27.5 mmol) D-Leucin-methylester Hydrochlorid in 100 ml Acetonitril wird mit 5.50 g (45.0 mmol) 4-Dimethylaminopyridin (DMAP) und 5.75 g (30.0 mmol) *N*-(3-Dimethylaminopropyl)-*N'*-ethylcarbodiimidhydrochlorid (DAPECI) versetzt und 18 Stunden bei Raumtemperatur gerührt. Das Reaktionsgemisch wird eingedampft und der Rückstand zwischen tert.Butylmethylether und Wasser verteilt. Die organische Phase wird mit Kaliumhydrogensulfatlösung, gesättigter Natriumhydrogencarbonatlösung und Wasser gewaschen. Die organische Phase wird über Natriumsulfat getrocknet und eingedampft: (R)-2-[(5-Chlorthiophen-2-carbonyl)-amino]-4-methyl-pentansäuremethylester als farbloses Öl; ESI 290.

Eine Lösung von 7.00 g (24.25 mmol) (R)-2-[(5-Chlorthiophen-2-carbonyl)-amino]-4-methyl-pentansäuremethylester in 60 ml THF wird mit einer Lösung von 1.20 g (24.0 mmol) Lithiumhydroxid in 60 ml Wasser versetzt und 18 Stunden bei Raumtemperatur gerührt. Das Reaktionsgemisch wird eingedampft und der Rückstand in 25 ml Wasser aufgenommen. Durch Zugabe von konz. Salzsäure wird ein pH von 3 eingestellt. Der entstandene Niederschlag wird abfiltriert, mit Wasser gewaschen und getrocknet: (R)-2-[(5-Chlorthiophen-2-carbonyl)-amino]-4-methyl-pentansäure als farbloser Feststoff; ESI 276.

Eine Lösung von 137 mg (0.500 mmol) (R)-2-[(5-Chlorthiophen-2-carbonyl)-amino]-4-methyl-pentansäure und 103 mg (0.500 mmol) 4-(4-Amino-2-methyl-phenyl)-morpholin-3-on in 1 ml Dimethylformamid (DMF) wird mit 202 mg (0.629 mmol) 2-(1*H*-Benzotriazol-1-yl)-1,1,3,3-tetramethyluronium-tetrafluoroborat (TBTU) versetzt und 18 Stunden bei Raumtemperatur gerührt. Das Reaktionsgemisch wird mit gesättigter wässriger Natriumhydrogencarbonatlösung versetzt, der entstandene Niederschlag abfiltriert, mit Wasser gewaschen und getrocknet: (R)-2-[(5-Chlor-thiophen-2-carbonyl)amino]-*N*-[3-methyl-4-(3-oxo-morpholin-4-yl)-phenyl]-4-methyl-valeriansäureamid ("1A") als farbloser Feststoff; ESI 464.

Analog erhält man nachstehende Verbindungen
(S)-2-[(5-Chlor-thiophen-2-carbonyl)-amino]-*N*-[4-(3-oxo-morpholin-4-yl)-phenyl]-4-methyl-valeriansäureamid, ESI 450;
(S)-2-[(5-Chlor-thiophen-2-carbonyl)-amino]-*N*-[3-methyl-4-(3-oxo-morpholin-4-yl)-phenyl]-4-methyl-valeriansäureamid, ESI 464;
(S)-2-[(5-Chlor-thiophen-2-carbonyl)-amino]-*N*-[4-(2-oxo-2*H-*pyrazin-1-yl)-phenyl]-4-methyl-valeriansäureamid, ESI 445;
(R)-2-[(5-Chlor-thiophen-2-carbonyl)-amino)-*N*-[4-(3-oxo-morpholin-4-yl)-phenyl]-4-methyl-valeriansäureamid, ESI 450;
(R)-2-[(5-Chlor-thiophen-2-carbonyl)-amino]-*N*-[4-(2-oxo-2*H*-pyrazin-1-yl)-phenyl]-4-methyl-valeriansäureamid ("2A"), ESI 445;
(R)-2-[(5-Chlor-thiophen-2-carbonyl)-amino]-*N*-[4-(2-oxo-2*H-*pyridin-1-yl)-phenyl]-4-methyl-valeriansäureamid, ESI 444;
2-[(5-Chlor-thiophen-2-carbonyl)-amino]-*N*-[3-methyl-4-(2-oxo-piperidin-1-yl)-phenyl]-acetamid,
3-[(5-Chlor-thiophen-2-carbonyl)-amino]-*N*-[3-methyl-4-(2-oxo-piperidin-1-yl)phenyl]-propionsäureamid,
(R)-2-[(5-Chlor-thiophen-2-carbonyl)-amino]-*N*-[3-methyl-4-(3-oxo-morpholin-4-yl)-phenyl]-propionsäureamid, ESI 422;
(R)-2-[(5-Chlor-thiophen-2-carbonyl)-amino]-*N*-[3-methyl-4-(3-oxo-morpholin-4-yl)-phenyl]-3-methyl-buttersäureamid, ESI 450;
(R)-2-[(5-Chlor-thiophen-2-carbonyl)-amino]-*N*-[3-methyl-4-(3-oxo-morpholin-4-yl)-phenyl]-buttersäureamid, ESI 436;
(R)-2-[(5-Chlor-thiophen-2-carbonyl)-amino]-*N*-[3-methyl-4-(3-oxo-morpholin-4-yl)-phenyl]-valeriansäureamid, ESI 450;
(R)-2-[(5-Chlor-thiophen-2-carbonyl)-amino]-*N*-[3-methyl-4-(3-oxo-morpholin-4-yl)-phenyl]-3-aminocarbonyl-propionsäureamid,
2-([(5-Chlor-thiophen-2-carbonyl)amino]-*N*-[3-methyl-4-(3-oxo-morpholin-4-yl)phenyl]-3-(N,N-dimethylamino)-propionsäureamid,
(R)-2-[(5-Brom-thiophen-2-carbonyl)amino]-*N*-[4-(3-oxo-morpholin-4-yl)-phenyl]-4-methyl-valeriansäureamid, ESI 494, 496;
(R)-2-[(5-Chlor-thiophen-2-carbonyl)amino]-*N*-[4-(2-oxo-piperidin-1-yl)benzyl]-4-methyl-valeriansäureamid, ESI 462;
2-[(5-Chlor-thiophen-2-methyl)-amino]-*N*-[4-(3-oxo-morpholin-4-yl)-phenyl]-4-methyl-valeriansäureamid,
(R)-2-[(5-Chlor-thiophen-2-carbonyl)-amino]-*N*-[4-(3-oxo-morpholin-4.-yl)-phenyl]-3-methylsulfanyl-propionsäureamid, ESI 454;
(S)-2-[(5-Chlor-thiophen-2-carbonylamino]-*N-*[4-(2-oxo-piperidin-1-yl)-benzyl]-4-methyl-valeriansäureamid, ESI 462;
(R)-2-[(5-Chlor-thiophen-2-carbonyl)-amino]-*N*-[4-(3-oxo-morpholin-4-yl)-phenyl]-3-methyl-buttersäureamid, ESI 436;
(R)-2-[(5-Chlor-thiophen-2-carbonyl)-amino]-*N*-[4-(2-oxo-2*H*-pyridin-1-yl)-phenyl]-3-methyl-buttersäureamid, ESI 430;
3-[(5-Chlor-thiophen-2-carbonyl)-amino]-*N*-[4-(3-oxo-morpholin-4-yl)-phenyl]-propionsäureamid, ESI 408;
3-[(5-Chlor-thiophen-2-carbonyl)-amino]-*N*-[3-methyl-4-(3-oxo-morpholin-4-yl)-phenyl]-propionsäureamid, ESI 422;
3-[(5-Chlor-thiophen-2-carbonyl)-amino]-*N*-[4-(2-oxo-2*H*-pyridin-1-yl) phenyl]-propionsäureamid, ESI 402;
2-[(5-Chlor-thiophen-2-carbonyl)-amino]-*N*-[3-methyl-4-(3-oxo-morpholin-4-yl)-phenyl]-acetamid, ESI 408;
2-[(5-Chlor-thiophen-2-carbonyl)-amino]-*N*-[4-(3-oxo-morpholin-4-yl) phenyl]-acetamid, ESI 394;
2-[(5-Chlor-thiophen-2-carbonylfamino]-*N*-[4-(2-oxo-2*H*-pyridin-1-yl)-phenyl]-acetamid, ESI 388;
3-[(5-Chlor-thiophen-2-carbonyl)-amino]-*N*-[4-(2-oxo-2*H*-pyridin-1-yl)-phenyl]-2-butyl-propionsäureamid;
(R)-2-[(5-Chlor-thiophen-2-carbonyl)-amino]-*N*-[4-(3-oxo-morpholin-4-yl)-phenyl]-propionsäureamid, ESI 408;
(R)-2-[(5-Chlor-thiophen-2-carbonyl)amino]-*N*-[4-(3-oxo-morpholin-4-yl)-phenyl]-valeriansäureamid, ESI 436;
(R)-2-[(5-Chlor-thiophen-2-carbonyl)-amino]-*N*-[4-(2-oxo-2*H*-pyridin-1-yl)-phenyl]-3-methylsulfanyl-propionsäureamid, ESI 448;
(R)-2-[(5-Chlor-thiophen-2-carbonyl)-amino]-*N*-[4-(2-oxo-2*H*-pyrazin-1-yl)-phenyl]-propionsäureamid, ESI 403;
(R)-2-[(5-Chlor-thiophen-2-carbonyl)-amino]-*N*-[4-(3-oxo-morpholin-4-yl)-phenyl]-4-methylsulfanyl-buttersäureamid, ESI 468;
(R)-2-[(5-Chlor-thiophen-2-carbonyl)-amino]-*N*-[4-(3-oxo-morpholin-4-yl)-phenyl]-buttersäureamid, ESI 422;
(R)-2-[(5-Chlor-thiophen-2-carbonyl)-amino]-*N*-[4-(3-oxo-morpholin-4-yl)-phenyl]-3-ethinyl-propionsäureamid, ESI 432;
(R)-2-[(5-Chlor-thiophen-2-carbonyl)-amino]-*N*-[3-methyl-4-(3-oxo-morpholin-4-yl)-phenyl]-3-ethinyl-propionsäureamid, ESI 446;
(R)-2-[(5-Chlor-thiophen-2-carbonyl)-amino]-*N*-[3-fluor-4-(3-oxo-morpholin-4-yl)-phenyl]-propionsäureamid, ESI 426;
(R)-2-[(5-Chlor-thiophen-2-carbonyl)-amino]-*N*-[3-methyl-4-(3-oxo-morpholin-4-yl)-phenyl]-4-methylsulfanyl-buttersäureamid, ESI 482;
(R)-2-[(5-Chlor-thiophen-2-carbonyl)-amino]-*N*-[4-(3-oxo-morpholin-4-yl)-pheny)]-3-(*tert*.-butyloxycarbonyl)-propionsäureamid, ESI 508;
(R)-2-[(5-Chlor-thiophen-2-carbonyl)-amino]-*N*-[4-(3-oxo-morpholin-4-yl)-phenyl]-3-vinyl-propionsäureamid, ESI 434;
(R)-2-[(5-Chlor-thiophen-2-carbonyl)-amino]-*N*-[3-methyl-4-(3-oxo-morpholin-4-yl)-phenyl]-3-vinyl-propionsäureamid, ESI 448;
(R)-2-[(5-Chlor-thiophen-2-carbonyl)-amino]-*N*-[3-methyl-4-(3-oxo-morpholin-4-yl)-phenyl]-3-(*tert*.-butyloxycarbonyl)-propionsäureamid, ESI 522;
(R)-2-[(5-Chlor-thiophen-2-carbonyl)-amino]-*N*-[4-(3-oxo-morpholin-4-yl)-phenyl]-4-methoxy-buttersäureamid, ESI 452;
(R)-2-[(5-Chlor-thiophen-2-carbonyl)-amino]-*N*-[3-methyl-4-(3-oxo-morpholin-4-yl)-phenyl]-4-methoxy-buttersäureamid, ESI 466;
(R)-2-[(5-Chlor-thiophen-2-carbonyl)-amino]-*N*-[3-fluor-4-(3-oxo-morpholin-4-yl)-phenyl]-4-methyl-valeriansäureamid, ESI 468;
(R)-2-[(5-Chlor-thiophen-2-carbonyl)-amino]-*N*-[3-fluor-4-(3-oxo-morpholin-4-yl)-phenyl]-valeriansäureamid, ESI 454;
(R)-2-[(5-Chlor-thiophen-2-carbonyl)-amino]-*N*-[3-chlor-4-(3-oxo-morpholin-4-yl)-phenyl]-propionsäureamid, ESI 443;
(R)-2-[(5-Chlor-thiophen-2-carbonyl)-amino]-*N*-[4-(3-oxo-morpholin-4-yl)-phenyl]-4-(*tert*.-butyloxycarbonyl)-buttersäureamid, ESI 467 (M-*tert.*-Butyl + H⁺); 545 (M + Na⁺);
(R)-2-[(5-Chlor-thiophen-2-carbonyl)-amino]-*N*-[3-methyl-4-(3-oxo-morpholin-4-yl)-phenyl]-4-(*tert*.-butyloxycarbonyl)-buttersäureamid, ESI 1071 (2 M+H⁺);
(R)-2-[(5-Chlor-thiophen-2-carbonyl)-amino]-*N*-[4-(2-oxo-piperidin-1-yl)-phenyl]-4-(*tert*.-butyloxycarbonyl)-buttersäureamid, ESI 1039 (2 M+H⁺);
(R)-2-[(5-Chlor-thiophen-2-carbonyl)-amino]-*N*-[4-(3-oxo-morpholin-4-yl)-phenyl]-4-(*tert*.-butyloxycarbonylamino)-buttersäureamid, ESI 437 (M - BOC⁺ H⁺);
(R)-2-[(5-Chlor-thiophen-2-carbonyl)-amino]-*N*-[3-methyl-4-(3-oxo-morpholin-4-yl)-phenyl]-4-(*tert*.-butyloxycarbonylamino)-buttersäureamid, ESI 451 (M - BOC + H⁺);
(R)-2-[(5-Chlor-thiophen-2-carbonyl)-amino]-*N*-[4-(3-oxo-morpholin-4-yl)-phenyl]-5-(*tert*.-butyloxycarbonylamino)-valeriansäureamid, ESI 451 (M - BOC + H⁺);
(R)-2-[(5-Chlor-thiophen-2-carbonyl)-amino]-*N*-[3-methyl-4-(3-oxo-morpholin-4-yl)-phenyl]-5-(*ter*t.-butyloxycarbonylamino)-valeriansäureamid, ESI 465 (M - BOC + H⁺);
(R)-2-[(5-Chlor-thiophen-2-carbonyl)-amino]-*N*-[4-(3-oxo-morpholin-4-yl)-phenyl]-3-(*tert*.-butyloxycarbonylamino)-propionsäureamid, ESI 423 (M - BOC + H⁺);
(R)-2-[(5-Chlor-thiophen-2-carbonyl)-amino]-*N*-[3-methyl-4-(3-oxo-morpholin-4-yl)-phenyl]-3-(*tert*.-butyloxycarbonylamino)-propionsäureamid, ESI 437 (M - BOC + H⁺);
(R)-3-[(5-Chlor-thiophen-2-carbonyl)-amino]-*N*-[3-methyl-4-(3-oxo-morpholin-4-yl)-phenyl]-buttersäureamid, ESI 436;
(R)-3-[(5-Chlor-thiophen-2-carbonyl)-amino]-*N*-[3-methyl-4-(3-oxo-morpholin-4-yl)-phenyl]-5-methyl-adipinsäureamid, ESI 478;
(S)-3-[(5-Chlor-thiophen-2-carbonyl)-amino]-*N*-[3-methyl-4-(3-oxo-morpholin-4-yl)-phenyl]-5-methyl-adipinsäureamid, ESI 478;
(R)-2-[(5-Chlor-thiophen-2-carbonyl)-amino]-*N*-[4-(2-oxo-2*H*-pyridin-1-yl)-phenyl]-3-methoxy-propionsäureamid, F. 123-127°;
(R)-2-[(5-Chlor-thiophen-2-carbonyl)-amino]-*N*-[3-methyl-4-(3-oxo-morpholin-4-yl)-phenyl]-3-methoxy-propionsäureamid, F. 74-81 °;
(2R,3R)-2-[(5-Chlor-thiophen-2-carbonyl)-amino]-*N*-[4-(3-oxo-morpholin-4.-yl)-phenyl]-3-methoxy-buttersäureamid, ESI 452;
(2R,3R)-2-[(5-Chlor-thiophen-2-carbonyl)-amino]-*N*-[3-methyl-4-(3-oxo-morpholin-4.-yl)-phenyl]-3-methoxy-buttersäureamid, ESI 466;
(S)-2-[(5-Chlor-thiophen-2-carbonyl)-amino]-*N*-[3-methyl-4-(3-oxo-morpholin-4-yl)-phenyl]-3-methoxy-propionsäureamid, ESI 452;
(R)-2-[(5-Chlor thiophen-2-carbonyl)-amino]-*N*-[3-trifluormethyl-4-(3-oxo-morpholin-4-ylj-phenyl]-3-methoxy-propionsäureamid, ESI 506;
(R)-2-[(5-Chlor-thiophen-2-carbonyl)amino]-*N*-[3-chlor-4-(2-azabicyclo[2.2.2]-octan-2-yl)-phenyl]-3-methoxy-propionsäureamid, ESI 496;
(R)-2-[(5-Chlor-thiophen-2-carbonyl)amino]-*N*-[3-trifluormethoxy-4-(2-aza-bicyclo[2.2.2]-octan-2-yl)-phenyl]-3-methoxy-propionsäureamid, ESI 530;
(R)-2-[(5-Chlor-thiophen-2-carbonyl)-amino]-*N*-[3-chlor-4-(3-oxo-morpholin-4-yl)-phenyl]-3-methoxy-propionsäureamid, ESI 472;
(R)-2-[(5-Chlor-thiophen-2-carbonyl)-amino]-*N*-[3-fluor-4-(3-oxo-morpholin-4-yl)-phenyl]-3-methoxy-propionsäureamid, ESI 456;
(R)-2-[(5-Chlor-thiophen-2-carbonyl)-amino]-*N*-[3-chlor-4-(2-oxo-2*H-*pyridin-1-yl)-phenyl]-3-methoxy-propionsäureamid, ESI 466;
(R)-2-[(5-Chlor-thiophen-2-carbonyl)-amino]-*N*-[3-methyl-4-(3-oxo-morpholin-4-yl)-phenyl]-3-allyl-propionsäureamid, ESI 478;
(R)-2-[(5-Chlor-thiophen-2-carbonyl)-amino]-*N*-[3-methyl-4-(3-oxo-morpholin-4-yl)-phenyl]-3-propoxy-propionsäureamid, ESI 480;
(R)-2-[(5-Chlor-thiophen-2-carbonyl)-amino]-*N*-[3-methyl-4-(3-oxo-morpholin-4-yl)-phenyl]-3-ethoxy-propionsäureamid, ESI 466;
(R)-2-[(5-Chlor-thiophen-2-carbonyl)-amino]-*N*-[3-methyl-4-(3-oxo-morpholin-4-yl)-phenyl]-3-(2-methoxyethoxy)-propionsäureamid, ESI 496;
(2R,3R)-2-[(5-Chlor-thiophen-2-carbonyl)-amino]-*N*-[3-methyl-4-(3-oxo-morpholin-4-yl)-phenyl]-3-ethoxy-buttersäureamid, ESI 480;
(2R,3R)-2-[(5-Chlor-thiophen-2-carbonyl)-amino]-*N*-[3-methyl-4-(3-oxo-morpholin-4-yl)-phenyl]-3-(2-methoxyethoxy)-buttersäureamid, ESI 510.

### Beispiel 2

Die Herstellung von (R)-2-[(4-Chlorphenyl-carbonyl)-amino]-*N*-[4-(3-Oxo-morpholin-4-yl)-phenyl]-4-methyl-valeriansäureamid, erfolgt analog nachstehendem Schema:

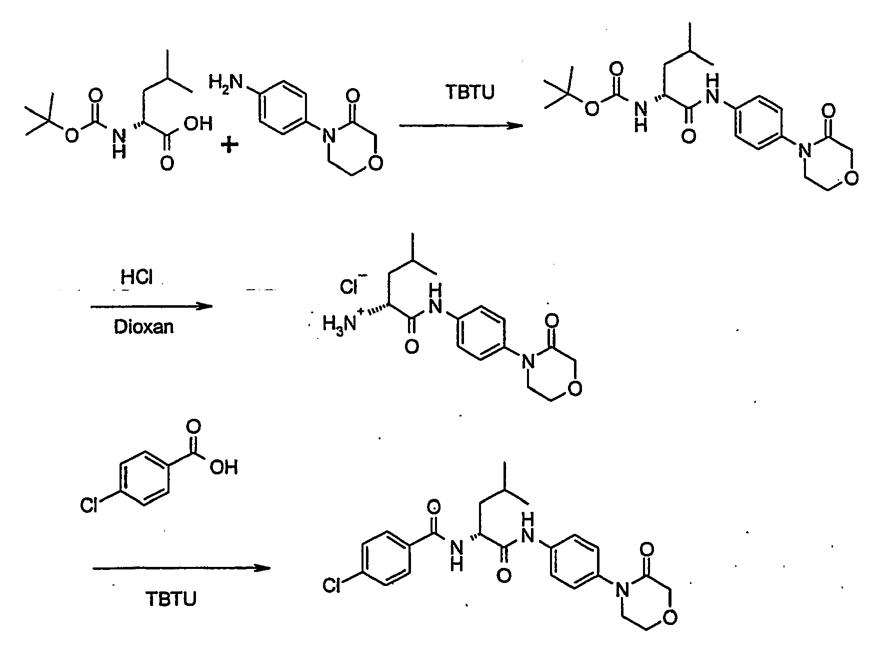

Eine Lösung von 2.28 g (9.15 mmol) Boc-D-Leucin Hydrat und 1.76 g (27.5 mmol) 4-(4-Aminophenyl)-morpholin-3-on in 10 ml DMF wird mit 3.52 g (11.0 mmol) 2-(1*H*-Benzotriazol-1-yl)-1,1,3,3-tetramethyluronium-tetrafluoroborat (TBTU) versetzt und 18 Stunden bei Raumtemperatur gerührt. Das Reaktionsgemisch wird mit gesättigter wässriger Natriumhydrogencarbonatlösung versetzt, der entstandene Niederschlag abfiltriert, mit Wasser gewaschen und getrocknet: (R)-{3-Methyl-1-[4-(3-oxo-morpholin-4-yl)-phenylcarbamoyl]-butyl}-carbaminsäure-*tert*-butylester als farbloser Feststoff; ESI 406.

1.10 g (2.71 mmol) (R)-{3-Methyl-1-[4-(3-oxo-morpholin-4-yl)-phenylcarbamoyl]-butyl}-carbaminsäure-*tert*-butylester wird mit 20 ml 4 N HCl in Dioxan versetzt und 18 Stunden bei Raumtemperatur gerührt. Das Reaktionsgemisch wird eingedampft: (R)-2-Amino-4-methyl-pentansäure-[4-(3-oxo-morpholin-4-yl)-phenyl]-amid Hydrochlorid als leicht rötlicher Feststoff; ESI 306.

Eine Lösung von 140 mg (0.410 mmol) (R)-2-Amino-4-methyl-pentansäure-[4-(3-oxo-morpholin-4-yl)-phenyl]-amid Hydrochlorid und 64.2 mg (0.410 mmol) 4-Chlorbenzoesäure in 2 ml Dimethylformamid (DMF) wird mit 54.6 mg (0.540 mmol) 4-Methylmorpholin und 173 mg (0.540 mmol) 2-(1*H*-Benzotriazol-1-yl)-1,1,3,3-tetramethyluronium-tetrafluoroborat (TBTU) versetzt und 18 Stunden bei Raumtemperatur gerührt. Das Reaktionsgemisch wird mit gesättigter wässriger Natriumhydrogencarbonatlösung versetzt, der entstandene Niederschlag abfiltriert, mit Wasser gewaschen und getrocknet: (R)-2-[(4-Chlorphenyl-carbonyl)-amino]-*N*-[4-(3-oxo-morpholin-4-yl)-phenyl]-4-methyl-valeriansäureamid als farbloser Feststoff; ESI 444.

Analog erhält man
(R)-2-[(4-Chlorphenyl-carbonyl)-amino]-*N*-[3-methyl-4-(3-oxo-morpholin-4-yl)-phenyl]-4-methyl-valeriansäureamid.

### Beispiel 2-1

Die Herstellung von (R)-2-[(5-Chlor-thiophen-2-carbonyl)-amino]-*N*-[3-methyl-4-(3-oxo-morpholin-4-yl)-phenyl]-3-methylsulfonyl-propionsäureamid, ESI 500; erfolgt analog nachstehendem Schema, wobei die zu oxidierende Sulfanilverbindung analog Beispiel 1 erhalten wird:

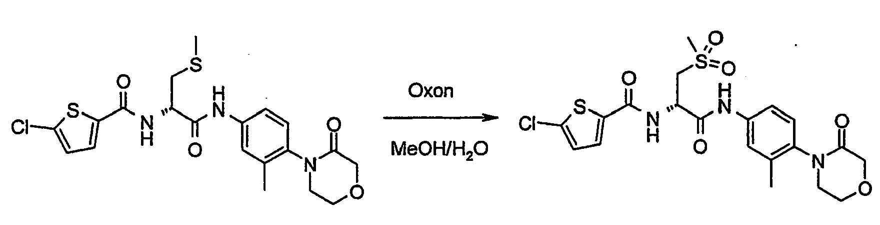

Analog erhält man nachstehende Verbindungen
(R)-2-[(5-Chlor-thiophen-2-carbonyl)-amino]-*N*-[4-(2-oxo-2*H*-pyridin-1-yl)-phenyl]-3-methylsulfonyl-propionsäureamid, ESI 480;
(R)-2-[(5-Chlor-thiophen-2-carbonyl)-amino]-*N*-[4-(3-oxo-morpholin-4-yl)-phenyl]-3-methylsulfonyl-propionsäureamid, ESI 486;
(R)-2-[(5-Chlor-thiophen-2-carbonyl)-amino]-*N*-[4-(3-oxo-morpholin-4-yl)-phenyl]-3-methylsulfonyl-buttersäureamid, ESI 500;
(R)-2-[(5-Chlor-thiophen-2-carbonyl)-amino]-*N*-[3-methyl-4-(3-oxo-morpholin-4-yl)-phenyl]-3-methylsulfonyl-buttersäureamid, ESI 514;
(R)-2-[(5-Chlor-thiophen-2-carbonyl)-amino]-*N*-[4-(2-oxo-2*H*-pyridin-1-yl)-phenyl]-3-methylsulfonyl-buttersäureamid, ESI 494;

### Beispiel 2-2

Die Herstellung von (R)-2-[(5-Chlor-thiophen-2-ylmethyl)-amino]-*N*-[4-(3-oxo-morpholin-4-yl)-phenyl]-valeriansäureamid erfolgt analog nachstehendem Schema:

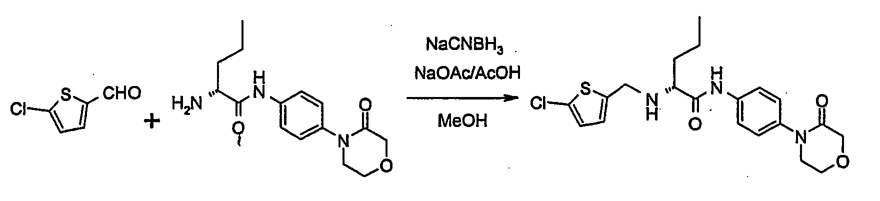

Eine Lösung von 120 mg (0.819 mmol) 5-Chlor-2-thiophencarboxaldehyd in 5 ml Methanol wird bei Raumtemperatur unter Stickstoff mit 230 mg (0.789 mmol) (R)-2-Aminopentansäure-[4-(3-oxo-morpholin-4-yl)-phenyl]-amid, 70 mg (0.853 mmol) Natriumacetat und 48 µl (0.839 mmol) Essigsäure versetzt und 30 min bei Raumtemperatur gerührt. Zu dieser Lösung wird langsam 52.0 mg (0.827 mmol) Natriumcyanoborhydrid gegeben und die entstandene Suspension 24 Stunden bei Raumtemperatur gerührt. Das Reaktionsgemisch wird eingeengt und zwischen Ethylacetat und verdünnter Natriumhydrogencarbonatlösung verteilt. Die organische Phase wird über Natriumsulfat getrocknet und eingedampft: (R)-2-([(5-Chlor-thiophen-2-ylmethyl)-amino]-*N*-[4-(3-oxo-morpholin-4-yl)-phenyl]- valeriansäureamid als farbloses Öl; ESI 422.

### Beispiel 2-3

Die Herstellung von (R)-2-[(5-Chlor-thiophen-2-carbonyl)-amino]-*N*-[4-(3-oxo-morpholin-4-yl)-phenyl]-3-carboxy-propionsäureamid, ESI 452, erfolgt analog nachstehendem Schema:

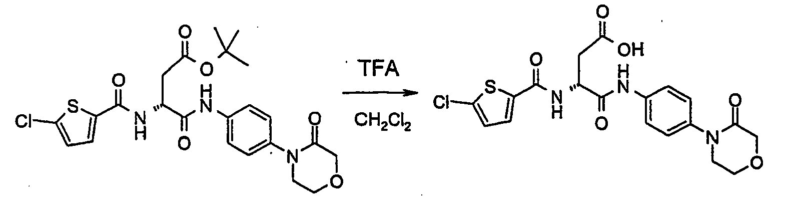

Analog erhält man nachstehende Verbindungen
(R)-2-[(5-Chlor-thiophen-2-carbonyl)-amino]-*N*-[3-methyl-4-(3-oxo-morpholin-4-yl)-phenyl]-3-carboxy-propionsäureamid, ESI 466;
(R)-2-[(5-Chlor-thiophen-2-carbonyl)-amino]-*N*-[4-(3-oxo-morpholin-4-yl)-phenyl]-4-carboxy-buttersäureamid, ESI 466;
(R)-2-[(5-Chlor-thiophen-2-carbonyl)-amino]-*N*-[3-methyl-4-(3-oxo-morpholin-4-yl)-phenyl]-4-carboxy-buttersäureamid, ESI 480.

### Beispiel 2-4

Die Herstellung von (R)-2-[(5-Chlor-thiophen-2-carbonyl)amino]-*N*-[4-(3-oxo-morpholin-4-yl)-phenyl]-4-amino-buttersäureamid, Trifluoracetat, ESI 437, erfolgt analog nachstehendem Schema:

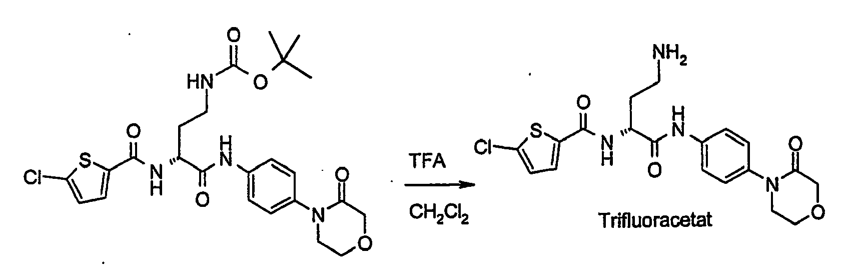

Analog erhält man die nachstehenden Verbindungen
(R)-2-[(5-Chlor-thiophen-2-carbonyl)-amino]-*N*-[3-methyl-4-(3-oxo-morpholin-4-yl)-phenyl]-4-amino-buttersäureamid, Trifluoracetat, ESI 451;
(R)-2-[(5-Chlor-thiophen-2-carbonyl)-amino]-*N*-[4-(3-oxo-morpholin-4-yl)-phenyl]-5-amino-valeriansäureamid, Trifluoracetat, ESI 451;
(R)-2-[(5-Chlor-thiophen-2-carbonyl)-amino]-*N*-[3-methyl-4-(3-oxo-morpholin-4-yl)-phenyl]-5-amino-valeriansäureamid, Trifluoracetat, ESI 465;
(R)-2-[(5-Chlor-thiophen-2-carbonyl)-amino]-*N*-[4-(3-oxo-morpholin-4-yl)-phenyl]-3-amino-propionsäureamid, Trifluoracetat, ESI 423;
(R)-2-[(5-Chlor-thiophen-2-carbonyl)-amino]-*N*-[3-methyl-4-(3-oxo-morpholin-4-yl)-phenyl]-3-amino-propionsäureamid, Trifluoracetat, ESI 437.

### 3. Beispiele zur Herstellung von Zwischenverbindungen

### 3.1 Nach folgendem Schema lassen sich alle Verbindungen der folgenden Formel VI (mit R = H oder Methyl; n = 3, 4 oder 5) synthetisieren.

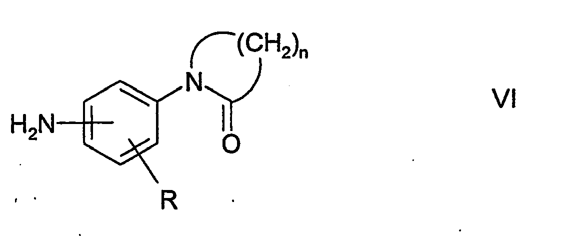

Z.B. Synthese von 1-(4-Amino-2-methylphenyl)-piperidin-2-on:

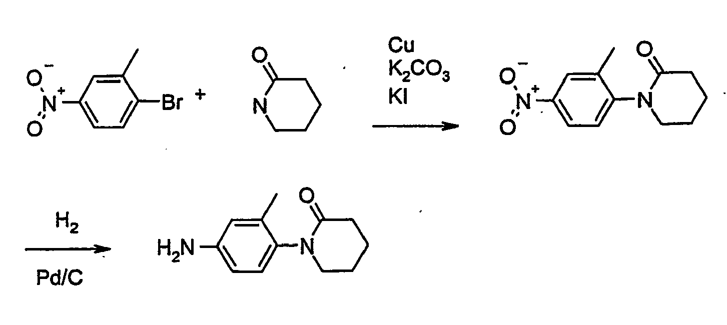

### 3.2 Synthese des Phenylpiperidonbausteins ohne Methylgruppe:

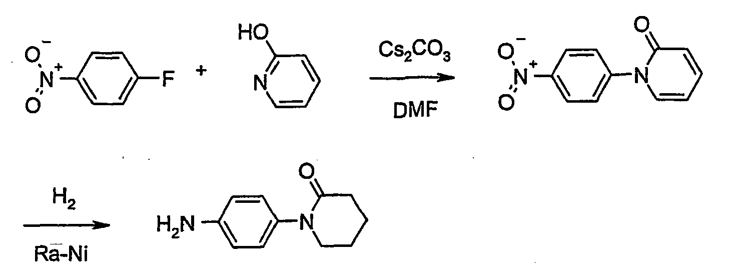

Die Herstellung von 1-(4-Amino-2-methyl-phenyl)-piperidin-2-on erfolgt z.B. wie nachfolgend angegeben:

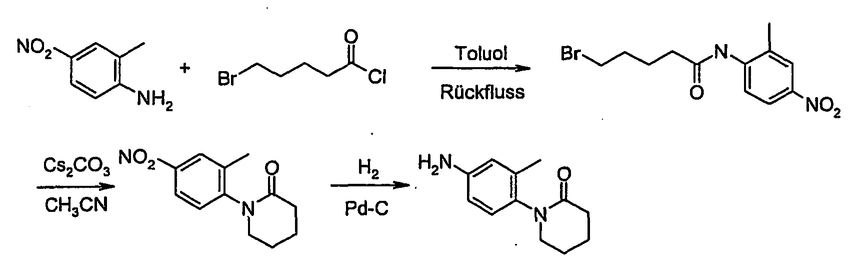

### 3.3 1-(4-Amino-phenyl)-1H-pyrazin-2-on

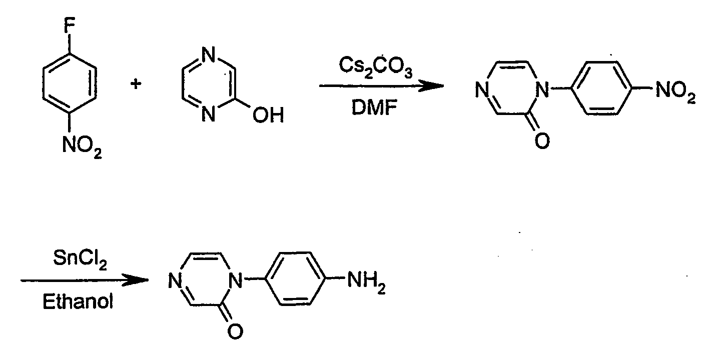

### 3.4 1-(4-Amino-2,5-dimethyl-phenyl)-piperidin-2-on

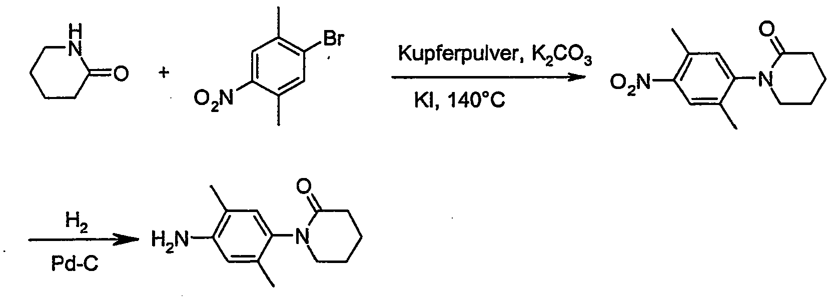

### 3.5 1-(4-Amino-3-methyl-phenyl)-piperidin-2-on

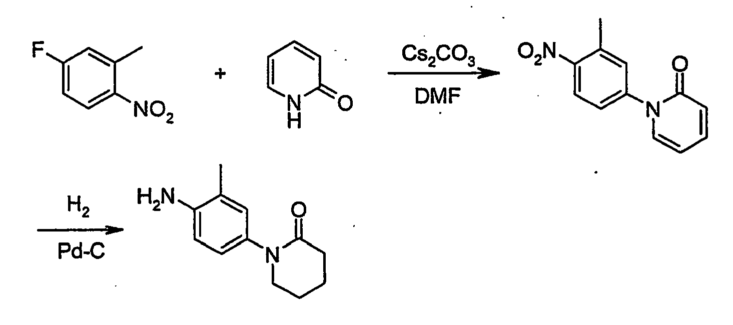

### 3.6 1-(5-Amino-pyridin-2-yl)-piperidin-2-on

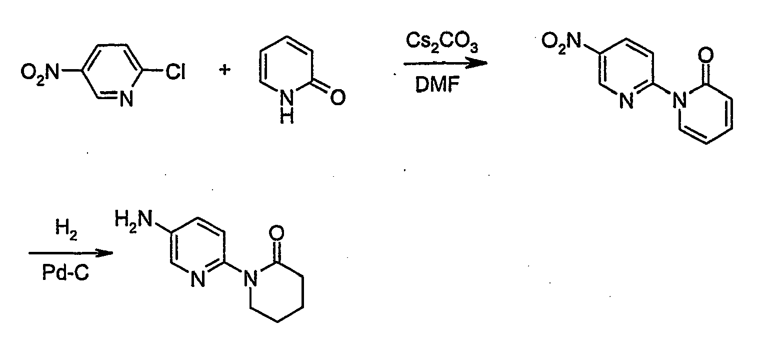

### 3.7 1-(4-Aminomethyl-phenyl)-piperidin-2-on

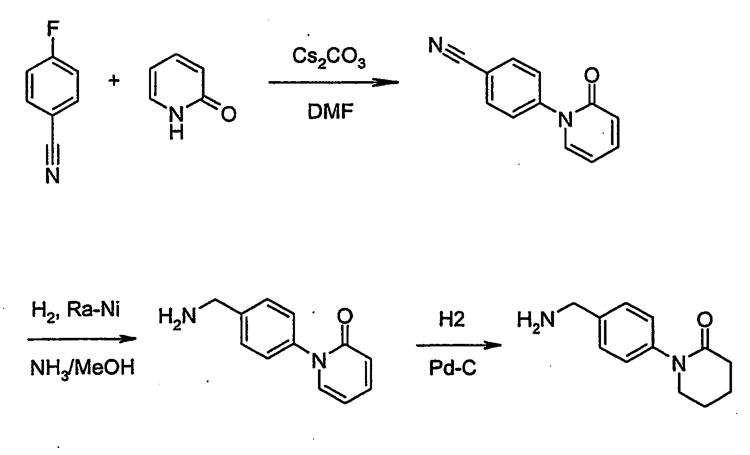

### 3.8 2-(4-Amino-phenyl)-2-aza-bicyclo[2.2.2]octan-3-on

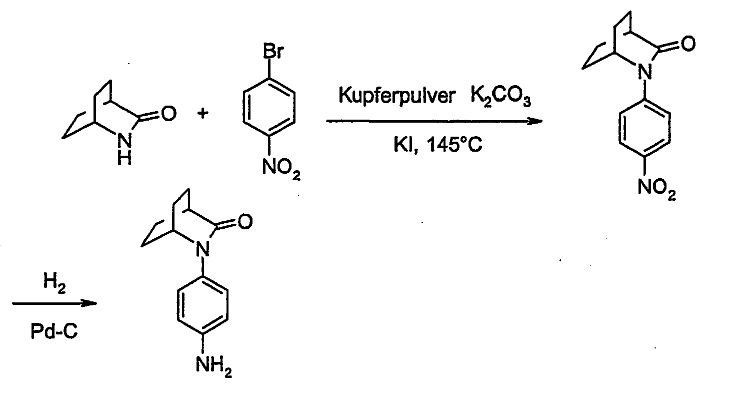

### 3.9 1-(3-Amino-6-ethyl-phenyl)-pyrrolidin-2-on

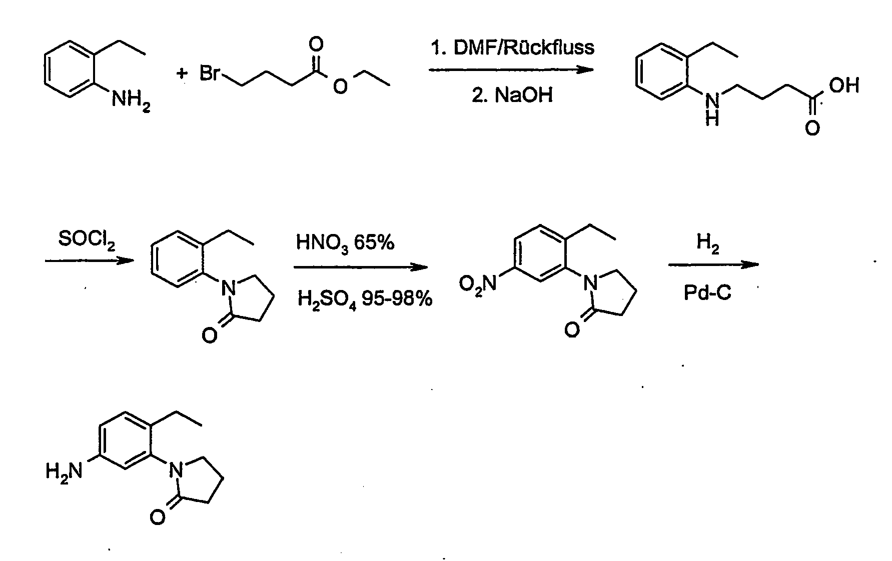

### 3.10 2-(4-Amino-2-trifluormethyl-phenyl)-2-aza-bicyclo[2.2.2]octan-3-on

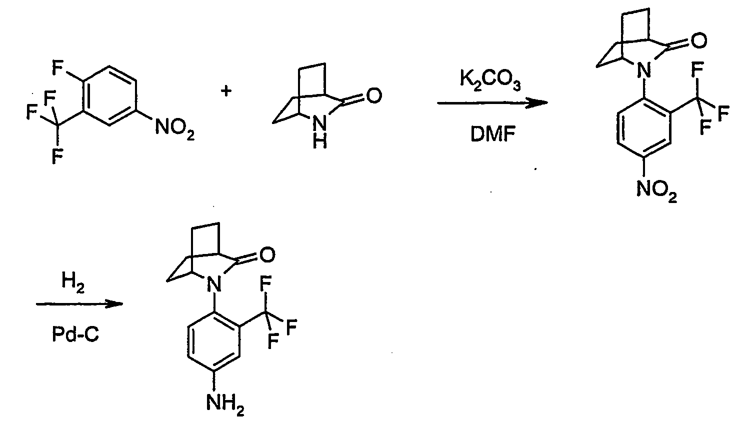

### 3.11 1-(4-Amino-3-chlor-phenyl)-pyrrolidin-2-on

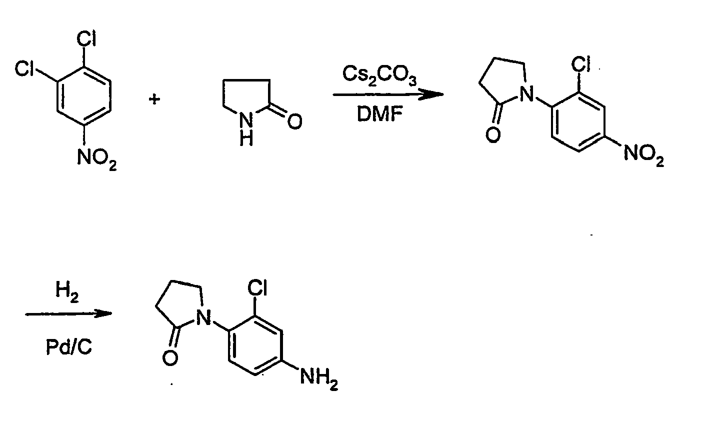

### 3.12 1-(4-Amino-2-trifluormethyl-phenyl)-piperidin-2-on

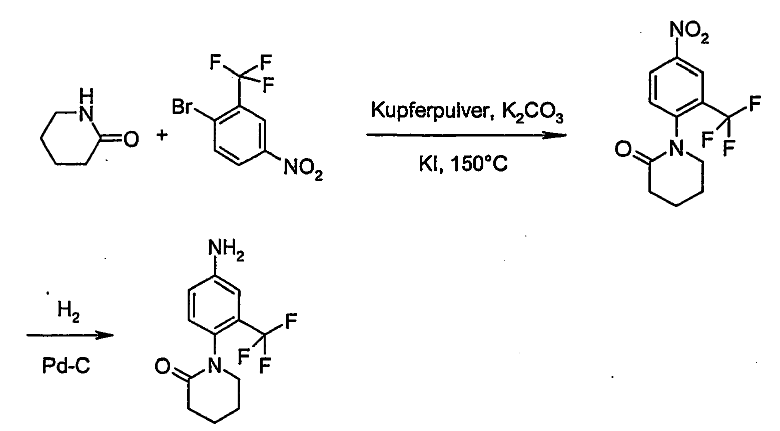

### 3.13 3-(4-Amino-2-methyl-phenyl)-[1,3]oxazinan-2-on

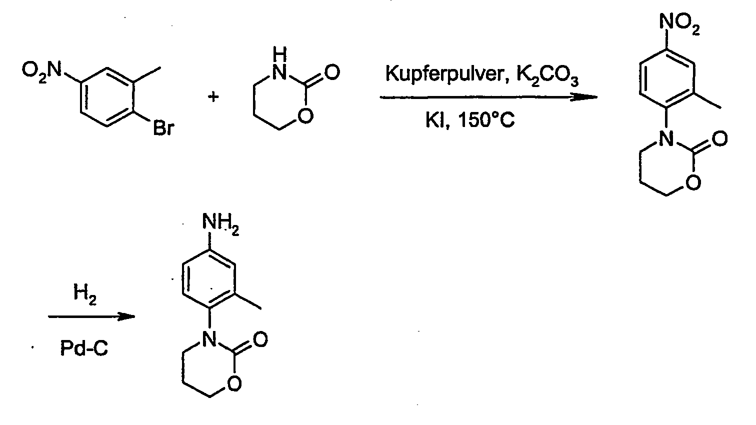

### 3.14 4-(4-Amino-phenyl)-morpholin-3-on

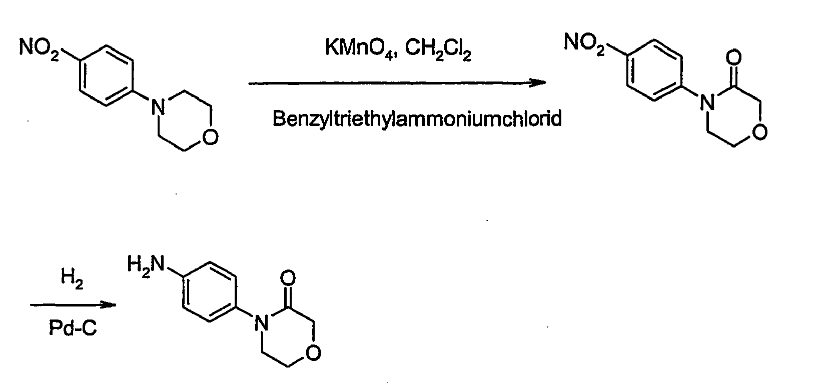

### 3.15 1-(4-Amino-phenyl)-pyridin-2-on

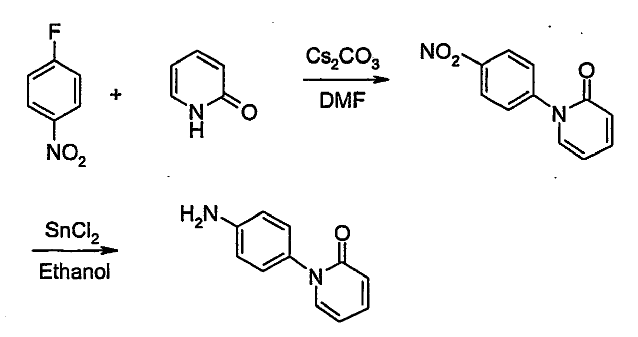

### 3.16 1-(4-Amino-2-methyl-phenyl)-piperidin-2-on

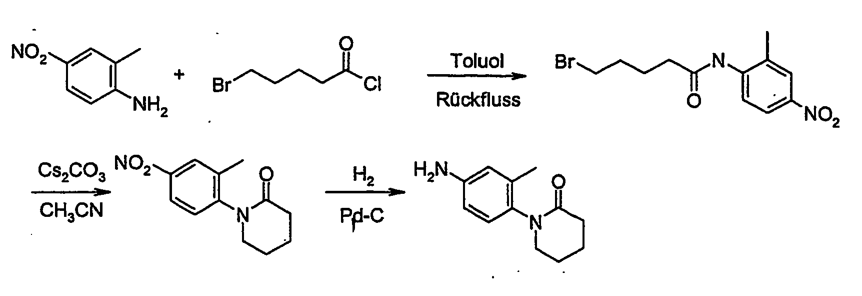

### 3.17 1-(4-Amino-phenyl)-1H-pyridin-4-on

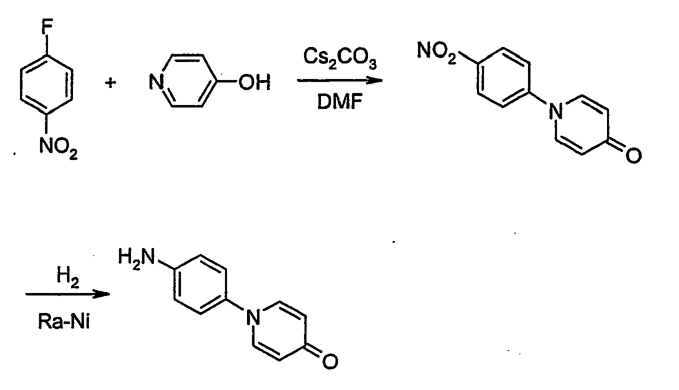

### 3.18 1-(4-Amino-phenyl)-4-tert.-butyloxycarbonyl-piperazin-2-on

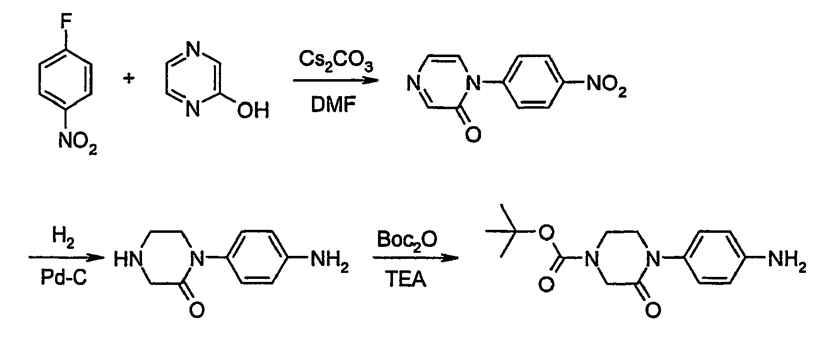

### 3:19 1-(3-Aminophenyl)-piperidin-2-on

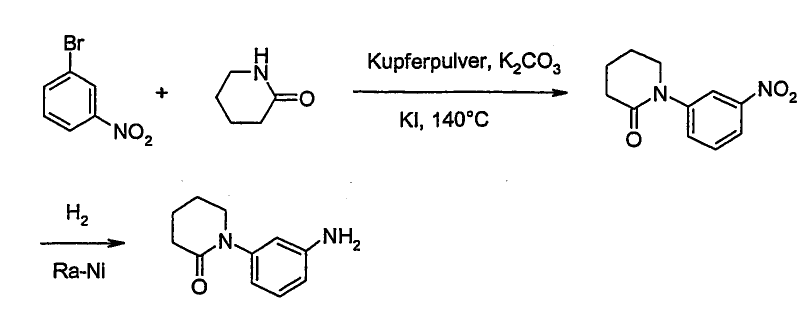

### 3.20 1-(4-Amino-phenyl)-2-caprolactam

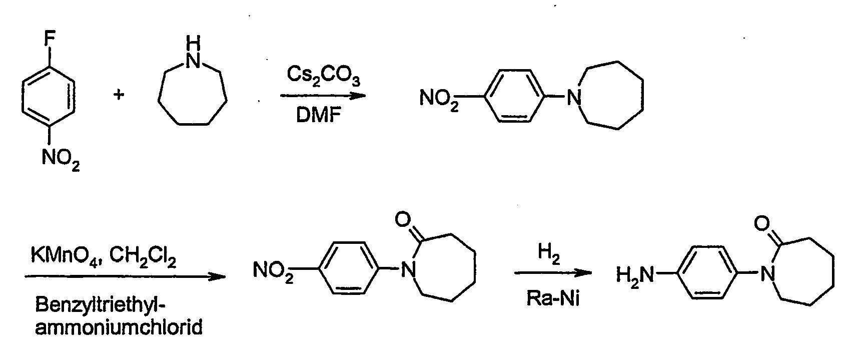

### 3.21 1-(4-Amino-3-fluor-phenyl)-piperidin-2-on

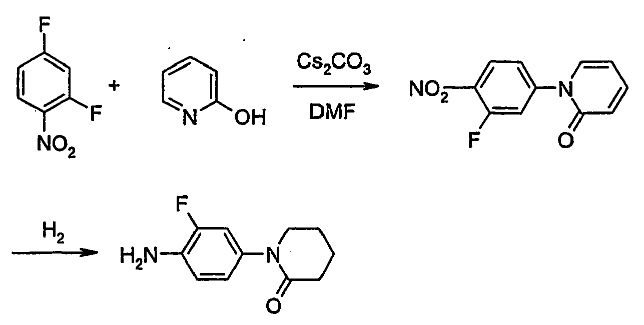

### 3.22 1-(4-Amino-2-fluor-phenyl)-piperidin-2-on

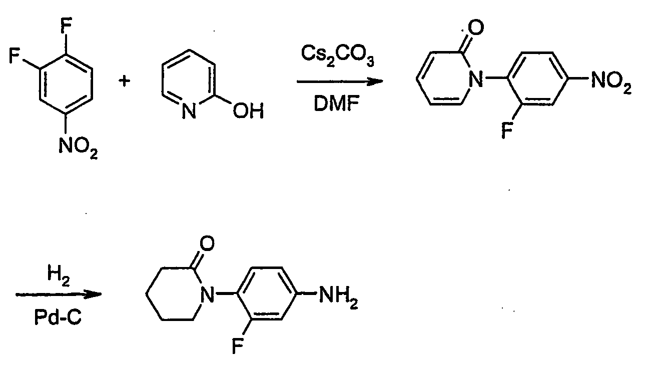

### 3.23 1-(4-Amino-2-fluor)-2-caprolactam

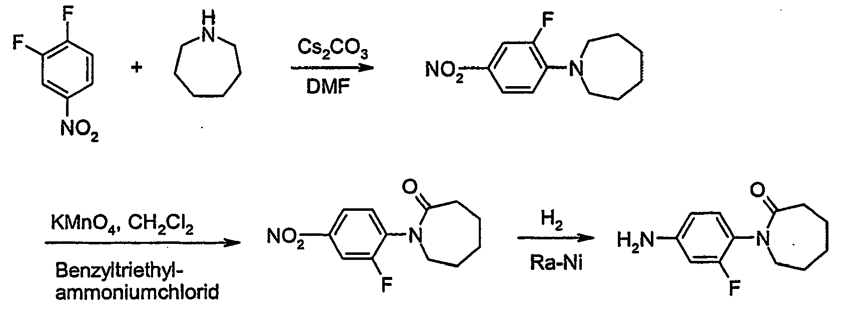

### 3.24 4-(2-Imino-piperidin-1-yl)-anilin, Hydrochlorid

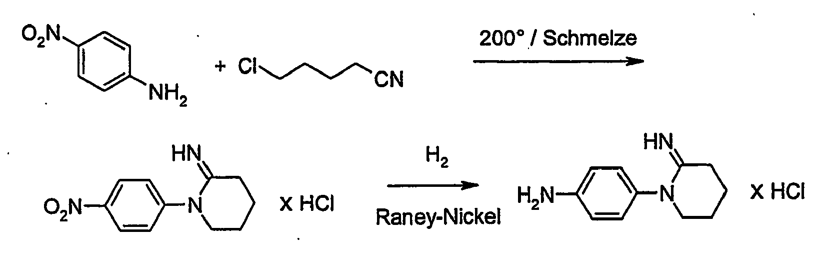

### Beispiel 4

Die Herstellung von (R)-2-[(5-Chlor-thiophen-2-carbonyl)-amino]-*N*-[4-(2-imino-piperidin-1-yl)-phenyl]-4-methyl-valeriansäureamid und (S)-2-[(5-Chlor-thiophen-2-carbonyl)-amino]-*N*-[4-(2-imino-piperidin-1-yl)-phenyl]-4-methyl-valeriansäureamid erfolgt analog nachstehendem Schema: 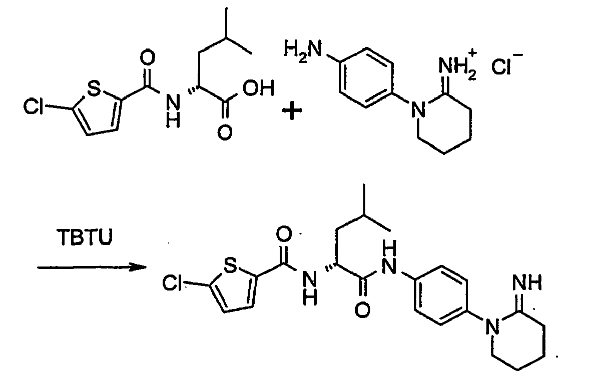
(R)-2-[(5-Chlor-thiophen-2-carbonyl)-amino]-*N*-[4-(2-imino-piperidin-1-yl)-phenyl]-4-methyl-valeriansäureamid ("4A"), ESI 447;
(S)-2-[(5-Chlor-thiophen-2-carbonyl)-amino]-*N*-[4-(2-imino-piperidin-1-yl)-phenyl]-4-methyl-valeriansäureamid ("4B"), ESI 447.

### Beispiel 5

Die Herstellung von (R)-2-[(5-Chlor-thiophen-2-carbonyl)-amino]-*N*-[3-methyl-4-(3-oxo-morpholin-4-yl)-phenyl]-3-hydroxy-propionsäureamid erfolgt wie nachstehend angegeben:

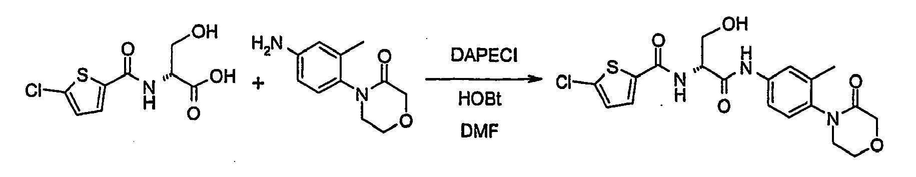

Eine Lösung von 250 mg (1.00 mmol) (R)-2-[(5-Chlorthiophen-2-carbonyl)-amino]-3-hydroxypropionsäure, 206 mg (1.00 mmol) 4-(4-Amino-2-methylphenylrmorpholin-3-on und 169 mg (1.10 mmol) Hydroxybenztriazol-Hydrat in 6 ml DMF wird bei Raumtemperatur mit 211 mg (1.10 mmol) N-(3-Dimethylaminopropyl)-N'-ethylcarbodiimid-hydrochlorid (DAPECI) versetzt und 48 Stunden bei Raumtemperatur gerührt. Zum Reaktionsgemisch werden 100 ml verdünnte Natriumhydrogencarbonatlösung gegeben und der entstandene Niederschlag abfiltriert und getrocknet: (R)-2-[(5-Chlor-thiophen-2-carbonyl)-amino]-N-[3-methyl-4-(3-oxo-morpholin-4-yl)-phenyl]-3-hydroxy-propionsäureamid als farbloser Feststoff; ESI 438.

Analog erhält man nachstehende Verbindungen
(R)-2-[(5-Chlor-thiophen-2-carbonyl)-amino]-*N*-[3-4-(3-oxo-morpholin-4-yl)-phenyl]-3-hydroxy-propionsäureamid, F. 227-233°;
(2R,3R)-2-[(5-Chlor-thiophen-2-carbonyl)-amino]-*N*-[4-(3-oxo-morpholin-4-yl)-phenyl]-3-hydroxy-buttersäureamid, F. 198-200°.

### Beispiel 6

Die Herstellung von (2R,3R)-2-[(5-Chlor-thiophen-2-carbonyl)-amino]-*N*-[4-(3-oxo-morpholin-4-yl)-phenyl]-3-aminocarbonyloxy-buttersäureamid, ESI 481 erfolgt wie nachstehend angegeben:

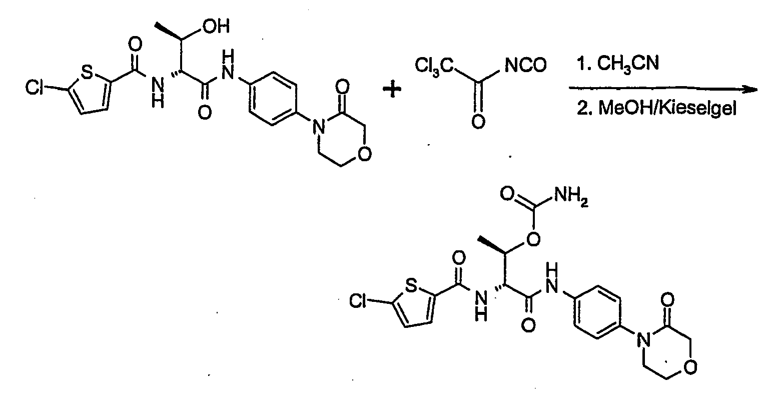

Analog erhält man die nachstehenden Verbindungen
(2R)-2-[(5-Chlor-thiophen-2-carbonyl)amino]-*N*-[3-methyl-4-(3-oxo-morpholin-4-yl)-phenyl]-3-aminocarbonyloxy-propionsäureamid, F. 211-215°;
(2R,3R)-2-[(5-Chlor-thiophen-2-carbonyl)-amino]-*N*-[3-methyl-4-(3-oxo-morpholin-4-yl)-phenyl]-3-aminocarbonyloxy-buttersäureamid, F. 167-170°.

### Pharmakologische Daten (Affinität zu Rezeptoren)

| Verbindung Nr. | FXa-IC₅₀ [M] | TF/FVIIa-IC₅₀ [M] |
|---|---|---|
| "1A" | 7.3 x 10⁻⁹ | 6.9 x 10⁻⁹ |
| "2A" | 3.9 x 10⁻⁸ | |
| "4A" | 2.5 x 10⁻⁸ | |
| "4B" | 2.6 x 10⁻⁷ | |

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 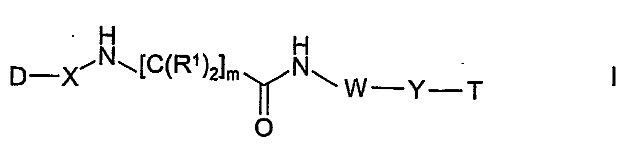 worin
D unsubstituierter oder ein- oder mehrfach durch Hal, A, OR², N(R²)₂, NO₂, CN, COOR² oder CON(R²)₂ substituierter aromatischer Carbo- oder Heterocyclus mit 0 bis 4 N-, O- und/oder S-Atomen,
X -C=O oder C(R³)₂,
W -[C(R³)₂]ₙ-,
R¹ H oder A, das durch OR³, S(O)ₙR³, N(R³)₂, CN, COOR³, CON(R³)₂, OCON(R³)₂, N(R³)COOR³, N(R³)CON(R³)₂, N(R³)SO₂R³, SO₂N(R³)₂ oder -C≡C- substituiert sein kann,
R² H, A, -[C(R³)₂]ₙ-Ar', -[C(R³)₂]ₙ-Het', -[C(R³)₂]ₙ-Cycloalkyl, -[C(R³)₂]ₙ-N(R³)₂ oder -[C(R³)₂]ₙ-OR³,
R³ H oder A,
Y Alkylen, Cycloalkylen, Het-diyl oder Ar-diyl,
T einen ein- oder zweikernigen gesättigten, ungesättigten oder aromatischen Carbo- oder Heterocyclus mit 0 bis 4 N-, O- und/oder S-Atomen, der ein- oder zweifach durch =O, =S, =NR², =N-CN, =N-NO₂, =NOR², =NCOR², =NCOOR², =NOCOR² substituiert ist und ferner ein-, zwei- oder dreifach durch R², Hal, A, -[C(R³)₂]ₙ-Ar, -[C(R³)₂]ₙ-Het, -[C(R³)₂]ₙ-Cycloalkyl, OR², N(R²)₂, NO₂, CN, COOR², CON(R²)₂, NR²COA, NR²CON(R²)₂, NR²SO₂A, COR², SO₂NR² und/oder S(O)ₙA substituiert sein kann,
A unverzweigtes oder verzweigtes Alkyl mit 1-10 C-Atomen, worin eine oder zwei CH₂-Gruppen durch O- oder S-Atome und/oder durch -CH=CH-Gruppen und/oder auch 1-7 H-Atome durch F ersetzt sein können,
Ar unsubstituiertes oder ein-, zwei- oder dreifach durch Hal, A, OR², N(R²)₂, NO₂, CN, COOR², CON(R²)₂, NR²COA, NR²CON(R²)₂, NR²SO₂A, COR², SO₂N(R²)₂, S(O)ₙA, -[C(R³)₂]ₙ-COOR² oder -O-[C(R³)₂]ₒ-COOR² substituiertes Phenyl, Naphthyl oder Biphenyl,
Ar' unsubstituiertes oder ein-, zwei- oder dreifach durch Hal, A, OR³, N(R³)₂, NO₂, CN, COOR³, CON(R³)₂, NR³COA, NR³CON(R³)₂, NR³SO₂A, COR³, SO₂N(R³)2, S(O)ₙA, -[C(R³)₂]ₙ-COOR³ oder -O-[C(R³)₂]ₒ-COOR³ substituiertes Phenyl,
Het einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Carbonylsauerstoff, =S, =N(R²)₂, Hal, A, -[C(R³)₂]ₙ-Ar, -[C(R³)₂]ₙ-Het', -[C(R³)₂]ₙ-Cycloalkyl, -[C(R³)₂]ₙ-OR², -[C(R³)₂]ₙ-N(R³)₂, NO₂, CN, -[C(R³)₂]ₙ-COOR^{2'} -[C(R³)₂]ₙ-CON(R²)₂, -[C(R³)₂]ₙ-NR²COA, NR²CON(R²)₂, -[C(R³)₂]ₙ-NR²SO₂A, COR², SO₂NR² und/oder S(O)ₙA substituiert sein kann,
Het' einen ein- oder zweikernigen gesättigten, ungesättigten oder aromatischen Heterocyclus mit 1 bis 4 N-, O- und/oder S-Atomen, der unsubstituiert oder ein- oder zweifach durch Carbonylsauerstoff, =S, =N(R³)₂, Hal, A, OR³, N(R³)₂, NO₂, CN, COOR³, CON(R³)_{2,} NR³COA, NR³CON(R³)₂, NR³SO₂A, COR³, SO₂NR³ und/oder S(O)ₙA substituiert sein kann,
Hal F, Cl, Br oder I,
m 1 oder 2,
n 0, 1 oder 2,
o 1, 2 oder 3
bedeuten,
sowie ihre pharmazeutisch verwendbaren Salze, Solvate und Stereoisomere, einschließlich deren Mischungen in allen Verhältnissen.

2. Verbindungen nach Anspruch 1, worin
D unsubstituierter oder ein- oder zweifach durch Hal substituierter aromatischer Fünfringheterocyclus mit 1 bis 2 N-, O- und/oder S-Atomen,
bedeutet,
sowie ihre pharmazeutisch verwendbaren Salze, Solvate und Stereoisomere, einschließlich deren Mischungen in allen Verhältnissen.

3. Verbindungen nach Anspruch 1 oder 2, worin
D ein- oder zweifach durch Hal substituierter Thienylring
bedeutet,
sowie ihre pharmazeutisch verwendbaren Salze, Solvate und Stereoisomere, einschließlich deren Mischungen in allen Verhältnissen.

4. Verbindungen gemäß einem oder mehreren der Ansprüche 1-3 , worin
R² H oder Alkyl mit 1, 2, 3, 4, 5 oder 6 C-Atomen bedeutet,
sowie ihre pharmazeutisch verwendbaren Salze, Solvate und Stereoisomere, einschließlich deren Mischungen in allen Verhältnissen.

5. Verbindungen gemäß einem oder mehreren der Ansprüche 1-4, worin
R¹ H oder A, das durch OR³, CON(R³)₂, N(R³)₂, S(O)ₙR³, COOR³, OCON(R³)₂, N(R³)COOR³ oder -C≡C- substituiert sein kann,
bedeutet,
sowie ihre pharmazeutisch verwendbaren Salze, Solvate und Stereoisomere, einschließlich deren Mischungen in allen Verhältnissen.

6. Verbindungen gemäß einem oder mehreren der Ansprüche 1-5, worin
X -C=O bedeutet,
sowie ihre pharmazeutisch verwendbaren Salze, Solvate und Stereoisomere, einschließlich deren Mischungen in allen Verhältnissen.

7. Verbindungen gemäß einem oder mehreren der Ansprüche 1-6, worin
W fehlt,
bedeutet,
sowie ihre pharmazeutisch verwendbaren Salze, Solvate und Stereoisomere, einschließlich deren Mischungen in allen Verhältnissen.

8. Verbindungen gemäß einem oder mehreren der Ansprüche 1-7, worin
Y Ar-diyl bedeutet,
sowie ihre pharmazeutisch verwendbaren Salze, Solvate und Stereoisomere, einschließlich deren Mischungen in allen Verhältnissen.

9. Verbindungen gemäß einem oder mehreren der Ansprüche 1-8, worin
T einen ein- oder zweikernigen gesättigten, ungesättigten oder aromatischen Heterocyclus mit 1 bis 2 N- und/oder O-Atomen, der ein- oder zweifach durch =O, =S, =NR², =N-CN, =N-NO₂, =NOR², =NCOR², =NCOOR² oder =NOCOR² substituiert ist und ferner ein- oder zweifach durch Hal oder A substituiert sein kann,
bedeutet,
sowie ihre pharmazeutisch verwendbaren Salze, Solvate und Stereoisomere, einschließlich deren Mischungen in allen Verhältnissen.

10. Verbindungen gemäß einem oder mehreren der Ansprüche 1-9, worin
T einen ein- oder zweikernigen gesättigten oder ungesättigten Heterocyclus mit 1 bis 2 N- und/oder O-Atomen, der ein- oder zweifach durch =O, =S oder =NH substituiert ist,
bedeutet,
sowie ihre pharmazeutisch verwendbaren Salze, Solvate und Stereoisomere, einschließlich deren Mischungen in allen Verhältnissen.

11. Verbindungen gemäß einem oder mehreren der Ansprüche 1-10, worin
T ein- oder zweifach durch =O oder =NH substituiertes Piperidin-1-yl, Pyrrolidin-1-yl, Pyridin-1-yl, Morpholin-4-yl, Piperazin-1-yl, 1,3-Oxazolidin-3-yl, Pyridazin-2-yl, Pyrazin-1-yl, Azepan-1-yl oder 2-Aza-bicyclo[2.2.2]-octan-2-yl,
bedeutet,
sowie ihre pharmazeutisch verwendbaren Salze, Solvate und Stereoisomere, einschließlich deren Mischungen in allen Verhältnissen.

12. Verbindungen gemäß einem oder mehreren der Ansprüche 1-11, worin
Ar unsubstituiertes oder ein- oder zweifach durch Hal, A, OA, SO₂A, COOR², SO₂NH₂ oder CN substituiertes Phenyl,
bedeutet,
sowie ihre pharmazeutisch verwendbaren Salze, Solvate und Stereoisomere, einschließlich deren Mischungen in allen Verhältnissen.

13. Verbindungen gemäß einem oder mehreren der Ansprüche 1-12, worin
Ar unsubstituiertes oder ein- oder zweifach durch A und/oder Hal substituiertes Phenyl,
bedeutet,
sowie ihre pharmazeutisch verwendbaren Salze, Solvate und Stereoisomere, einschließlich deren Mischungen in allen Verhältnissen.

14. Verbindungen gemäß einem oder mehreren der Ansprüche 1-13, worin
D unsubstituierter oder ein- oder zweifach durch Hal substituierter aromatischer Fünfringheterocyclus mit 1 bis 2 N-, O- und/oder S-Atomen,
R¹ H oder A, das durch OR³, CON(R³)₂, N(R³)₂, S(O)ₙR³, COOR³, OCON(R³)2, N(R³)COOR³ oder -C=C- substituiert sein kann,
R² H oder Alkyl mit 1, 2, 3, 4, 5 oder 6 C-Atomen,
X -C=O oder CH₂,
W fehlt,
Y Ar-diyl,
Ar unsubstituiertes oder ein- oder zweifach durch A und/oder Hal substituiertes Phenyl,
T einen ein- oder zweikernigen gesättigten oder ungesättigten Heterocyclus mit 1 bis 2 N- und/oder O-Atomen, der ein- oder zweifach durch =O, =S oder =NH substituiert ist,
bedeuten,
sowie ihre pharmazeutisch verwendbaren Salze, Solvate und Stereoisomere, einschließlich deren Mischungen in allen Verhältnissen.

15. Verbindungen gemäß einem oder mehreren der Ansprüche 1-14, worin
D ein- oder zweifach durch Hal substituiertes Thienyl, Thiazolyl oder Furyl,
R¹ H oder A, das durch OR³, CON(R³)₂, N(R³)₂, S(O)ₙR³, COOR³, OCON(R³)₂, N(R³)COOR³ oder -C=C- substituiert sein kann,
R² H oder Alkyl mit 1, 2, 3, 4, 5 oder 6 C-Atomen,
X -C=O oder CH₂,
W fehlt,
Y Ar-diyl,
Ar unsubstituiertes oder ein- oder zweifach durch A und/oder Hal substituiertes Phenyl,
T ein- oder zweifach durch =O oder =NH substituiertes Piperidin-1-yl, Pyrrolidin-1-yl, Pyridin-1-yl, Morpholin-4-yl, Piperazin-1-yl, 1,3-Oxazolidin-3-yl, Pyridazin-2-yl, Pyrazin-1-yl, Azepan-1-yl oder 2-Aza-bicyclo[2.2.2]-octan-2-yl,
bedeuten,
sowie ihre pharmazeutisch verwendbaren Salze, Solvate und Stereoisomere, einschließlich deren Mischungen in allen Verhältnissen.

16. Verbindungen gemäß einem oder mehreren der Ansprüche 1-15, worin
D ein- oder zweifach durch Hal substituiertes Thienyl oder Phenyl,
R¹ H oder A, das durch OR³, CON(R³)₂, N(R³)₂, S(O)ₙR³, COOR³, OCON(R³)₂, N(R³)COOR³ oder -C≡C- substituiert sein kann,
R² H oder Alkyl mit 1, 2, 3, 4, 5 oder 6 C-Atomen,
R³ H oder Alkyl mit 1, 2, 3, 4, 5 oder 6 C-Atomen,
X -C=O oder CH₂,
W fehlt oder CH₂,
Y Ar-diyl,
A Alkyl mit 1, 2, 3, 4, 5 oder 6 C-Atomen, worin eine oder zwei CH₂-Gruppen durch O- oder S-Atome und/oder durch -CH=CH-Gruppen und/oder auch 1-7 H-Atome durch F ersetzt sein können,
Ar unsubstituiertes oder ein- oder zweifach durch A und/oder Hal substituiertes Phenyl,
T ein- oder zweifach durch =O oder =NH substituiertes Piperidin-1-yl, Pyrrolidin-1-yl, Pyridin-1-yl, Morpholin-4-yl, Piperazin-1-yl, 1,3-Oxazolidin-3-yl, Pyridazin-2-yl, Pyrazin-1-yl, Azepan-1-yl oder 2-Aza-bicyclo[2.2.2]-octan-2-yl,
bedeuten,
sowie ihre pharmazeutisch verwendbaren Salze, Solvate und Stereoisomere, einschließlich deren Mischungen in allen Verhältnissen.

17. Verbindungen gemäß Anspruch 1 ausgewählt aus der Gruppe
(S)-2-([(5-Chlor-thiophen-2-carbonyl)-amino]-*N*-[4-(3-oxo-morpholin-4-yl)-phenyl]-4-methyl-valeriansäureamid,
(S)-2-[(5-Chlor-thiophen-2-carbonyl)-amino]-*N*-[3-methyl-4-(3-oxo-morpholin-4-yl)-phenyl]-4-methyl-valeriansäureamid,
(S)-2-[(5-Chlor-thiophen-2-carbonyl)-amino]-N-[4-(2-oxo-2H-pyrazin-1-yl)-phenyl]-4-methyl-valeriansäureamid,
(R)-2-[(5-Chlor-thiophen-2-carbonyl)-amino]-*N*-[4-(3-oxo-morpholin-4-yl)-phenyl]-4-methyl-valeriansäureamid,
(R)-2-[(5-Chlor-thiophen-2-carbonyl)-amino]-*N*-[3-methyl-4-(3-oxo-morpholin-4-yl)-phenyl]-4-methyl-valeriansäureamid,
(R)-2-[(5-Chlor-thiophen-2-carbonyl)-amino]-*N*-[4-(2-oxo-2*H-*pyrazin-1-yl)-phenyl]-4-methyl-valeriansäureamid,
(R)-2-[(5-Chlor-thiophen-2-carbonyl)-amino]-*N*-[4-(2-oxo-2*H-*pyridin-1-yl)-phenyl]-4-methyl-valeriansäureamid,
(R)-2-[(5-Chlor-thiophen-2-carbonyl)-amino]-*N*-[4-(2-imino-piperidin-1-yl)phenyl]-4-methyl-valeriansäureamid,
(S)-2-[(5-Chlor-thiophen-2-carbonyl)-amino]*-N-*[4-(2-imino-piperidin-1-yl)-phenyl]-4-methyl-valeriansäureamid,
2-[(5-Chlor-thiophen-2-carbonyl)-amino]-*N*-[3-methyl-4-(2-oxo-piperidin-1-yl)-phenyl]-acetamid,
3-[(5-Chlor-thiophen-2-carbonyl)-amino]-*N*-[3-methyl-4-(2-oxo-piperidin-1-yl)-phenyl]-propionsäureamid,
(R)-2-[(5-Chlor-thiophen-2-carbonyl)amino]-*N*-[3-methyl-4-(3-oxo-morpholin-4-yl)-phenyl]-propionsäureamid,
(R)-2-[(5-Chlor-thiophen-2-carbonyl)amino]-*N*-[3-methyl-4-(3-oxo-morpholin-4-yl)-phenyl]-3-methyl-buttersäureamid,
(R)-2-[(5-Chlor-thiophen-2-carbonyl)-amino]-*N*-[3-methyl-4-(3-oxo-morpholin-4-yl)-phenyl]-buttersäureamid,
(R)-2-[(5-Chlor-thlophen-2-carbonyl)-amino]-*N*-[3-methyl-4-(3-oxo-morpholin-4-yl)-phenyl]-valeriansäureamid,
(R)-2-[(5-Chlor-thiophen-2-carbonyl)-amino]-*N*-[3-methyl-4-(3-oxo-morpholin-4-yl)-phenyl]-3-aminocarbonyl-propionsäureamid,
(R)-2-[(4-Chlorphenyl-carbonyl)-amino]-*N*-[4-(3-oxo-morpholin-4-yl)-phenyl]-4-methyl-valeriansäureamid,
(R)-2-[(4-Chlorphenyl-carbonyl)-amino]-*N*-[3-methyl-4-(3-oxo-morpholin-4-yl)-phenyl]-4-methyl-valeriansäureamid,
2-[(5-Chlor-thiophen-2-carbonyl)-amino]-*N*-[3-methyl-4-(3-oxo-morpholin-4-yl)-phenyl]-3-(N,N-dimethylamino)-propionsäureamid,
(R)-2-[(5-Brom-thiophen-2-carbonyl)-amino]-*N*-[4-(3-oxo-morpholin-4-yl)-phenyl]-4-methyl-valeriansäureamid,
(R)-2-[(5-Chlor-thiophen-2-carbonyl)-amino]-*N*-[4-(2-oxo-piperidin-1-yl)-benzyl]-4-methyl-valeriansäureamid,
2-[(5-Chlor-thiophen-2-methyl)-amino]-*N*-[4-(3-oxo-morpholin-4-yl)-phenyl]-4-methyl-valeriansäureamid,
(R)-2-[(5-Chlor-thiophen-2-carbonyl)-amino]-*N*-[4-(3-oxo-morpholin-4-yl)-phenyl]-3-methylsulfanyl-propionsäureamid,
(S)-2-[(5-Chlor-thiophen-2-carbonyl)-amino]-*N*-[4-(2-oxo-piperidin-1-yl)-benzyl]-4-methyl-valeriansäureamid,
(R)-2-[(5-Chlor-thiophen-2-carbonyl)-amino]-*N*-[4-(3-oxo-morpholin-4-yl)-phenyl]-3-methyl-buttersäureamid,
(R)-2-[(5-Chlor-thiophen-2-carbonyl)-amino]-*N*-[4-(2-oxo-2*H-*pyridin-1-yl)-phenyl]-3-methyl-buttersäureamid,
3-[(5-Chlor-thiophen-2-carbonyl)-amino]-*N*-[4-(3-oxo-morpholin-4-yl)-phenyl]-propionsäureamid,
3-[(5-Chlor-thiophen-2-carbonyl)-amino]-*N*-[3-methyl-4-(3-oxo-morpholin-4-yl)-phenyl]-propionsäureamid,
3-[(5-Chlor-thiophen-2-carbonyl)-amino]-*N*-[4-(2-oxo-2*H*-pyridin-1-yl)-phenyl]-propionsäureamid,
2-[(5-Chlor-thiophen-2-carbonyl)-amino]-*N*-[3-methyl-4-(3-oxo-morpholin-4-yl)-phenyl]-acetamid,
2-[(5-Chlor-thiophen-2-carbonyl)-amino]-*N*-[4-(3-oxo-morpholin-4-yl)-phenyl]-acetamid,
2-[(5-Chlor-thiophen-2-carbonyl)-amino]-*N*-[4-(2-oxo-2*H*-pyridin-1-yl)-phenyl]-acetamid,
3-[(5-Chlor-thiophen-2-carbonyl)-amino]-N-[4-(2-oxo-2H-pyridin-1-yl)-phenyl]-2-butyl-propionsäureamid,
(R)-2-[(5-Chlor-thiophen-2-carbonyl)-amino]-*N*-[4-(3-oxo-morpholin-4-yl)-phenyl]-propionsäureamid,
(R)-2-[(5-Chlor-thiophen-2-carbonyl)-amino]-*N*-[4-(3-oxo-morpholin-4-yl)-phenyl]-valeriansäureamid,
(R)-2-[(5-Chlor-thiophen-2-carbonyl)-amino]-*N*-[4-(2-oxo-2*H-*pyridin-1-yl)-phenyl]-3-methylsulfanyl-propionsäureamid,
(R)-2-[(5-Chlor-thiophen-2-carbonyl)-amino]-*N*-[4-(2-oxo-2*H-*pyrazin-1-yl)-phenyl]-propionsäureamid,
(R)-2-[(5-Chlor-thiophen-2-carbonyl)-amino]-*N*-[4-(3-oxo-morpholin-4-yl)-phenyl]-4-methylsulfanyl-buttersäureamid,
(R)-2-[(5-Chlor-thiophen-2-carbonyl)-amino]-*N*-[4-(3-oxo-morpholin-4-yl)-phenyl]-buttersäureamid,
(R)-2-[(5-Chlor-thiophen-2-carbonyl)-amino]-*N*-[4-(3-oxo-morpholin-4-yl)-phenyl]-3-ethinyl-propionsäureamid,
(R)-2-[(5-Chlor-thiophen-2-carbonyl)-amino]-*N*-[3-methyl-4-(3-oxo-morpholin-4-yl)-phenyl]-3-ethinyl-propionsäureamid,
(R)-2-[(5-Chlor-thiophen-2-carbonyl)-amino]-*N*-[3-fluor-4-(3-oxo-morpholin-4-yl)-phenyl]-propionsäureamid,
(R)-2-[(5-Chlor-thiophen-2-carbonyl)-amino]-*N*-[3-methyl-4-(3-oxo-morpholin-4-yl)-phenyl]-4-methylsulfanyl-buttersäureamid,
(R)-2-[(5-Chlor-thiophen-2-carbonyl)-amino]-*N*-[4-(3-oxo-morpholin-4-yl)-phenyl]-3-(*tert*.-butyloxycarbonyl)-propionsäureamid,
(R)-2-[(5-Chlor-thiophen-2-carbonyl)-amino]-*N*-[4-(3-oxo-morpholin-4-yl)-phenyl]-3-vinyl-propionsäureamid,
(R)-2-[(5-Chlor-thiophen-2-carbonyl)-amino]-*N*-[3-methyl-4-(3-oxo-morpholin-4-yl)-phenyl]-3-vinyl-propionsäureamid,
(R)-2-[(5-Chlor-thiophen-2-carbonyl)-amino]-*N*-[3-methyl-4-(3-oxo-morpholin-4-yl)-phenyl]-3-(*tert*.-butyloxycarbonyl)-propionsäureamid,
(R)-2-[(5-Chlor-thiophen-2-carbonyl)amino]-*N*-[4-(3-oxo-morpholin-4-yl)-phenyl]-4-methoxy-buttersäureamid,
(R)-2-[(5-Chlor-thiophen-2-carbonyl)-amino]-*N*-[3-methyl-4-(3-oxo-morpholin-4-yl)-phenyl]-4-methoxy-buttersäureamid,
(R)-2-[(5-Chlor-thiophen-2-carbonyl)-amino]-*N*-[3-fluor-4-(3-oxo-morpholin-4-yl)-phenyl]-4-methyl-valeriansäureamid,
(R)-2-[(5-Chlor-thiophen-2-carbonyl)-amino]-*N*-[3-fluor-4-(3-oxo-morpholin-4-yl)-phenyl]-valeriansäureamid,
(R)-2-[(5-Chlor-thiophen-2-carbonyl)-amino]-*N*-[3-chlor-4-(3-oxo-morpholin-4-yl)-phenyl]-propionsäureamid,
(R)-2-[(5-Chlor-thiophen-2-carbonyl)-amino]-*N*-[4-(3-oxo-morpholin-4-yl)phenyl]-4-(*tert*.-butyloxycarbonyl)-buttersäureamid,
(R)-2-[(5-Chlor-thiophen-2-carbonyl)-amino]-*N*-[3-methyl-4-(3-oxo-morpholin-4-yl)-phenyl]-4-(*tert*.-butyloxycarbonyl)-buttersäureamid,
(R)-2-[(5-Chlor-thiophen-2-carbonyl)-amino]-*N*-[4-(2-oxo-piperidin-1-yl)-phenyl]-4-(*tert*.-butyloxycarbonyl)-buttersäureamid,
(R)-2-[(5-Chlor-thiophen-2-carbonyl)amino]-*N*-[4-(3-oxo-morpholin-4-yl)-phenyl]-4-(*tert*.-butyloxycarbonylamino)-buttersäureamid,
(R)-2-[(5-Chlor-thiophen-2-carbonyl)-amino]-*N*-[3-methyl-4-(3-oxo-morpholin-4-yl)-phenyl]-4-(*tert*.-butyloxycarbonylamino)-buttersäureamid,
(R)-2-[(5-Chlor-thiophen-2-carbonyl)-amino]-*N*-[4-(3-oxo-morpholin-4-yl)-phenyl]-5-(*tert*.-butyloxycarbonylamino)-valeriansäureamid,
(R)-2-[(5-Chlor-thiophen-2-carbonyl)-amino]-*N*-[3-methyl-4-(3-oxo-morpholin-4-yl)-phenyl]-5-(*tert*.-butyloxycarbonylamino)-valeriansäureamid,
(R)-2-[(5-Chlor-thiophen-2-carbonyl)-amino]-*N*-[4-(3-oxo-morpholin-4-yl)-phenyl]-3-(*tert*.-butyloxycarbonylamino)-propionsäureamid,
(R)-2-[(5-Chlor-thiophen-2-carbonyl)-amino]-*N*-[3-methyl-4-(3-oxo-morpholin-4-yl)-phenyl]-3-(*tert*.-butyloxycarbonylamino)-propionsäureamid,
(R)-3-[(5-Chlor-thiophen-2-carbonyl)amino]-*N*-[3-methyl-4-(3-oxo-morpholin-4-yl)-phenyl]-buttersäureamid,
(R)-3-[(5-Chlor-thiophen-2-carbonyl)-amino]-*N*-[3-methyl-4-(3-oxo-morpholin-4-yl)-phenyl]-5-methyl-adipinsäureamid,
(S)-3-[(5-Chlor-thiophen-2-carbonyl)-amino]-*N*-[3-methyl-4-(3-oxo-morpholin-4-yl)-phenyl]-5-methyl-adipinsäureamid,
(R)-2-[(5-Chlor-thiophen-2-carbonyl)-amino]-*N*-[4-(2-oxo-2*H-*pyridin-1-yl)-phenyl]-3-methoxy-propionsäureamid,
(R)-2-[(5-Chlor-thiophen-2-carbonyl)-amino]-*N*-[3-methyl-4-(3-oxo-morpholin-4-yl)-phenyl]-3-methoxy-propionsäureamid,
(2R,3R)-2-[(5-Chlor-thiophen-2-carbonyl)-amino]-*N*-[4-(3-oxo-morpholin-4-yl)-phenyl]-3-methoxy-buttersäureamid,
(2R,3R)-2-[(5-Chlor-thiophen-2-carbonyl)-amino]-*N*-[3-methyl-4-(3-oxo-morpholin-4-yl)-phenyl]-3-methoxy-buttersäureamid,
(S)-2-[(5-Chlor-thiophen-2-carbonyl)-amino]-*N*-[3-methyl-4-(3-oxo-morpholin-4-yl)-phenyl]-3-methoxy-propionsäureamid,
(R)-2-[(5-Chlor-thiophen-2-carbonyl)amino]-*N*-[3-trifluormethyl-4-(3-oxo-morpholin-4-yl)-phenyl]-3-methoxy-propionsäureamid,
(R)-2-[(5-Chlor-thiophen-2-carbonyl)-amino]-*N*-[3-chlor-4-(2-azabicyclo[2.2.2]-octan-2-yl)-phenyl]-3-methoxy-propionsäureamid,
(R)-2-[(5-Chlor-thiophen-2-carbonyl)-amino]-*N*-[3-trifluormethoxy-4-(2-aza-bicyclo[2.2.2]-octan-2-yl)-phenyl]-3-methoxy-propionsäureamid,
(R)-2-[(5-Chlor-thiophen-2-carbonyl)-amino]-*N*-[3-chlor-4-(3-oxo-morpholin-4-yl)-phenyl]-3-methoxy-propionsäureamid,
(R)-2-[(5-Chlor-thiophen-2-carbonyl)-amino]-*N*-[3-fluor-4-(3-oxo-morpholin-4-yl)-phenyl]-3-methoxy-propionsäureamid,
(R)-2-[(5-Chlor-thiophen-2-carbonyl)-amino]-*N*-[3-chlor-4-(2-oxo-2*H*-pyridin-1-yl)-phenyl]-3-methoxy-propionsäureamid,
(R)-2-[(5-Chlor-thiophen-2-carbonyl)-amino]-*N*-[3-methyl-4-(3-oxo-morpholin-4-yl)-phenyl]-3-allyl-propionsäureamid,
(R)-2-[(5-Chlor-thiophen-2-carbonyl)-amino]-*N*-[3-methyl-4-(3-oxo-morpholin-4-yl)-phenyl]-3-propoxy-propionsäureamid,
(R)-2-[(5-Chlor-thiophen-2-carbonyl)-amino]-*N*-[3-methyl-4-(3-oxo-morpholin-4-yl)-phenyl]-3-ethoxy-propionsäureamid,
(R)-2-[(5-Chlor-thiophen-2-carbonyl)-amino]-*N*-[3-methyl-4-(3-oxo-morpholin-4-yl)-phenyl]-3-(2-methoxyethoxy)-propionsäureamid,
(2R,3R)-2-[(5-Chlor-thiophen-2-carbonyl)-amino]-*N*-[3-methyl-4-(3-oxo-morpholin-4-yl)-phenyl]-3-ethoxy-buttersäureamid,
(2R,3R)-2-[(5-Chlor-thiophen-2-carbonyl)-amino]-*N*-[3-methyl-4-(3-oxo-morpholin-4-yl)-phenyl]-3-(2-methoxyethoxy)-buttersäureamid,
(R)-2-[(5-Chlor-thiophen-2-carbonyl)-amino]-*N*-[3-methyl-4-(3-oxo-morpholin-4-yl)-phenyl]-3-methylsulfonyl-propionsäureamid,
(R)-2-[(5-Chlor-thiophen-2-carbonyl)-amino]-*N*-[4-(2-oxo-2*H-*pyridin-1-yl)-phenyl]-3-methylsulfonyl-propionsäureamid,
(R)-2-[(5-Chlor-thiophen-2-carbonyl)-amino]-*N*-[4-(3-oxo-morpholin-4-yl)-phenyl]-3-methylsulfonyl-propionsäureamid,
(R)-2-[(5-Chlor-thiophen-2-carbonyl)-amino]-*N*-[4-(3-oxo-morpholin-4-yl)-phenyl]-3-methylsulfonyl-buttersäureamid,
(R)-2-[(5-Chlor-thiophen-2-carbonyl)-amino]-*N*-[3-methyl-4-(3-oxo-morpholin-4-yl)-phenyl]-3-methylsulfonyl-buttersäureamid,
(R)-2-[(5-Chlor-thiophen-2-carbonyl)-amino]-*N*-[4-(2-oxo-2*H-*pyridin-1-yl)-phenyl]-3-methylsulfonyl-buttersäureamid,
(R)-2-[(5-Chlor-thiophen-2-ylmethyl)-amino]-*N*-[4-(3-oxo-morpholin-4-yl)-phenyl]-valeriansäureamid,
(R)-2-[(5-Chlor-thiophen-2-carbonyl)-amino]-*N*-[4-(3-oxo-morpholin-4-yl)-phenyl]-3-carboxy-propionsäureamid,
(R)-2-[(5-Chlor-thiophen-2-carbonyl)-amino]-*N*-[3-methyl-4-(3-oxo-morpholin-4-yl)-phenyl]-3-carboxy-propionsäureamid,
(R)-2-[(5-Chlor-thiophen-2-carbonyl)-amino]-*N*-[4-(3-oxo-morpholin-4-yl)-phenyl]-4-carboxy-buttersäureamid,
(R)-2-[(5-Chlor-thiophen-2-carbonyl)-amino]-*N*-[3-methyl-4-(3-oxo-morpholin-4-yl)-phenyl]-4-carboxy-buttersäureamid,
(R)-2-[(5-Chlor-thiophen-2-carbonyl)-amino]-*N*-[4-(3-oxo-morpholin-4-yl)-phenyl]-4-amino-buttersäureamid,
(R)-2-[(5-Chlor-thiophen-2-carbonyl)-amino]-*N*-[3-methyl-4-(3-oxo-morpholin-4-yl)-phenyl]-4-amino-buttersäureamid,
(R)-2-[(5-Chlor-thiophen-2-carbonyl)-amino]-*N*-[4-(3-oxo-morpholin-4-yl)-phenyl]-5-amino-valeriansäureamid,
(R)-2-[(5-Chlor-thiophen-2-carbonyl)-amino]-*N*-[3-methyl-4-(3-oxo-morpholin-4-yl)-phenyl]-5-amino-valeriansäureamid,
(R)-2-[(5-Chlor-thiophen-2-carbonyl)-amino]-*N*-[4-(3-oxo-morpholin-4-yl)-phenyl]-3-amino-propionsäureamid,
(R)-2-[(5-Chlor-thiophen-2-carbonyl)-amino]-*N*-[3-methyl-4-(3-oxo-morpholin-4-yl)-phenyl]-3-amino-propionsäureamid,
(R)-2-[(5-Chlor-thiophen-2-carbonyl)-amino]-*N*-[3-methyl-4-(3-oxo-morpholin-4-yl)-phenyl]-3-hydroxy-propionsäureamid,
(R)-2-[(5-Chlor-thiophen-2-carbonyl)-amino]-*N*-[3-4-(3-oxo-morpholin-4-yl)-phenyl]-3-hydroxy-propionsäureamid,
(2R,3R)-2-[(5-Chlor-thiophen-2-carbonyl)-amino]-*N*-[4-(3-oxo-morpholin-4-yl)-phenyl]-3-hydroxy-buttersäureamid,
(2R,3R)-2-[(5-Chlor-thiophen-2-carbonyl)-amino]-*N*-[4-(3-oxo-morpholin-4-yl)-phenyl]-3-aminocarbonyloxy-buttersäureamid,
(2R)-2-[(5-Chlor-thiophen-2-carbonyl)-amino]-*N*-[3-methyl-4-(3-oxo-morpholin-4-yl)-phenyl]-3-aminocarbonyloxy-propionsäureamid,
(2R,3R)-2-[(5-Chlor-thiophen-2-carbonyl)-amino]-*N*-[3-methyl-4-(3-oxo-morpholin-4-yl)-phenyl]-3-aminocarbonyloxy-buttersäureamid,
sowie ihre pharmazeutisch verwendbaren Salze, Solvate und Stereoisomere, einschließlich deren Mischungen in allen Verhältnissen.

18. Verfahren zur Herstellung von Verbindungen der Formel I nach den Ansprüchen 1-17 sowie ihrer pharmazeutisch verwendbaren Salze, Solvate und Stereoisomere, **dadurch gekennzeichnet, daß** man
a) eine Verbindung der Formel II
H₂N-W-Y-T II
worin
W, Y und T die in Anspruch 1 angegebenen Bedeutungen haben,
mit einer Verbindung der Formel III 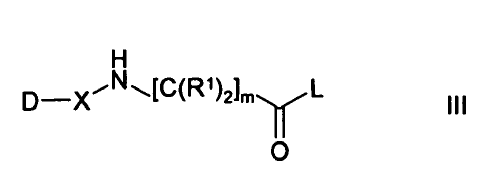 worin
L Cl, Br, I oder eine freie oder reaktionsfähig funktionell abgewandelte OH-Gruppe bedeutet und
R¹, m, X und D die in Anspruch 1 angegebenen Bedeutungen haben, umsetzt,
oder
b) zur Herstellung von Verbindungen der Formel I,
in denen X -C=O bedeutet,
eine Verbindung der Formel IV 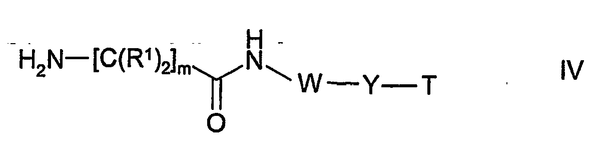 worin R¹, m, W, Y und T die in Anspruch 1 angegebenen Bedeutungen haben,
mit einer Verbindung der Formel V
D-CO-L V
worin
L Cl, Br, I oder eine freie oder reaktionsfähig funktionell abgewandelte OH-Gruppe bedeutet und
D die in Anspruch 1 angegebene Bedeutung hat,
umsetzt,
oder
c) zur Herstellung von Verbindungen der Formel I,
in denen X CH₂ bedeutet,
eine Verbindung der Formel IV 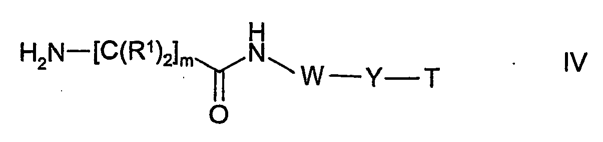 worin R¹, m, W, Y und T die in Anspruch 1 angegebenen Bedeutungen haben,
mit einer Verbindung der Formel VI
D-CHO VI
worin
D die in Anspruch 1 angegebene Bedeutung hat,
in einer reduktiven Aminierung
umsetzt,
und/oder
eine Base oder Säure der Formel I in eines ihrer Salze umwandelt.

19. Verbindungen der Formel I nach einem oder mehreren der Ansprüche 1 bis 17 als Inhibitoren des Koagulationsfaktors Xa.

20. Verbindungen der Formel nach einem oder mehreren der Ansprüche 1 bis 17 als Inhibitoren des Koagulationsfaktors VIIa.

21. Arzneimittel, enthaltend mindestens eine Verbindung der Formel I nach einem oder mehreren der Ansprüche 1 bis 17 und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

22. Arzneimittel enthaltend mindestens eine Verbindung der Formel I gemäß einem oder mehreren der Ansprüche 1 bis 17 und/oder ihre pharmazeutisch verwendbaren Salze, Solvate und Stereoisomere, einschließlich deren Mischungen in allen Verhältnissen, und mindestens einen weiteren Arzneimittelwirkstoff.

23. Verwendung von Verbindungen gemäß einem oder mehreren der Ansprüche 1 bis 17 und/oder ihre physiologisch unbedenklichen Salze und Solvate zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen.

24. Set (Kit), bestehend aus getrennten Packungen von
(a) einer wirksamen Menge an einer Verbindung der Formel I gemäß einem oder mehreren der Ansprüche 1 bis 17 und/oder ihrer pharmazeutisch verwendbaren Salze, Solvate und Stereoisomere, einschließlich deren Mischungen in allen Verhältnissen,
und
(b) einer wirksamen Menge eines weiteren Arzneimittelswirkstoffs.

## Claims

1. Compounds of the formula I 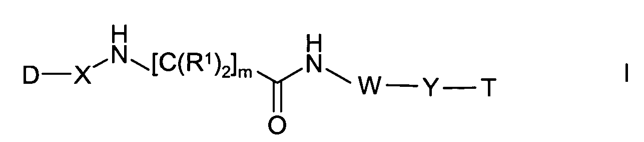 in which
D denotes an aromatic carbo- or heterocycle having 0 to 4 N, O and/or S atoms which is unsubstituted or mono- or polysubstituted by Hal, A, OR², N(R²)₂, NO₂, CN, COOR² or CON(R²)₂,
X denotes -C=O or C(R³)₂,
W denotes -[C(R³)₂]ₙ-,
R¹ denotes H or A, which may be substituted by OR³, S(O)ₙR³, N(R³)₂, CN, COOR³, CON(R³)2, OCON(R³)₂, N(R³)COOR³, N(R³)CON(R³)2, N(R³)SO₂R³, SO₂N(R³)₂ or -C≡C-,
R² denotes H, A, -[C(R³)₂]ₙ-Ar', -[C(R³)₂]ₙ-Het', -[C(R³)₂]ₙ-cyclo-alkyl, -[C(R³)₂]ₙ-N(R³)₂ or -[C(R³)₂]ₙ-OR³,
R³ denotes H or A,
Y denotes alkylene, cycloalkylene, Het-diyl or Ar-diyl,
T denotes a mono- or bicyclic saturated, unsaturated or aromatic carbo- or heterocycle having 0 to 4 N, O and/or S atoms which is mono- or disubstituted by =O, =S, =NR², =N-CN, =N-NO₂, =NOR², =NCOR², =NCOOR², =NOCOR² and may furthermore be mono-, di- or trisubstituted by R², Hal, A, -[C(R³)₂]ₙ-Ar, -[C(R³)₂]ₙ-Het, -[C(R³)₂]ₙ-cycloalkyl, OR², N(R²)₂, NO₂, CN, COOR², CON(R²)₂, NR²COA, NR²CON(R²)₂, NR²SO₂A, COR², SO₂NR² and/or S(O)ₙA,
A denotes unbranched or branched alkyl having 1-10 C atoms, in which one or two CH₂ groups may be replaced by O or S atoms and/or by -CH=CH- groups and/or also 1-7 H atoms may be replaced by F,
Ar denotes phenyl, naphthyl or biphenyl, each of which is unsubstituted or mono-, di- or trisubstituted by Hal, A, OR², N(R²)₂, NO₂, CN, COOR², CON(R²)₂, NR²COA, NR²CON(R²)₂, NR²SO₂A, COR², SO₂N(R²)₂, S(O)ₙA, -[C(R³)₂]ₙ-COOR² or -O-[C(R³)₂]ₒ-COOR²,
Ar' denotes phenyl which is unsubstituted or mono-, di- or trisubstituted by Hal, A, OR³, N(R³)₂, NO₂, CN, COOR³, CON(R³)₂, NR³COA, NR³CON(R³)₂, NR³SO₂A, COR³, SO₂N(R³)₂, S(O)ₙA, -[C(R³)₂]ₙ-COOR³ or -O-[C(R³)₂]ₒ-COOR³,
Het denotes a mono- or bicyclic saturated, unsaturated or aromatic heterocycle having 1 to 4 N, O and/or S atoms, which may be unsubstituted or mono-, di- or trisubstituted by carbonyl oxygen, =S, =N(R²)₂, Hal, A, -[C(R³)₂]ₙ-Ar, -[C(R³)₂]ₙ-Het', -[C(R³)₂]ₙ-cyclcloalkyl, -[C(R³)₂]ₙ-OR², -[C(R³)₂]ₙ-N(R³)₂, NO₂, CN, -[C(R³)₂]ₙ-COOR^{2'}, -[C(R³)₂]ₙ-CON(R²)₂, -[C(R³)₂]ₙ-NR²COA, NR²CON(R²)₂, -[C(R³)₂]ₙ-NR²SO₂A, COR², SO₂NR² and/or S(O)ₙA,
Het' denotes a mono- or bicyclic saturated, unsaturated or aromatic heterocycle having 1 to 4 N, O and/or S atoms, which may be unsubstituted or mono- or disubstituted by carbonyl oxygen, =S, =N(R³)₂, Hal, A, OR³, N(R³)₂, NO₂, CN, COOR³, CON(R³)₂, NR³COA, NR³CON(R³)₂, NR³SO₂A, COR³, SO₂NR³ and/or S(O)ₙA,
Hal denotes F, Cl, Br or I,
m denotes 1 or 2,
n denotes 0, 1 or 2,
o denotes 1, 2 or 3,
and pharmaceutically usable salts, solvates and stereoisomers thereof, including mixtures thereof in all ratios.

2. Compounds according to Claim 1, in which
D denotes an aromatic five-ring heterocycle having 1 to 2 N, O and/or S atoms which is unsubstituted or mono- or disubstituted by Hal,
and pharmaceutically usable salts, solvates and stereoisomers thereof, including mixtures thereof in all ratios.

3. Compounds according to Claim 1 or 2, in which
D denotes a thienyl ring which is mono- or disubstituted by Hal,
and pharmaceutically usable salts, solvates and stereoisomers thereof, including mixtures thereof in all ratios.

4. Compounds according to one or more of Claims 1-3, in which
R² denotes H or alkyl having 1, 2, 3, 4, 5 or 6 C atoms,
and pharmaceutically usable salts, solvates and stereoisomers thereof, including mixtures thereof in all ratios.

5. Compounds according to one or more of Claims 1-4, in which
R¹ denotes H or A, which may be substituted by OR³, CON(R³)₂, N(R³)₂, S(O)ₙR³, COOR³, OCON(R³)₂, N(R³)COOR³ or -C≡C-,
and pharmaceutically usable salts, solvates and stereoisomers thereof, including mixtures thereof in all ratios.

6. Compounds according to one or more of Claims 1-5, in which
X denotes -C=O,
and pharmaceutically usable salts, solvates and stereoisomers thereof, including mixtures thereof in all ratios.

7. Compounds according to one or more of Claims 1-6, in which
W is absent,
and pharmaceutically usable salts, solvates and stereoisomers thereof, including mixtures thereof in all ratios.

8. Compounds according to one or more of Claims 1-7, in which
Y denotes Ar-diyl,
and pharmaceutically usable salts, solvates and stereoisomers thereof, including mixtures thereof in all ratios.

9. Compounds according to one or more of Claims 1-8, in which
T denotes a mono- or bicyclic saturated, unsaturated or aromatic heterocycle having 1 to 2 N and/or O atoms which is mono- or disubstituted by =O, =S, =NR², =N-CN, =N-NO₂, =NOR², =NCOR², =NCOOR² or =NOCOR² and may further-more be mono- or disubstituted by Hal or A,
and pharmaceutically usable salts, solvates and stereoisomers thereof, including mixtures thereof in all ratios.

10. Compounds according to one or more of Claims 1-9, in which
T denotes a mono- or bicyclic saturated or unsaturated hetero-cycle having 1 to 2 N and/or O atoms which is mono- or disubstituted by =O, =S or =NH,
and pharmaceutically usable salts, solvates and stereoisomers thereof, including mixtures thereof in all ratios.

11. Compounds according to one or more of Claims 1-10, in which
T denotes piperidin-1-yl, pyrrolidin-1-yl, pyridin-1-yl, morpholin-4-yl, piperazin-1-yl, 1,3-oxazolidin-3-yl, pyridazin-2-yl, pyrazin-1-yl, azepan-1-yl or 2-azabicyclo[2.2.2]octan-2-yl, each of which is mono- or disubstituted by =O or =NH,
and pharmaceutically usable salts, solvates and stereoisomers thereof, including mixtures thereof in all ratios.

12. Compounds according to one or more of Claims 1-11, in which
Ar denotes phenyl which is unsubstituted or mono- or disubstituted by Hal, A, OA, SO₂A, COOR², SO₂NH₂ or CN,
and pharmaceutically usable salts, solvates and stereoisomers thereof, including mixtures thereof in all ratios.

13. Compounds according to one or more of Claims 1-12, in which
Ar denotes phenyl which is unsubstituted or mono- or disubstituted by A and/or Hal,
and pharmaceutically usable salts, solvates and stereoisomers thereof, including mixtures thereof in all ratios.

14. Compounds according to one or more of Claims 1-13, in which
D denotes an aromatic five-ring heterocycle having 1 to 2 N, O and/or S atoms which is unsubstituted or mono- or disubstituted by Hal,
R¹ denotes H or A, which may be substituted by OR³, CON(R³)₂, N(R³)₂, S(O)ₙR³, COOR³, OCON(R³)₂, N(R³)COOR³ or -C≡C-,
R² denotes H or alkyl having 1, 2, 3, 4, 5 or 6 C atoms,
X denotes -C=O or CH₂,
W is absent,
Y denotes Ar-diyl,
Ar denotes phenyl which is unsubstituted or mono- or disubstituted by A and/or Hal,
T denotes a mono- or bicyclic saturated or unsaturated hetero-cycle having 1 to 2 N and/or O atoms which is mono- or disubstituted by =O, =S or =NH,
and pharmaceutically usable salts, solvates and stereoisomers thereof, including mixtures thereof in all ratios.

15. Compounds according to one or more of Claims 1-14, in which
D denotes thienyl, thiazolyl or furyl, each of which is mono- or disubstituted by Hal,
R¹ denotes H or A, which may be substituted by OR³, CON(R³)₂, N(R³)₂, S(O)ₙR³, COOR³, OCON(R³)₂, N(R³)COOR³ or -C≡C-,
R² denotes H or alkyl having 1, 2, 3, 4, 5 or 6 C atoms,
X denotes -C=O or CH₂,
W is absent,
Y denotes Ar-diyl,
Ar denotes phenyl which is unsubstituted or mono- or disubstituted by A and/or Hal,
T denotes piperidin-1-yl, pyrrolidin-1-yl, pyridin-1-yl, morpholin-4-yl, piperazin-1-yl, 1,3-oxazolidin-3-yl, pyridazin-2-yl, pyrazin-1-yl, azepan-1-yl or 2-azabicyclo[2.2.2]octan-2-yl, each of which is mono- or disubstituted by =O or =NH,
and pharmaceutically usable salts, solvates and stereoisomers thereof, including mixtures thereof in all ratios.

16. Compounds according to one or more of Claims 1-15, in which
D denotes thienyl or phenyl, each of which is mono- or disubstituted by Hal,
R¹ denotes H or A, which may be substituted by OR³, CON(R³)₂, N(R³)₂, S(O)ₙR³, COOR³, OCON(R³)₂, N(R³)COOR³ or -C≡C-,
R² denotes H or alkyl having 1, 2, 3, 4, 5 or 6 C atoms,
R³ denotes H or alkyl having 1, 2, 3, 4, 5 or 6 C atoms,
X denotes -C=O or CH₂,
W is absent or denotes CH₂,
Y denotes Ar-diyl,
A denotes alkyl having 1, 2, 3, 4, 5 or 6 C atoms, in which one or two CH₂ groups may be replaced by O or S atoms and/or by -CH=CH- groups and/or also 1-7 H atoms may be replaced by F,
Ar denotes phenyl which is unsubstituted or mono- or disubstituted by A and/or Hal,
T denotes piperidin-1-yl, pyrrolidin-1-yl, pyridin-1-yl, morpholin-4-yl, piperazin-1-yl, 1,3-oxazolidin-3-yl, pyridazin-2-yl, pyrazin-1-yl, azepan-1-yl or 2-azabicyclo[2.2.2]octan-2-yl, each of which is mono- or disubstituted by =O or =NH,
and pharmaceutically usable salts, solvates and stereoisomers thereof, including mixtures thereof in all ratios.

17. Compounds according to Claim 1, selected from the group
(S)-2-[(5-chlorothiophene-2-carbonyl)amino]-*N*-[4-(3-oxomorpholin-4-yl)phenyl]-4-methylvaleramide,
(S)-2-[(5-chlorothiophene-2-carbonyl)amino]-*N*-[3-methyl-4-(3-oxomorpholin-4-yl)phenyl]-4-methylvaleramide,
(S)-2-[(5-chlorothiophene-2-carbonyl)amino]-*N*-[4-(2-oxo-2*H-*pyrazin-1-yl)phenyl]-4-methylvaleramide,
(R)-2-[(5-chlorothiophene-2-carbonyl)amino]-*N*-[4-(3-oxomorpholin-4-yl)phenyl]-4-methylvaleramide,
(R)-2-[(5-chlorothiophene-2-carbonyl)amino]-*N*-[3-methyl-4-(3-oxomorpholin-4-yl)phenyl]-4-methylvaleramide,
(R)-2-[(5-chlorothiophene-2-carbonyl)amino]-*N*-[4-(2-oxo-2*H-*pyrazin-1-yl)phenyl]-4-methylvaleramide,
(R)-2-[(5-chlorothiophene-2-carbonyl)amino]-*N*-[4-(2-oxo-2*H-*pyridin-1-yl)phenyl]-4-methylvaleramide,
(R)-2-[(5-chlorothiophene-2-carbonyl)amino]-*N*-[4-(2-imino-piperidin-1-yl)phenyl]-4-methylvaleramide,
(S)-2-[(5-chlorothiophene-2-carbonyl)amino]-*N*-[4-(2-imino-piperidin-1-yl)phenyl]-4-methylvaleramide,
2-[(5-chlorothiophene-2-carbonyl)amino]-*N*-[3-methyl-4-(2-oxopiperidin-1-yl)phenyl]acetamide,
3-[(5-chlorothiophene-2-carbonyl)amino]-*N*-[3-methyl-4-(2-oxopiperidin-1-yl)phenyl]propionamide,
(R)-2-[(5-chlorothiophene-2-carbonyl)amino]-*N*-[3-methyl-4-(3-oxomorpholin-4-yl)phenyl]propionamide,
(R)-2-[(5-chlorothiophene-2-carbonyl)amino]-*N*-[3-methyl-4-(3-oxomorpholin-4-yl)phenyl]-3-methylbutyramide,
(R)-2-[(5-chlorothiophene-2-carbonyl)amino]-*N*-[3-methyl-4-(3-oxomorpholin-4-yl)phenyl]butyramide,
(R)-2-[(5-chlorothiophene-2-carbonyl)amino]-*N*-[3-methyl-4-(3-oxomorpholin-4-yl)phenyl]valeramide,
(R)-2-[(5-chlorothiophene-2-carbonyl)amino]-*N*-[3-methyl-4-(3-oxomorpholin-4-yl)phenyl]-3-aminocarbonylpropionamide,
(R)-2-[(4-chlorophenylcarbonyl)amino]-*N*-[4-(3-oxomorpholin-4-yl)phenyl]-4-methylvaleramide,
(R)-2-[(4-chlorophenylcarbonyl)amino]-*N*-[3-methyl-4-(3-oxo-morpholin-4-yl)phenyl]-4-methylvaleramide,
2-[(5-chlorothiophene-2-carbonyl)amino]-*N*-[3-methyl-4-(3-oxomorpholin-4-yl)phenyl]-3-(*N*,*N*-dimethylamino)propionamide,
(R)-2-[(5-bromothiophene-2-carbonyl)amino]-*N*-[4-(3-oxomorpholin-4-yl)phenyl]-4-methylvaleramide,
(R)-2-[(5-chlorothiophene-2-carbonyl)amino]-*N*-[4-(2-oxo-piperidin-1-yl)benzyl]-4-methylvaleramide,
2-[(5-chlorothiophene-2-methyl)amino]-*N*-[4-(3-oxomorpholin-4-yl)phenyl]-4-methylvaleramide,
(R)-2-[(5-chlorothiophene-2-carbonyl)amino]-*N*-[4-(3-oxomorpholin-4-yl)phenyl]-3-methylsulfanylpropionamide,
(S)-2-[(5-chlorothiophene-2-carbonyl)amino]-*N*-[4-(2-oxopiperidin-1-yl)benzyl]-4-methylvaleramide,
(R)-2-[(5-chlorothiophene-2-carbonyl)amino]-*N*-[4-(3-oxomorpholin-4-yl)phenyl]-3-methylbutyramide,
(R)-2-[(5-chlorothiophene-2-carbonyl)amino]-*N*-[4-(2-oxo-2*H-*pyridin-1-yl)phenyl]-3-methylbutyramide, .
3-[(5-chlorothiophene-2-carbonyl)amino]-*N*-[4-(3-oxomorpholin-4-yl)phenyl]propionamide,
3-[(5-chlorothiophene-2-carbonyl)amino]-*N*-[3-methyl-4-(3-oxomorpholin-4-yl)phenyl]propionamide,
3-[(5-chlorothiophene-2-carbonyl)amino]-*N*-[4-(2-oxo-2*H*-pyridin-1-yl)phenyl]propionamide,
2-[(5-chlorothiophene-2-carbonyl)amino]-*N*-[3-methyl-4-(3-oxomorpholin-4-yl)phenyl]acetamide,
2-[(5-chlorothiophene-2-carbonyl)amino]-*N*-[4-(3-oxomorpholin-4-yl)phenyl]acetamide,
2-[(5-chlorothiophene-2-carbonyl)amino]-*N*-[4-(2-oxo-2*H*-pyridin-1-yl)phenyl]acetamide,
3-[(5-chlorothiophene-2-carbonyl)amino]-*N*-[4-(2-oxo-2*H*-pyridin-1-yl)phenyl]-2-butylpropionamide,
(R)-2-[(5-chlorothiophene-2-carbonyl)amino]-*N*-[4-(3-oxomorpholin-4-yl)phenyl]propionamide,
(R)-2-[(5-chlorothiophene-2-carbonyl)amino]-*N*-[4-(3-oxomorpholin-4-yl)phenyl]valeramide,
(R)-2-[(5-chlorothiophene-2-carbonyl)amino]-*N*-[4-(2-oxo-2*H-*pyridin-1-yl)phenyl]-3-methylsulfanylpropionamide,
(R)-2-[(5-chlorothiophene-2-carbonyl)amino]-*N*-[4-(2-oxo-2*H-*pyrazin-1-yl)phenyl]propionamide,
(R)-2-[(5-chlorothiophene-2-carbonyl)amino]-*N*-[4-(3-oxomorpholin-4-yl)phenyl]-4-methylsulfanylbutyramide,
(R)-2-[(5-chlorothiophene-2-carbonyl)amino]-*N*-[4-(3-oxomorpholin-4-yl)phenyl]butyramide,
(R)-2-[(5-chlorothiophene-2-carbonyl)amino]-*N*-[4-(3-oxomorpholin-4-yl)phenyl]-3-ethynylpropionamide,
(R)-2-[(5-chlorothiophene-2-carbonyl)amino]-*N*-[3-methyl-4-(3-oxomorpholin-4-yl)phenyl]-3-ethynylpropionamide,
(R)-2-[(5-chlorothiophene-2-carbonyl)amino]-*N*-[3-fluoro-4-(3-oxomorpholin-4-yl)phenyl]propionamide,
(R)-2-[(5-chlorothiophene-2-carbonyl)amino]-*N*-[3-methyl-4-(3-oxomorpholin-4-yl)phenyl]-4-methylsulfanylbutyramide,
(R)-2-[(5-chlorothiophene-2-carbonyl)amino]-*N*-[4-(3-oxomorpholin-4-yl)phenyl]-3-(*tert*-butyloxycarbonyl)propionamide,
(R)-2-[(5-chlorothiophene-2-carbonyl)amino]-*N*-[4-(3-oxomorpholin-4-yl)phenyl]-3-vinylpropionamide,
(R)-2-[(5-chlorothiophene-2-carbonyl)amino]-*N*-[3-methyl-4-(3-oxomorpholin-4-yl)phenyl]-3-vinylpropionamide,
(R)-2-[(5-chlorothiophene-2-carbonyl)amino]-*N*-[3-methyl-4-(3-oxomorpholin-4-yl)phenyl]-3-(*tert*-butyloxycarbonyl)propionamide,
(R)-2-[(5-chlorothiophene-2-carbonyl)amino]-*N*-[4-(3-oxomorpholin-4-yl)phenyl]-4-methoxybutyramide,
(R)-2-[(5-chlorothiophene-2-carbonyl)amino]-*N*-[3-methyl-4-(3-oxomorpholin-4-yl)phenyl]-4-methoxybutyramide,
(R)-2-[(5-chlorothiophene-2-carbonyl)amino]-*N*-[3-fluoro-4-(3-oxomorpholin-4-yl)phenyl]-4-methylvaleramide,
(R)-2-[(5-chlorothiophene-2-carbonyl)amino]-*N*-[3-fluoro-4-(3-oxomorpholin-4-yl)phenyl]valeramide,
(R)-2-[(5-chlorothiophene-2-carbonyl)amino]-*N*-[3-chloro-4-(3-oxomorpholin-4-yl)phenyl]propionamide,
(R)-2-[(5-chlorothiophene-2-carbonyl)amino]-*N*-[4-(3-oxomorpholin-4-yl)phenyl]-4-(*tert*-butyloxycarbonyl)butyramide,
(R)-2-[(5-chlorothiophene-2-carbonyl)amino]-*N*-[3-methyl-4-(3-oxomorpholin-4-yl)phenyl]-4-(*tert*-butyloxycarbonyl)butyramide,
(R)-2-[(5-chlorothiophene-2-carbonyl)amino]-*N*-[4-(2-oxopiperidin-1-yl)phenyl]-4-(*tert*-butyloxycarbonyl)butyramide,
(R)-2-[(5-chlorothiophene-2-carbonyl)amino]-*N*-[4-(3-oxomorpholin-4-yl)phenyl]-4-(*tert*-butyloxycarbonylamino)butyramide,
(R)-2-[(5-chlorothiophene-2-carbonyl)amino]-*N*-[3-methyl-4-(3-oxomorpholin-4-yl)phenyl]-4-(*tert*-butyloxycarbonylamino)butyramide,
(R)-2-[(5-chlorothiophene-2-carbonyl)amino]-*N*-[4-(3-oxomorpholin-4-yl)phenyl]-5-(*tert*-butyloxycarbonylamino)valeramide,
(R)-2-[(5-chlorothiophene-2-carbonyl)amino]-*N*-[3-methyl-4-(3-oxomorpholin-4-yl)phenyl]-5-(*tert*-butyloxycarbonylamino)valeramide,
(R)-2-[(5-chlorothiophene-2-carbonyl)amino]-*N*-[4-(3-oxomorpholin-4-yl)phenyl]-3-(*tert*-butyloxycarbonylamino)propionamide,
(R)-2-[(5-chlorothiophene-2-carbonyl)amino]-*N*-[3-methyl-4-(3-oxomorpholin-4-yl)phenyl]-3-(*tert*-butyloxycarbonylamino)propionamide,
(R)-3-[(5-chlorothiophene-2-carbonyl)amino]-*N*-[3-methyl-4-(3-oxomorpholin-4-yl)phenyl]butyramide,
(R)-3-[(5-chlorothiophene-2-carbonyl)amino]-*N*-[3-methyl-4-(3-oxomorpholin-4-yl)phenyl]-5-methyladipamide,
(S)-3-[(5-chlorothiophene-2-carbonyl)amino]-*N*-[3-methyl-4-(3-oxomorpholin-4-yl)phenyl]-5-methyladipamide,
(R)-2-[(5-chlorothiophene-2-carbonyl)amino]-*N*-[4-(2-oxo-2*H-*pyridin-1-yl)phenyl]-3-methoxypropionamide,
(R)-2-[(5-chlorothiophene-2-carbonyl)amino]-*N*-[3-methyl-4-(3-oxomorpholin-4-yl)phenyl]-3-methoxypropionamide,
(2R,3R)-2-[(5-chlorothiophene-2-carbonyl)amino]-*N*-[4-(3-oxo-morpholin-4-yl)phenyl]-3-methoxybutyramide,
(2R,3R)-2-[(5-chlorothiophene-2-carbonyl)amino]-*N*-[3-methyl-4-(3-oxomorpholin-4-yl)phenyl]-3-methoxybutyramide,
(S)-2-[(5-chlorothiophene-2-carbonyl)amino]-*N*-[3-methyl-4-(3-oxomorpholin-4-yl)phenyl]-3-methoxypropionamide,
(R)-2-[(5-chlorothiophene-2-carbonyl)amino]-*N*-[3-trifluoromethyl-4-(3-oxomorpholin-4-yl)phenyl]-3-methoxypropionamide,
(R)-2-[(5-chlorothiophene-2-carbonyl)amino]-*N*-[3-chloro-4-(2-azabicyclo[2.2.2]octan-2-yl)phenyl]-3-methoxypropionamide,
(R)-2-[(5-chlorothiophene-2-carbonyl)amino]-*N*-[3-trifluoromethoxy-4-(2-azabicyclo[2.2.2]octan-2-yl)phenyl]-3-methoxypropion-amide,
(R)-2-[(5-chlorothiophene-2-carbonyl)amino]-*N*-[3-chloro-4-(3-oxomorpholin-4-yl)phenyl]-3-methoxypropionamide,
(R)-2-[(5-chlorothiophene-2-carbonyl)amino]-*N*-[3-fluoro-4-(3-oxomorpholin-4-yl)phenyl]-3-methoxypropionamide,
(R)-2-[(5-chlorothiophene-2-carbonyl)amino]-*N*-[3-chloro-4-(2-oxo-2*H*-pyridin-1-yl)phenyl]-3-methoxypropionamide,
(R)-2-[(5-chlorothiophene-2-carbonyl)amino]-*N*-[3-methyl-4-(3-oxomorpholin-4-yl)phenyl]-3-allylpropionamide,
(R)-2-[(5-chlorothiophene-2-carbonyl)amino]-*N*-[3-methyl-4-(3-oxomorpholin-4-yl)phenyl]-3-propoxypropionamide,
(R)-2-[(5-chlorothiophene-2-carbonyl)amino]-*N*-[3-methyl-4-(3-oxomorpholin-4-yl)phenyl]-3-ethoxypropionamide,
(R)-2-[(5-chlorothiophene-2-carbonyl)amino]-*N*-[3-methyl-4-(3-oxomorpholin-4-yl)phenyl]-3-(2-methoxyethoxy)propionamide,
(2R,3R)-2-[(5-chlorothiophene-2-carbonyl)amino]-*N*-[3-methyl-4-(3-oxomorpholin-4-yl)phenyl]-3-ethoxybutyramide,
(2R,3R)-2-[(5-chlorothiophene-2-carbonyl)amino]-*N*-[3-methyl-4-(3-oxomorpholin-4-yl)phenyl]-3-(2-methoxyethoxy)butyramide,
(R)-2-[(5-chlorothiophene-2-carbonyl)amino]-*N*-[3-methyl-4-(3-oxomorpholin-4-yl)phenyl]-3-methylsulfonylpropionamide,
(R)-2-[(5-chlorothiophene-2-carbonyl)amino]-*N*-[4-(2-oxo-2*H-*pyridin-1-yl)phenyl]-3-methylsulfonylpropionamide,
(R)-2-[(5-chlorothiophene-2-carbonyl)amino]-*N*-[4-(3-oxomorpholin-4-yl)phenyl]-3-methylsulfonylpropionamide,
(R)-2-[(5-chlorothiophene-2-carbonyl)amino]-*N*-[4-(3-oxomorpholin-4-yl)phenyl]-3-methylsulfonylbutyramide,
(R)-2-[(5-chlorothiophene-2-carbonyl)amino]-*N*-[3-methyl-4-(3-oxomorpholin-4-yl)phenyl]-3-methylsulfonylbutyramide,
(R)-2-[(5-chlorothiophene-2-carbonyl)amino]-*N*-[4-(2-oxo-2*H-*pyridin-1-yl)phenyl]-3-methylsulfonylbutyramide,
(R)-2-[(5-chlorothiophen-2-ylmethyl)amino]-*N*-[4-(3-oxomorpholin-4-yl)phenyl]valeramide,
(R)-2-[(5-chlorothiophene-2-carbonyl)amino]-*N*-[4-(3-oxomorpholin-4-yl)phenyl]-3-carboxypropionamide,
(R)-2-[(5-chlorothiophene-2-carbonyl)amino]-*N*-[3-methyl-4-(3-oxomorpholin-4-yl)phenyl]-3-carboxypropionamide,
(R)-2-[(5-chlorothiophene-2-carbonyl)amino]-*N*-[4-(3-oxomorpholin-4-yl)phenyl]-4-carboxybutyramide,
(R)-2-[(5-chlorothiophene-2-carbonyl)amino]-*N*-[3-methyl-4-(3-oxomorpholin-4-yl)phenyl]-4-carboxybutyramide,
(R)-2-[(5-chlorothiophene-2-carbonyl)amino]-*N*-[4-(3-oxomorpholin-4-yl)phenyl]-4-aminobutyramide,
(R)-2-[(5-chlorothiophene-2-carbonyl)amino]-*N*-[3-methyl-4-(3-oxomorpholin-4-yl)phenyl]-4-aminobutyramide,
(R)-2-[(5-chlorothiophene-2-carbonyl)amino]-*N*-[4-(3-oxomorpholin-4-yl)phenyl]-5-aminovaleramide,
(R)-2-[(5-chlorothiophene-2-carbonyl)amino]-*N*-[3-methyl-4-(3-oxomorpholin-4-yl)phenyl]-5-aminovaleramide,
(R)-2-[(5-chlorothiophene-2-carbonyl)amino]-*N*-[4-(3-oxomorpholin-4-yl)phenyl]-3-aminopropionamide,
(R)-2-[(5-chlorothiophene-2-carbonyl)amino]-*N*-[3-methyl-4-(3-oxomorpholin-4-yl)phenyl]-3-aminopropionamide,
(R)-2-[(5-chlorothiophene-2-carbonyl)amino]-*N*-[3-methyl-4-(3-oxomorpholin-4-yl)phenyl]-3-hydroxypropionamide,
(R)-2-[(5-chlorothiophene-2-carbonyl)amino]-*N*-[3-4-(3-oxomorpholin-4-yl)phenyl]-3-hydroxypropionamide,
(2R,3R)-2-[(5-chlorothiophene-2-carbonyl)amino]-*N*-[4-(3-oxo-morpholin-4-yl)phenyl]-3-hydroxybutyramide,
(2R,3R)-2-[(5-chlorothiophene-2-carbonyl)amino]-*N*-[4-(3-oxo-morpholin-4-yl)phenyl]-3-aminocarbonyloxybutyramide,
(2R)-2-[(5-chlorothiophene-2-carbonyl)amino]-*N*-[3-methyl-4-(3-oxomorpholin-4-yl)phenyl]-3-aminocarbonyloxypropionamide,
(2R,3R)-2-[(5-chlorothiophene-2-carbonyl)amino]-*N*-[3-methyl-4-(3-oxomorpholin-4-yl)phenyl]-3-aminocarbonyloxybutyramide,
and pharmaceutically usable salts, solvates and stereoisomers thereof, including mixtures thereof in all ratios.

18. Process for the preparation of compounds of the formula I according to Claims 1-17 and pharmaceutically usable salts, solvates and stereoisomers thereof, **characterised in that**
a) a compound of the formula II
H₂N-W-Y-T II
in which
W, Y and T have the meanings indicated in Claim 1,
is reacted with a compound of the formula III 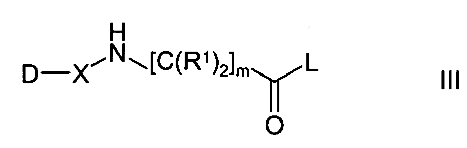 in which
L denotes Cl, Br, I or a free or reactively functionally modified OH group, and
R¹, m, X and D have the meanings indicated in Claim 1,
or
b) for the preparation of compounds of the formula I
in which X denotes -C=O,
a compound of the formula IV 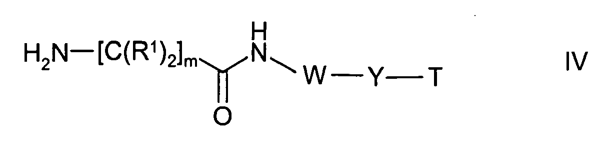 in which R¹, m, W, Y and T have the meanings indicated in Claim 1,
is reacted with a compound of the formula V
D-CO-L V
in which
L denotes Cl, Br, I or a free or reactively functionally modified OH group, and
D has the meaning indicated in Claim 1,
or
c) for the preparation of compounds of the formula I
in which X denotes CH₂,
a compound of the formula IV 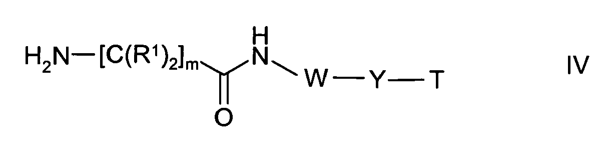 in which R¹, m, W, Y and T have the meanings indicated in Claim 1,
is reacted with a compound of the formula VI
D-CHO VI
in which
D has the meaning indicated in Claim 1,
in a reductive amination,
and/or
a base or acid of the formula I is converted into one of its salts.

19. Compounds of the formula I according to one or more of Claims 1 to 17 as inhibitors of coagulation factor Xa.

20. Compounds of the formula I according to one or more of Claims 1 to 17 as inhibitors of coagulation factor VIIa.

21. Medicaments comprising at least one compound of the formula I according to one or more of Claims 1 to 17 and/or pharmaceutically usable salts, solvates and stereoisomers thereof, including mixtures thereof in all ratios, and optionally excipients and/or adjuvants.

22. Medicaments comprising at least one compound of the formula I according to one or more of Claims 1 to 17 and/or pharmaceutically usable salts, solvates and stereoisomers thereof, including mixtures thereof in all ratios, and at least one further medicament active ingredient.

23. Use of compounds according to one or more of Claims 1 to 17 and/or physiologically acceptable salts and solvates thereof for the preparation of a medicament for the treatment of thromboses, myocardial infarction, arteriosclerosis, inflammation, apoplexy, angina pectoris, restenosis after angioplasty, claudicatio intermittens, migraine, tumours, tumour diseases and/or tumour metastases.

24. Set (kit) consisting of separate packs of
(a) an effective amount of a compound of the formula I according to one or more of Claims 1 to 17 and/or pharmaceutically usable salts, solvates and stereoisomers thereof, including mixtures thereof in all ratios,
and
(b) an effective amount of a further medicament active ingredient.

## Revendications

1. Composés de formule I 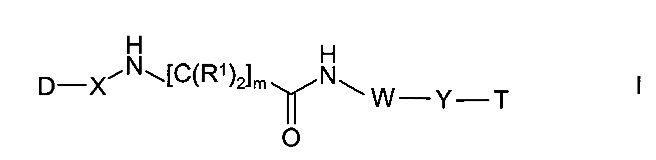 dans laquelle
D désigne un carbo- ou hétérocycle aromatique ayant de 0 à 4 atomes de N, O et/ou S qui est non substitué ou mono- ou polysubstitué par Hal, A, OR², N(R²)₂, NO₂, CN, COOR² ou CON(R²)₂,
X désigne -C=O ou C(R³)₂,
W désigne -[C(R³)₂]ₙ-,
R¹ désigne H ou A, pouvant être substitué par OR³, S(O)ₙR³, N(R³)₂, CN, COOR³, CON(R³)₂, OCON(R³)₂, N(R³)COOR³, N(R³)CON(R³)₂, N(R³)SO₂R³, SO₂N(R³)₂ ou -C=C-,
R² désigne H, A, -[C(R³)₂]ₙ-Ar', -[C(R³)₂]ₙ-Hét', -[C(R³)₂]ₙ-cyclo-alkyle, -[C(R³)₂]ₙ-N(R³)₂ ou -[C(R³)₂]ₙ-OR³,
R³ désigne H ou A,
Y désigne alkylène, cycloalkylène, Hét-diyle ou Ar-diyle,
T désigne un carbo- ou hétérocycle mono- ou bicyclique saturé, insaturé ou aromatique, ayant de 0 à 4 atomes de N, O et/ou S qui est mono- ou disubstitué par =O, =S, =NR², =N-CN, =N-NO₂, =NOR², =NCOR², =NCOOR², =NOCOR² et pouvant en outre être mono-, di- ou trisubstitué par R², Hal, A, -[C(R³)₂]ₙ-Ar, -[C(R₃)₂]ₙ-Hét, -[C(R³)₂]ₙ-cycloalkyle, OR², N(R²)_{2,} NO₂, CN, COOR², CON(R²)₂, NR²COA, NR²CON(R²)₂, NR²SO₂A, COR², SO₂NR² et/ou S(O)ₙA,
A désigne alkyle linéaire ou ramifié ayant 1-10 atomes de C, dans lequel un ou deux groupements CH₂ peuvent être remplacés par des atomes de O ou S et/ou par des groupements -CH=CH- et/ou également 1-7 atomes de H peuvent être remplacés par F,
Ar désigne phényle, naphtyle ou biphényle, chacun d'entre eux étant non substitué ou mono-, di- ou trisubstitué par Hal, A, OR², N(R²)₂, NO₂, CN, COOR², CON(R²)₂, NR²COA, NR²CON(R²)₂, NR²SO₂A, COR², SO₂N(R²)₂, S(O)ₙA, -[C(R³)₂]ₙ-COOR² ou -O-[C(R³)₂]ₒ-COOR²,
Ar' désigne phényle qui est non substitué ou mono-, di- ou trisubstitué par Hal, A, OR³, N(R³)₂, NO₂, CN, COOR³, CON(R³)₂, NR³COA, NR³CON(R³)₂, NR³SO₂A, COR³, SO(R³)₂, S(O)ₙA, -[C(R³)₂]ₙ-COOR³ ou -O-[C(R³)₂]ₒ-COOR³,
Hét désigne un hétérocycle mono- ou bicyclique saturé, insaturé
ou aromatique ayant de 1 à 4 atomes de N, O et/ou S, pouvant être non substitué ou mono-, di- ou trisubstitué par oxygène carbonyle, =S, =N(R²)₂, Hal, A, -[C(R³)₂]ₙ-Ar, -[C(R³)₂]ₙ-Hét', -[C(R³)₂]ₙ-cycloalkyle, -[C(R³)₂]ₙ-OR², -[C(R³)₂]ₙ-N(R³)₂, NO₂, CN, -[C(R³)₂]n-COOR^{2'}, -[C(R³)₂]ₙ-CON(R²)₂, -[C(R³)₂]ₙ-NR²COA, NR²CON(R²)₂, -[C(R³)₂]ₙ-NR²SO₂A, COR², SO₂NR² et/ou S(O)ₙA,
Hét' désigne un hétérocycle mono- ou bicyclique saturé, insaturé ou aromatique ayant de 1 à 4 atomes de N, O et/ou S, pouvant être non substitué ou mono- ou disubstitué par oxygène carbonyle, =S, =N(R³)₂, Hal, A, OR³, N(R³)₂, NO₂, CN, COOR³, CON(R³)₂, NR³COA, NR³CON(R³)₂, NR³SO₂A, COR³, SO₂NR³ et/ou S(O)ₙA,
Hal désigne F, Cl, Br ou I,
m désigne 1 ou 2,
n désigne 0, 1 ou 2,
o désigne 1, 2 ou 3,
et les sels, solvats et stéréoisomères pharmaceutiquement utilisables de ceux-ci, y compris leurs mélanges en toutes proportions.

2. Composés selon la revendication 1, dans lesquels
D désigne un hétérocycle aromatique de cinq chaînons ayant de 1 à 2 atomes de N, O et/ou S qui est non substitué ou mono- ou disubstitué par Hal,
et les sels, solvats et stéréoisomères pharmaceutiquement utilisables de ceux-ci, y compris leurs mélanges en toutes proportions.

3. Composés selon la revendication 1 ou 2, dans lesquels
D désigne un cycle thiényle qui est mono- ou disubstitué par Hal,
et les sels, solvats et stéréoisomères pharmaceutiquement utilisables de ceux-ci, y compris leurs mélanges en toutes proportions.

4. Composés selon l'une ou plusieurs des revendications 1-3, dans lesquels
R² désigne H ou alkyle ayant 1, 2, 3, 4, 5 ou 6 atomes de C,
et les sels, solvats et stéréoisomères pharmaceutiquement utilisables de ceux-ci, y compris leurs mélanges en toutes proportions.

5. Composés selon l'une ou plusieurs des revendications 1-4, dans lesquels
R¹ désigne H ou A, pouvant être substitué par OR³, CON(R³)₂, N(R³)₂, S(O)ₙR³, COOR³, OCON(R³)₂, N(R³)COOR³ ou -C≡C-,
et les sels, solvats et stéréoisomères pharmaceutiquement utilisables de ceux-ci, y compris leurs mélanges en toutes proportions.

6. Composés selon l'une ou plusieurs des revendications 1-5, dans lesquels
X désigne -C=O,
et les sels, solvats et stéréoisomères pharmaceutiquement utilisables de ceux-ci, y compris leurs mélanges en toutes proportions.

7. Composés selon l'une ou plusieurs des revendications 1-6, dans lesquels
W est absent,
et les sels, solvats et stéréoisomères pharmaceutiquement utilisables de ceux-ci, y compris leurs mélanges en toutes proportions.

8. Composés selon l'une ou plusieurs des revendications 1-7, dans lesquels
Y désigne Ar-diyle,
et les sels, solvats et stéréoisomères pharmaceutiquement utilisables de ceux-ci, y compris leurs mélanges en toutes proportions.

9. Composés selon l'une ou plusieurs des revendications 1-8, dans lesquels
T désigne un hétérocycle mono- ou bicyclique saturé, insaturé ou aromatique ayant de 1 à 2 atomes de N et/ou O qui est mono- ou disubstitué par =O, =S, =NR², =N-CN, =N-NO₂, =NOR², =NCOR², =NCOOR² ou =NOCOR² et pouvant être en outre mono- ou disubstitué par Hal ou A,
et les sels, solvats et stéréoisomères pharmaceutiquement utilisables de ceux-ci, y compris leurs mélanges en toutes proportions.

10. Composés selon l'une ou plusieurs des revendications 1-9, dans lesquels
T désigne un hétérocycle mono- ou bicyclique saturé ou insaturé ayant de 1 à 2 atomes de N et/ou O qui est mono- ou disubstitué par =O, =S ou =NH,
et les sels, solvats et stéréoisomères pharmaceutiquement utilisables de ceux-ci, y compris leurs mélanges en toutes proportions.

11. Composés selon l'une ou plusieurs des revendications 1-10, dans lesquels
T désigne pipéridin-1-yle, pyrrolidin-1-yle, pyridin-1-yle, morpholin-4-yle, pipérazin-1-yle, 1,3-oxazolidin-3-yle, pyridazin-2-yle, pyrazin-1-yle, azépan-1-yle ou 2-azabicyclo[2.2.2]octan-2-yle, chacun d'entre eux étant mono- ou disubstitué par =O ou =NH,
et les sels, solvats et stéréoisomères pharmaceutiquement utilisables de ceux-ci, y compris leurs mélanges en toutes proportions.

12. Composés selon l'une ou plusieurs des revendications 1-11, dans lesquels
Ar désigne phényle qui est non substitué ou mono- ou disubstitué par Hal, A, OA, SO₂A, COOR², SO₂NH₂ ou CN,
et les sels, solvats et stéréoisomères pharmaceutiquement utilisables de ceux-ci, y compris leurs mélanges en toutes proportions.

13. Composés selon l'une ou plusieurs des revendications 1-12, dans lesquels
Ar désigne phényle qui est non substitué ou mono- ou disubstitué par A et/ou Hal,
et les sels, solvats et stéréoisomères pharmaceutiquement utilisables de ceux-ci, y compris leurs mélanges en toutes proportions.

14. Composés selon l'une ou plusieurs des revendications 1-13, dans lesquels
D désigne un hétérocycle aromatique de cinq chaînons ayant de 1 à 2 atomes de N, O et/ou S qui est non substitué ou mono- ou disubstitué par Hal,
R¹ désigne H ou A, pouvant être substitué par OR³, CON(R³)₂, N(R³)₂, S(O)ₙR³, COOR³, OCON(R³)₂, N(R³)COOR³ ou -C≡C-,
R² désigne H ou alkyle ayant 1, 2, 3, 4, 5 ou 6 atomes de C,
X désigne -C=O ou CH₂,
W est absent,
Y désigne Ar-diyle,
Ar désigne phényle qui est non substitué ou mono- ou disubstitué par A et/ou Hal,
T désigne un hétérocycle mono- ou bicyclique saturé ou insaturé ayant de 1 à 2 atomes de N et/ou O qui est mono- ou disubstitué par =O, =S ou =NH,
et les sels, solvats et stéréoisomères pharmaceutiquement utilisables de ceux-ci, y compris leurs mélanges en toutes proportions.

15. Composés selon l'une ou plusieurs des revendications 1-14, dans lesquels
D désigne thiényle, thiazolyle ou furyle, chacun d'entre eux étant mono- ou disubstitué par Hal,
R¹ désigne H ou A, pouvant être substitué par OR³, CON(R³)₂, N(R³)₂, S(O)ₙR³, COOR³, OCON(R³)₂, N(R³)COOR³ ou -C≡C-,
R² désigne H ou alkyle ayant 1, 2, 3, 4, 5 ou 6 atomes de C,
X désigne -C=O ou CH₂,
W est absent,
Y désigne Ar-diyle,
Ar désigne phényle qui est non substitué ou mono- ou disubstitué par A et/ou Hal,
T désigne pipéridin-1-yle, pyrrolidin-1-yle, pyridin-1-yle, morpholin-4-yle, pipérazin-1-yle, 1,3-oxazolidin-3-yle, pyridazin-2-yle, pyrazin-1-yle, azépan-1-yle ou 2-azabicyclo[2.2.2]octan-2-yle, chacun d'entre eux étant mono- ou disubstitué par =O ou =NH,
et les sels, solvats et stéréoisomères pharmaceutiquement utilisables de ceux-ci, y compris leurs mélanges en toutes proportions.

16. Composés selon l'une ou plusieurs des revendications 1-15, dans lesquels
D désigne thiényle ou phényle, chacun d'entre eux étant mono- ou disubstitué par Hal,
R¹ désigne H ou A, pouvant être substitué par OR³, CON(R³)₂, N(R³)₂, S(O)ₙR³, COOR³, OCON(R³)₂, N(R³)COOR³ ou -C≡C-,
R² désigne H ou alkyle ayant 1, 2, 3, 4, 5 ou 6 atomes de C,
R³ désigne H ou alkyle ayant 1, 2, 3, 4, 5 ou 6 atomes de C,
X désigne -C=O ou CH₂,
W est absent ou désigne CH₂,
Y désigne Ar-diyle,
A désigne alkyle ayant 1, 2, 3, 4, 5 ou 6 atomes de C, dans lequel un ou deux groupements CH₂ peuvent être remplacés par des atomes de O ou S et/ou par des groupements -CH=CH- et/ou également 1-7 atomes de H peuvent être remplacés par F,
Ar désigne phényle qui est non substitué ou mono- ou disubstitué par A et/ou Hal,
T désigne pipéridin-1-yle, pyrrolidin-1-yle, pyridin-1-yle, morpholin-4-yle, pipérazin-1-yle, 1,3-oxazolidin-3-yle, pyridazin-2-yle, pyrazin-1-yle, azépan-1-yle ou 2-azabicyclo[2.2.2]octan-2-yle, chacun d'entre eux étant mono- ou disubstitué par =O ou =NH,
et les sels, solvats et stéréoisomères pharmaceutiquement utilisables de ceux-ci, y compris leurs mélanges en toutes proportions.

17. Composés selon la revendication 1, choisis parmi le groupe constitué par:
le (S)-2-[(5-chlorothiophène-2-carbonyl)amino]-*N*-[4-(3-oxo-morpholin-4-yl)phényl]-4-méthylvaléramide,
le (S)-2-[(5-chlorothiophène-2-carbonyl)amino]-*N*-[3-méthyl-4-(3-oxomorpholin-4-yl)phényl]-4-méthylvaléramide,
le (S)-2-[(5-chlorothiophène-2-carbonyl)amino]-*N*-[4-(2-oxo-2*H*-pyrazin-1-yl)phényl]-4-méthylvaléramide,
le (R)-2-[(5-chlorothiophène-2-carbonyl)amino]-*N*-[4-(3-oxo-morpholin-4-yl)phényl]-4-méthylvaléramide,
le (R)-2-[(5-chlorothiophène-2-carbonyl)amino]-*N*-[3-méthyl-4-(3-oxomorpholin-4-yl)phényl]-4-méthylvaléramide,
le (R)-2-[(5-chlorothiophène-2-carbonyl)amino]-*N*-[4-(2-oxo-2*H*-pyrazin-1-yl)phényl]-4-méthylvaléramide,
le (R)-2-[(5-chlorothiophène-2-carbonyl)amino]-*N*-[4-(2-oxo-2*H*-pyridin-1-yl)phényl]-4-méthylvaléramide,
le (R)-2-[(5-chlorothiophène-2-carbonyl)amino]-*N*-[4-(2-imino-pipéridin-1-yl)phényl]-4-méthylvaléramide,
le (S)-2-[(5-chlorothiophène-2-carbonyl)amino]-*N*-[4-(2-imino-pipéridin-1-yl)phényl]-4-méthylvaléramide,
le 2-[(5-chlorothiophène-2-carbonyl)amino]-*N*-[3-méthyl-4-(2-oxopipéridin-1-yl)phényl]acétamide,
le 3-[(5-chlorothiophène-2-carbonyl)amino]-*N*-[3-méthyl-4-(2-oxopipéridin-1-yl)phényl]propionamide,
le (R)-2-[(5-chlorothiophène-2-carbonyl)amino]-*N*-[3-méthyl-4-(3-oxomorpholin-4-yl)phényl]propionamide,
le (R)-2-[(5-chlorothiophène-2-carbonyl)amino]-*N*-[3-méthyl-4-(3-oxomorpholin-4-yl)phényl]-3-méthylbutyramide,
le (R)-2-[(5-chlorothiophène-2-carbonyl)amino]-*N*-[3-méthyl-4-(3-oxomorpholin-4-yl)phényl]butyramide,
le (R)-2-[(5-chlorothiophène-2-carbonyl)amino]-*N*-[3-méthyl-4-(3-oxomorpholin-4-yl)phényl]valéramide,
le (R)-2-[(5-chlorothiophène-2-carbonyl)amino]-*N*-[3-méthyl-4-(3-oxomorpholin-4-yl)phényl]-3-aminocarbonylpropionamide,
le (R)-2-[(4-chlorophénylcarbonyl)amino]-*N*-[4-(3-oxomorpholin-4-yl)phényl]-4-méthylvaléramide,
le (R)-2-[(4-chlorophénylcarbonyl)amino]-*N*-[3-méthyl-4-(3-oxomorpholin-4-yl)phényl]-4-méthylvaléramide,
le 2-[(5-chlorothiophène-2-carbonyl)amino]-*N*-[3-méthyl-4-(3-oxomorpholin-4-yl)phényl]-3-(*N*,*N*-diméthylamino)propionamide,
le (R)-2-[(5-bromothiophène-2-carbonyl)amino]-*N*-[4-(3-oxo-morpholin-4-yl)phényl]-4-méthylvaléramide,
le (R)-2-[(5-chlorothiophéne-2-carbonyl)amino]-*N*-[4-(2-oxo-pipéridin-1-yl)benzyl]-4-méthylvaléramide,
le 2-[(5-chlorothiophène-2-méthyl)amino]-*N*-[4-(3-oxomorpholin-4-yl)phényl]-4-méthylvaléramide,
le (R)-2-[(5-chlorothiophène-2-carbonyl)amino]-*N*-[4-(3-oxo-morpholin-4-yl)phényl]-3-méthylsulfanylpropionamide,
le (S)-2-[(5-chlorothiophène-2-carbonyl)amino]-*N*-[4-(2-oxo-pipéridin-1-yl)benzyl]-4-méthylvaléramide,
le (R)-2-[(5-chlorothiophène-2-carbonyl)amino]-*N*-[4-(3-oxo-morpholin-4-yl)phényl]-3-méthylbutyramide,
le (R)-2-[(5-chlorothiophène-2-carbonyl)amino]-*N*-[4-(2-oxo-2*H*-pyridin-1-yl)phényl]-3-méthylbutyramide,
le 3-[(5-chlorothiophène-2-carbonyl)amino]-*N*-[4-(3-oxomorpholin-4-yl)phényl]propionamide,
le 3-[(5-chlorothiophène-2-carbonyl)amino]-*N*-[3-méthyl-4-(3-oxomorpholin-4-yl)phényl]propionamide,
le 3-[(5-chlorothiophène-2-carbonyl)amino]-*N*-[4-(2-oxo-2*H-*pyridin-1-yl)phényl]propionamide,
le 2-[(5-chlorothiophène-2-carbonyl)amino]-*N*-[3-méthyl-4-(3-oxomorpholin-4-yl)phényl]acétamide,
le 2-[(5-chlorothiophène-2-carbonyl)amino]-*N*-[4-(3-oxomorpholin-4-yl)phényl]acétamide,
le 2-[(5-chlorothiophène-2-carbonyl)amino]-*N*-[4-(2-oxo-2*H-*pyridin-1-yl)phényl]acétamide,
le 3-[(5-chlorothiophène-2-carbonyl)amino]-*N*-[4-(2-oxo-2*H-*pyridin-1-yl)phényl]-2-butylpropionamide,
le (R)-2-[(5-chlorothiophène-2-carbonyl)amino]-*N*-[4-(3-oxomorpholin-4-yl)phényl]propionamide,
le (R)-2-[(5-chlorothiophène-2-carbonyl)amino]-*N*-[4-(3-oxomorpholin-4-yl)phényl]valéramide,
le (R)-2-[(5-chlorothiophène-2-carbonyl)amino]-*N*-[4-(2-oxo-2*H-*pyridin-1-yl)phényl]-3-méthylsulfanylpropionamide,
le (R)-2-[(5-chlorothiophène-2-carbonyl)amino]-*N*-[4-(2-oxo-2*H-*pyrazin-1-yl)phényl]propionamide,
le (R)-2-[(5-chlorothiophène-2-carbonyl)amino]-*N*-[4-(3-oxomorpholin-4-yl)phényl]-4-méthylsulfanylbutyramide,
le (R)-2-[(5-chlorothiophène-2-carbonyl)amino]-*N*-[4-(3-oxomorpholin-4-yl)phényl]butyramide,
le (R)-2-[(5-chlorothiophène-2-carbonyl)amino]-*N*-[4-(3-oxomorpholin-4-yl)phényl]-3-éthynylpropionamide,
le (R)-2-[(5-chlorothiophène-2-carbonyl)amino]-*N*-[3-méthyl-4-(3-oxomorpholin-4-yl)phényl]-3-éthynylpropionamide,
le (R)-2-[(5-chlorothiophène-2-carbonyl)amino]-*N*-[3-fluoro-4-(3-oxomorpholin-4-yl)phényl]propionamide,
le (R)-2-[(5-chlorothiophène-2-carbonyl)amino]-*N*-[3-méthyl-4-(3-oxomorpholin-4-yl)phényl]-4-méthylsulfanylbutyramide,
le (R)-2-[(5-chlorothiophène-2-carbonyl)amino]-*N*-[4-(3-oxomorpholin-4-yl)phényl]-3-(*tertio*-butyloxycarbonyl)propionamide,
le (R)-2-[(5-chlorothiophène-2-carbonyl)amino]-*N*-[4-(3-oxomorpholin-4-yl)phényl]-3-vinylpropionamide,
le (R)-2-[(5-chlorothiophène-2-carbonyl)amino]-*N*-[3-méthyl-4-(3-oxomorpholin-4-yl)phényl]-3-vinylpropionamide,
le (R)-2-[(5-chlorothiophène-2-carbonyl)amino]-*N*-[3-méthyl-4-(3-oxomorpholin-4-yl)phényl]-3-(*tertio*-butyloxycarbonyl)propion-amide,
le (R)-2-[(5-chlorothiophène-2-carbonyl)amino]-*N*-[4-(3-oxomorpholin-4-yl)phényl]-4-méthoxybutyramide,
le (R)-2-[(5-chlorothiophène-2-carbonyl)amino]-*N*-[3-méthyl-4-(3-oxomorpholin-4-yl)phényl]-4-méthoxybutyramide,
le (R)-2-[(5-chlorothiophène-2-carbonyl)amino]-*N*-[3-fluoro-4-(3-oxomorpholin-4-yl)phényl]-4-méthylvaléramide,
le (R)-2-[(5-chlorothiophène-2-carbonyl)amino]-*N*-[3-fluoro-4-(3-oxomorpholin-4-yl)phényl]valéramide,
le (R)-2-[(5-chlorothiophène-2-carbonyl)amino]-*N*-[3-chloro-4-(3-oxomorpholin-4-yl)phényl]propionamide,
le (R)-2-[(5-chlorothiophène-2-carbonyl)amino]-*N*-[4-(3-oxomorpholin-4-yl)phényl]-4-(*tertio*-butyloxycarbonyl)butyramide,
le (R)-2-[(5-chlorothiophène-2-carbonyl)amino]-*N*-[3-méthyl-4-(3-oxomorpholin-4-yl)phényl]-4-(*tertio*-butyloxycarbonyl)butyramide,
le (R)-2-[(5-chlorothiophène-2-carbonyl)amino]-*N*-[4-(2-oxopipéridin-1-yl)phényl]-4-(*tertio*-butyloxycarbonyl)butyramide,
le (R)-2-[(5-chlorothiophène-2-carbonyl)amino]-*N*-[4-(3-oxomorpholin-4-yl)phényl]-4-(*tertio*-butyloxycarbonylamino)butyramide,
le (R)-2-[(5-chlorothiophène-2-carbonyl)amino]-*N*-[3-méthyl-4-(3-oxomorpholin-4-yl)phényl]-4-(*tertio*-butyloxycarbonylamino)butyramide,
le (R)-2-[(5-chlorothiophène-2-carbonyl)amino]-*N*-[4-(3-oxomorpholin-4-yl)phényl]-5-(*tertio*-butyloxycarbonylamino)valéramide,
le (R)-2-[(5-chlorothiophène-2-carbonyl)amino]-*N*-[3-méthyl-4-(3-oxomorpholin-4-yl)phényl]-5-(*tertio*-butyloxycarbonylamino)valéramide,
le (R)-2-[(5-chlorothiophène-2-carbonyl)amino]-*N*-[4-(3-oxomorpholin-4-yl)phényl]-3-(*tertio*-butyloxycarbonylamino)propionamide,
le (R)-2-[(5-chlorothiophène-2-carbonyl)amino]-*N*-[3-méthyl-4-(3-oxomorpholin-4-yl)phényl]-3-(*tertio*-butyloxycarbonylamino)propionamide,
le (R)-3-[(5-chlorothiophène-2-carbonyl)amino]-*N*-[3-méthyl-4-(3-oxomorpholin-4-yl)phényl]butyramide,
le (R)-3-[(5-chlorothiophène-2-carbonyl)amino]-*N*-[3-méthyl-4-(3-oxomorpholin-4-yl)phényl]-5-méthyladipamide,
le (S)-3-[(5-chlorothiophène-2-carbonyl)amino]-*N*-[3-méthyl-4-(3-oxomorpholin-4-yl)phényl]-5-méthyladipamide,
le (R)-2-[(5-chlorothiophène-2-carbonyl)amino]-*N*-[4-(2-oxo-2*H-*pyridin-1-yl)phényl]-3-méthoxypropionamide,
le (R)-2-[(5-chlorothiophène-2-carbonyl)amino]-*N*-[3-méthyl-4-(3-oxomorpholin-4-yl)phényl]-3-méthoxypropionamide,
le (2R,3R)-2-[(5-chlorothiophène-2-carbonyl)amino]-*N*-[4-(3-oxo-morpholin-4-yl)phényl]-3-méthoxybutyramide,
le (2R,3R)-2-[(5-chlorothiophène-2-carbonyl)amino]-*N*-[3-méthyl-4-(3-oxomorpholin-4-yl)phényl]-3-méthoxybutyramide,
le (S)-2-[(5-chlorothiophène-2-carbonyl)amino]-*N*-[3-méthyl-4-(3-oxomorpholin-4-yl)phényl]-3-méthoxypropionamide,
le (R)-2-[(5-chlorothiophène-2-carbonyl)amino]-*N*-[3-trifluorométhyl-4-(3-oxomorpholin-4-yl)phényl]-3-méthoxypropionamide,
le (R)-2-[(5-chlorothiophène-2-carbonyl)amino]-*N*-[3-chloro-4-(2-azabicyclo[2.2.2]octan-2-yl)phényl]-3-méthoxypropionamide,
le (R)-2-[(5-chlorothiophène-2-carbonyl)amino]-*N*-[3-trifluoro-méthoxy-4-(2-azabicyclo[2.2.2]octan-2-yl)phényl]-3-méthoxypropion-amide,
le (R)-2-[(5-chlorothiophène-2-carbonyl)amino]-*N*-[3-chloro-4-(3-oxomorpholin-4-yl)phényl]-3-méthoxypropionamide,
le (R)-2-[(5-chlorothiophène-2-carbonyl)amino]-*N*-[3-fluoro-4-(3-oxomorpholin-4-yl)phényl]-3-méthoxypropionamide,
le (R)-2-[(5-chlorothiophène-2-carbonyl)amino]-*N*-[3-chloro-4-(2-oxo-2*H*-pyridin-1-yl)phényl]-3-méthoxypropionamide,
le (R)-2-[(5-chlorothiophène-2-carbonyl)amino]-*N*-[3-méthyl-4-(3-oxomorpholin-4-yl)phényl]-3-allylpropionamide,
le (R)-2-[(5-chlorothiophène-2-carbonyl)amino]-*N*-[3-méthyl-4-(3-oxomorpholin-4-yl)phényl]-3-propoxypropionamide,
le (R)-2-[(5-chlorothiophène-2-carbonyl)amino]-*N*-[3-méthyl-4-(3-oxomorpholin-4-yl)phényl]-3-éthoxypropionamide,
le (R)-2-[(5-chlorothiophène-2-carbonyl)amino]-*N*-[3-méthyl-4-(3-oxomorpholin-4-yl)phényl]-3-(2-méthoxyéthoxy)propionamide,
le (2R,3R)-2-[(5-chlorothiophène-2-carbonyl)amino]-*N*-[3-méthyl-4-(3-oxomorpholin-4-yl)phényl]-3-éthoxybutyramide,
le (2R,3R)-2-[(5-chlorothiophène-2-carbonyl)amino]-*N*-[3-méthyl-4-(3-oxomorpholin-4-yl)phényl]-3-(2-méthoxyéthoxy)butyramide,
le (R)-2-[(5-chlorothiophène-2-carbonyl)amino]-*N*-[3-méthyl-4-(3-oxomorpholin-4-yl)phényl]-3-méthylsulfonylpropionamide,
le (R)-2-[(5-chlorothiophène-2-carbonyl)amino]-*N*-[4-(2-oxo-2*H-*pyridin-1-yl)phényl]-3-méthylsulfonylpropionamide,
le (R)-2-[(5-chlorothiophène-2-carbonyl)amino]-*N*-[4-(3-oxomorpholin-4-yl)phényl]-3-méthylsulfonylpropionamide,
le (R)-2-[(5-chlorothiophène-2-carbonyl)amino]-*N*-[4-(3-oxomorpholin-4-yl)phényl]-3-méthylsulfonylbutyramide,
le (R)-2-[(5-chlorothiophène-2-carbonyl)amino]-*N*-[3-méthyl-4-(3-oxomorpholin-4-yl)phényl]-3-méthylsulfonylbutyramide,
le (R)-2-[(5-chlorothiophène-2-carbonyl)amino]-*N*-[4-(2-oxo-2*H-*pyridin-1-yl)phényl]-3-méthylsulfonylbutyramide,
le (R)-2-[(5-chlorothiophén-2-ylméthyl)amino]-*N*-[4-(3-oxomorpholin-4-yl)phényl]valéramide,
le (R)-2-[(5-chlorothiophène-2-carbonyl)amino]-*N*-[4-(3-oxomorpholin-4-yl)phényl]-3-carboxypropionamide,
le (R)-2-[(5-chlorothiophène-2-carbonyl)amino]-*N*-[3-méthyl-4-(3-oxomorpholin-4-yl)phényl]-3-carboxypropionamide,
le (R)-2-[(5-chlorothiophène-2-carbonyl)amino]-*N*-[4-(3-oxomorpholin-4-yl)phényl]-4-carboxybutyramide,
le (R)-2-[(5-chlorothiophène-2-carbonyl)amino]-*N*-[3-méthyl-4-(3-oxomorpholin-4-yl)phényl]-4-carboxybutyramide,
le (R)-2-[(5-chlorothiophène-2-carbonyl)amino]-*N*-[4-(3-oxomorpholin-4-yl)phényl]-4-aminobutyramide,
le (R)-2-[(5-chlorothiophène-2-carbonyl)amino]-*N*-[3-méthyl-4-(3-oxomorpholin-4-yl)phényl]-4-aminobutyramide,
le (R)-2-[(5-chlorothiophène-2-carbonyl)amino]-*N*-[4-(3-oxomorpholin-4-yl)phényl]-5-aminovaléramide,
le (R)-2-[(5-chlorothiophène-2-carbonyl)amino]-*N*-[3-méthyl-4-(3-oxomorpholin-4-yl)phényl]-5-aminovaléramide,
le (R)-2-[(5-chlorothiophène-2-carbonyl)amino]-*N*-[4-(3-oxomorpholin-4-yl)phényl]-3-aminopropionamide,
le (R)-2-[(5-chlorothiophène-2-carbonyl)amino]-*N*-[3-méthyl-4-(3-oxomorpholin-4-yl)phényl]-3-aminopropionamide,
le (R)-2-[(5-chlorothiophène-2-carbonyl)amino]-*N*-[3-méthyl-4-(3-oxomorpholin-4-yl)phényl]-3-hydroxypropionamide,
le (R)-2-[(5-chlorothiophène-2-carbonyl)amino]-*N*-[3-4-(3-oxo-morpholin-4-yl)phényl]-3-hydroxypropionamide,
le (2R,3R)-2-[(5-chlorothiophène-2-carbonyl)amino]-*N*-[4-(3-oxomorpholin-4-yl)phényl]-3-hydroxybutyramide,
le (2R,3R)-2-[(5-chlorothiophène-2-carbonyl)amino]-*N*-[4-(3-oxomorpholin-4-yl)phényl]-3-aminocarbonyloxybutyramide,
le (2R)-2-[(5-chlorothiophène-2-carbonyl)amino]-*N*-[3-méthyl-4-(3-oxomorpholin-4-yl)phényl]-3-aminocarbonyloxypropionamide,
le (2R,3R)-2-[(5-chlorothiophène-2-carbonyl)amino]-*N*-[3-méthyl-4-(3-oxomorpholin-4-yl)phényl]-3-aminocarbonyloxybutyramide,
et les sels, solvats et stéréoisomères pharmaceutiquement utilisables de ceux-ci, y compris leurs mélanges en toutes proportions.

18. Procédé de préparation de composés de formule I selon les revendications 1-17 et de sels, solvats et stéréoisomères pharmaceutiquement utilisables de ceux-ci, **caractérisé en ce que**
a) un composé de formule II
H₂N-W-Y-T II
dans laquelle
W, Y et T ont les significations indiquées selon la revendication 1,
est réagi avec un composé de formule III 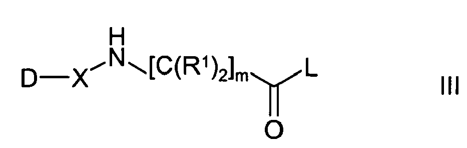 dans laquelle
L désigne Cl, Br, I ou un groupement OH libre ou modifié de manière réactive et fonctionnelle, et
R¹, m, X et D ont les significations indiquées selon la revendication 1,
ou
b) pour la préparation de composés de formule I
dans laquelle X désigne -C=O,
un composé de formule IV 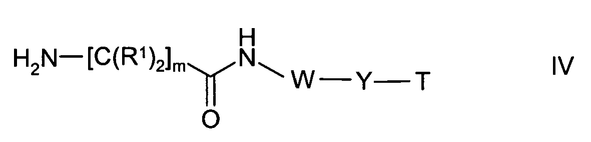 dans laquelle R¹, m, W, Y et T ont les significations indiquées selon la revendication 1,
est réagi avec un composé de formule V
D-CO-L V
dans laquelle
L désigne Cl, Br, I ou un groupement OH libre ou modifié de manière réactive et fonctionnelle, et
D a la signification indiquée selon la revendication 1,
ou
c) pour la préparation de composés de formule I
dans laquelle X désigne CH₂,
un composé de formule IV 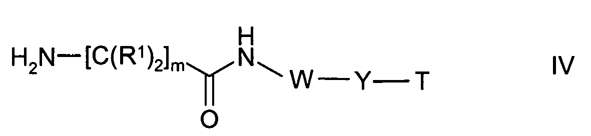 dans laquelle R¹, m, W, Y et T ont les significations indiquées selon la revendication 1,
est réagi avec un composé de formule VI
D-CHO VI
dans laquelle
D a la signification indiquée selon la revendication 1,
dans une amination réductrice,
et/ou
une base ou acide de formule I est converti(e) en l'un de ses sels.

19. Composés de formule I selon l'une ou plusieurs des revendications 1 à 17, comme inhibiteurs du facteur de coagulation Xa.

20. Composés de formule I selon l'une ou plusieurs des revendications 1 à 17, comme inhibiteurs du facteur de coagulation VIIa.

21. Médicaments comprenant au moins un composé de formule I selon l'une ou plusieurs des revendications 1 à 17, et/ou des sels, solvats et stéréoisomères pharmaceutiquement utilisables de ceux-ci, y compris des mélanges de ceux-ci en toutes proportions, et éventuellement des excipients et/ou adjuvants.

22. Médicaments comprenant au moins un composé de formule I selon l'une ou plusieurs des revendications 1 à 17, et/ou des sels, solvats et stéréoisomères pharmaceutiquement utilisables de ceux-ci, y compris des mélanges de ceux-ci en toutes proportions, et au moins un autre ingrédient actif médicamenteux.

23. Utilisation de composés selon l'une ou plusieurs des revendications 1 à 17 et/ou de sels et solvats physiologiquement acceptables de ceux-ci, pour la préparation d'un médicament destiné au traitement des thromboses, de l'infarctus du myocarde, de l'artériosclérose, des inflammations, de l'apoplexie, de l'angine de poitrine, de la resténose après angioplastie, de la boiterie intermittente, de la migraine, des tumeurs, des maladies tumorales et/ou des métastases tumorales.

24. Ensemble (kit) constitué de conditionnements séparés
(a) d'une quantité efficace d'un composé de formule I selon l'une ou plusieurs des revendications 1 à 17 et/ou de sels, solvats et stéréoisomères pharmaceutiquement utilisables de ceux-ci, y compris des mélanges de ceux-ci en toutes proportions,
et
(b) d'une quantité efficace d'un autre ingrédient actif médicamenteux.
